(19) Europäisches Patentamt
European Patent Office
Office européen des brevets

(11) **EP 4 012 014 A1**

(12) **EUROPEAN PATENT APPLICATION**
published in accordance with Art. 153(4) EPC

(43) Date of publication:
**15.06.2022  Bulletin 2022/24**

(21) Application number: **20853066.7**

(22) Date of filing: **11.08.2020**

(51) International Patent Classification (IPC):
*C12M 1/00* *(2006.01)*        *C12M 3/00* *(2006.01)*
*C12N 5/07* *(2010.01)*        *C12N 5/071* *(2010.01)*
*C12N 5/0735* *(2010.01)*      *C12N 5/074* *(2010.01)*
*C12N 5/075* *(2010.01)*       *C12N 5/076* *(2010.01)*
*C12N 5/0775* *(2010.01)*      *C12N 5/09* *(2010.01)*
*C12N 5/10* *(2006.01)*

(52) Cooperative Patent Classification (CPC):
**C12M 1/00; C12M 3/00; C12N 5/10**

(86) International application number:
**PCT/JP2020/030613**

(87) International publication number:
**WO 2021/029408 (18.02.2021 Gazette 2021/07)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **09.08.2019  JP 2019148238**

(71) Applicant: Ube Industries, Ltd.
Yamaguchi 755-8633 (JP)

(72) Inventors:
• **HAGIHARA, Masahiko**
  **Ube-shi, Yamaguchi 755-8633 (JP)**
• **HARADA, Takashi**
  **Ube-shi, Yamaguchi 755-8633 (JP)**
• **YAMADA, Shotaro**
  **Ube-shi, Yamaguchi 755-8633 (JP)**
• **TAKAMA, Akira**
  **Ube-shi, Yamaguchi 755-8633 (JP)**

(74) Representative: **Plougmann Vingtoft a/s**
**Strandvejen 70**
**2900 Hellerup (DK)**

(54) **METHOD FOR PRODUCING EXOSOME**

(57)    The present invention provides a method for producing an exosome, comprising a step of applying a cell to a cell culture module to culture the cell and a step of producing an exosome by the cell, wherein the cell culture module is provided with a polymer porous film and a casing having at least two culture medium in-flow/out-flow ports and having the polymer porous film placed therein.

FIG. 27

COMPARISON OF EXOSOME PRODUCTION AMOUNT PER CELL

EP 4 012 014 A1

**Description**

FIELD

The present invention relates to a method for producing an exosome using a cell.

BACKGROUND

An exosome is one kind of membranous vesicular structure, 50 to 300 nm, which a cell secretes out of the cell. An exosome is formed in a multivesicular endosome when the membrane of the endosome has recessed in after the endosome is formed through endocytosis. Thus, the surface of the exosome contains a surface protein derived from the cell membrane, and the inside of the exosome contains a nucleic acid and a protein that are derived from the cytoplasm. In recent years, there has been a report that various cell species have an endosome that has a finely controlled constitution of proteins and nucleic acids, and is involved in information transmission among cells in the vicinity or in the distance (NPL 1).

An attempt is being made to utilize an exosome mainly as a diagnostic marker, drug delivery carrier, and bio-pharmaceutical. A cancer cell and a disease cell secrete an exosome which reflects the pathological state of such a cell, and thus, profiling the characteristics of the exosome makes it possible to diagnose the disease (NPL 2). An exosome made to support a drug can be utilized also for drug delivery targeted at a cell that receives the exosome. Examples of methods of supporting a drug include: an approach by which a drug is bonded to or taken in a secreted exosome; and an approach by which an exosome containing a drug is secreted into a cell containing a drug in the cytoplasm (NPL 3).

There is also a report that an exosome can itself function as a bioactive substance. The report mentions that an exosome isolated from a cultured neural stem cell line induces the migration of a fibroblast, the branching of a human umbilical vein endothelial cell, and the outgrowth of a neurite, in vitro (PTL 1). There is a report that an exosome of a myeloid dendritic cell has an immune regulation function, and is being tested for a therapeutic effect on an infectious disease, allergy, autoimmunity disease, and the like in a mouse model (NPLs 4 and 5).

The bioactivity of an exosome is considered to be supported by the constitution of nucleic acids and proteins, wherein the constitution is controlled by the cell from which the exosome is derived. In particular, the relationship between a miRNA contained in an exosome and the bioactivity is attracting attention. PTL 2 states that an exosome isolated from a cardiosphere (cardiosphere) or a cardiac-muscle-derived cell (CDC) contains miR-146a, miR-210, miR-22, and miR-24, and that these have a therapeutic effect on a damaged or diseased cardiac tissue. In addition, PTL 3 states that a liposome containing at least one or more miRNAs out of miR-34b, miR-132, miR-137, miR-193a, and miR-203 acts as an inhibitor of oral squamous cell carcinoma. Furthermore, NPL 6 states that miR-26a and miR-26b inhibit the cell migration and wetting of a gastric cell cancer, and NPL 7 states that miR-23b, miR-27b, and miR-24 superiorly inhibit the cell migration and wetting of a prostate cancer cell.

Utilizing an exosome as a drug delivery carrier or a bioactive substance involves the establishment of an approach for producing a sufficient amount of exosomes having uniform quality. Exosomes are usually obtained by performing ultracentrifugation to collect exosomes secreted into a culture system by culture cells. The conventional technologies in the above-mentioned articles of literature or the like that present a method for utilizing an exosome include using the respective suitable methods for collecting an exosome to be used for a test, but none of the technologies are directed at the development of a stable and highly efficient production method that makes it possible to produce exosomes on a pharmaceutical level on an industrial scale.

PTL 4 discloses a method for producing an exosome. This method is characterized by inducing the secretion of an exosome by bringing a prostaglandin E receptor 4 (EP4) antagonist in contact with a stem cell. In NPL 8, a system having a combination of a 3D culture system of a mesenchymal stem cell and a tangential flow filtration (TFF) is presented as an example of a cell culture system constituted to highly efficiently produce exosomes having favorable bioactivity.

As above-mentioned, the constitution of a nucleic acid, protein, and various signaling molecules characterizes an exosome, and is controlled by a cell from which the exosome is derived, and thus, the quality of an exosome produced in each phase of the induction phase, logarithmic growth phase, stationary phase, and death phase in a cell culture is considered to vary. However, considering the production of exosomes on an industrial scale, it is undesirable from a quality control viewpoint that the characteristics of an exosome varies depending on the culture time, and it is desirable that exosomes having uniform quality can be produced in a once established production system over a long period of time. A conventional exosome production technology using a culture cell does not sufficiently solve such a problem in that the quality of an exosome varies with the time course of the cell culture system, and the problem creates a technical barrier which inhibits the supply of exosomes having stable quality and the establishment of an exosome production system on an industrial scale.

In cases where a cultured cell is used for production of a substance, which is not limited to an exosome, it is important to establish a cell culture method that is the most suitable to produce a desired substance highly efficiently and stably. A cell exists in a living body generally as a group having a three-dimensional structure. On the other hand, in cases where cells are cultured in an artificial environment, examples of methods to be generally used include: a classic plane culture method in which cells are cultured two-dimensionally in the form of a monolayer sticking to the bottom of a culture vessel; and a suspension culture method in which cells are cultured in a state of dispersion in a liquid culture medium. A cell suitable to be used for a plane culture method is a cell having relatively high adhesiveness, but in some cases, even the use of such a suitable cell causes a large variation in the nature of the cell, depending on the difference in the culture environment. Also for a suspension culture method, some cells are suitable, and others are not.

Mass-producing in vivo proteins and the like using a cell culture is attracting attention as a demand rises for in vivo proteins and the like to be used in medical applications. For suspension cells such as *E. coli,* a technology of mass culture in a large culture vessel has been under study. Mass-culturing suspension cells using a large culture vessel involves a large amount of culture solution and a stirring device. On the other hand, studies on the production of a substance with the use of an adherent cell are attracting much interest, along with the progress in studies on a cell to be used. In a classic plane culture method, adherent cells expand only two-dimensionally, and thus, involve a large culture area. Many studies on a cell culture carrier and a bioreactor are being conducted to culture large volumes of cells more three-dimensionally for the purpose of mass-producing in vivo proteins and the like.

A typical cell culture carrier considered widely as a subject of study is a microcarrier which is a small particle to which a cell can be adhered and grow (PTL 5). Various kinds of microcarriers have been under research and development, and many of them are commercially available. These microcarriers are often used in the production of vaccines and proteins, and widely accepted in a methodology and as a system that meets an increase in the scale. However, microcarrier culture involves stirring and diffusing microcarriers sufficiently not to agglomerate them, and thus, the cell culture has a quantitative upper limit. Additionally, for example, to produce a substance, separating a carrier itself involves separating the carrier using a filter or the like which separates fine particles. This involves complicated work also in terms of methodology. Furthermore, the cells are apt to be detached by a shearing force, and thus, the stirring conditions are extremely limited. Accordingly, such a culture method is not compatible with the culture conditions for a high flow rate such as used for *E. coli.*

Another method discovered as a method different from a microcarrier culture is a method in which spheroid cells are continuously mass-cultured with a three-dimensional culture method using methyl cellulose or gellan gum. Indeed, a bioreactor or the like in which a cellulose sponge is used makes it possible to culture large volumes of cells. However, such a system results in a large closed system, and, for example, does not allow easy contact with a culture environment, thus having many restrictions from an operational viewpoint.

A demand is rising for creation of a novel system that makes it possible that exosomes having uniform quality are efficiently produced by cells over a long period of time in a system suited for simplification and automation.

<Porous polyimide membrane>

The cell culture method which includes applying cells to a porous polyimide membrane and culturing them is reported (PTL 6).

Polyimide is a general term for polymers containing imide bonds in the repeating unit. An aromatic polyimide refers to a polymer in which aromatic compounds are directly linked by imide bonds. An aromatic polyimide has an aromatic-aromatic conjugated structure via an imide bond, and therefore has a strong rigid molecular structure, and since the imide bonds provide powerful intermolecular force, it has very high levels of thermal, mechanical and chemical properties.

Porous polyimide membranes have been utilized in the prior art for filters and low permittivity membranes, and especially for battery-related purposes, such as fuel cell electrolyte membrane and the like. PTLs 7 to 9 describe porous polyimide membranes with numerous macrovoids, having excellent permeability to objects such as gases, high porosity, excellent smoothness on both surfaces, relatively high strength and, despite high porosity, excellent resistance against compression stress in the membrane thickness direction. All of these are porous polyimide membranes formed via amic acid.

[CITATION LIST]

[PATENT LITERATURE]

[PTL 1] WO2013/150303
[PTL 2] Japanese Unexamined Patent Publication (Kokai) No. 2019-38840
[PTL 3] Japanese Unexamined Patent Publication (Kokai) No. 2009-171876

[PTL 4] Japanese Unexamined Patent Publication (Kokai) No. 2018-064550
[PTL 5] WO2003/054174
[PTL 6] WO2015/012415
[PTL 7] WO2010/038873
[PTL 8] Japanese Unexamined Patent Publication (Kokai) No. 2011-219585
[PTL 9] Japanese Unexamined Patent Publication (Kokai) No. 2011-219586
[PTL 10] WO2018/021368

[NON PATENT LITERATURE]

[NPL 1] J. of Extracellular Vesicles, 2015, 4, Article: 27066
[NPL 2] JAMA Oncology, 2016, 2, p882-889
[NPL 3] Trends in Biotechnology, 2017, 7, p665-676
[NPL 4] Transplantation 2003, 76: 1503-10
[NPL 5] Am. J. Transplant. 2006, 6:1541-50
[NPL 6] Int J Oncol. 2016 May;48(5):1837-46
[NPL 7] Oncotarget. 2014; 5:7748-7759
[NPL 8] Molecular Therapy Vol. 26 No 12 2018, 2838-2847

SUMMARY

[TECHNICAL PROBLEM]

An object of the invention is to provide the following: a method for continuously producing an exosome having uniform quality in a high-yield manner with the use of a cell over a long period of time; an exosome production device; a kit; the use of these; and an exosome produced using these.

[SOLUTION TO PROBLEM]

The present inventors have discovered that a module constituted by a porous polymer membrane contained in a casing is suitable for mass cell culture and cell removal (PTL 10). The inventors have vigorously studied to solve the above-mentioned problems, that is, to provide the following: a method that enables a cell to produce an exosome simply and efficiently; a cell culture system; and a stable-quality exosome produced using the cell culture system. As a result, the inventors have reached the present invention through the discovery that culturing a cell using such a module comprising a porous polymer membrane and a casing makes it possible that an exosome having stable quality is produced in a stable production amount over a long period of time.

Surprisingly, using a method of the invention makes it possible that the culture cell produces a uniform-quality exosome sustainably over several months. This is extremely advantageous for producing, on an industrial scale, an exosome to be utilized as a bioactive substance, from a viewpoint of being able to utilize, for a long period of time, a once established production system of an exosome having a desired nature, and from a viewpoint of being able to produce a large amount of uniform-quality exosomes.

Accordingly, one of the characteristics of the invention is to culture cells using a module comprising a porous polymer membrane and a casing.

Cells are stably and three-dimensionally grown utilizing the communication holes of the porous polymer membrane which have a large diameter and can be passed by the cells. Even if large volumes of cells are present, compared with a plane culture method, the communication holes secure the contact with a culture medium, thus making it possible to continue the growth stably. Furthermore, the thin membrane characteristics, flexible characteristics, shape stability, and free formability of the porous polymer membrane make it possible, for example, that many sheets coexist or many membranes are stacked in a small unit space, and in addition, the super heat resistance and solvent resistance of the porous polymer membrane make it possible to sterilize the sheets simply and rapidly using a plurality of means. Furthermore, the three-dimensional scaffold structure of the porous polymer membrane makes it promising that the exosome production amount per cell is enhanced, compared with a planar culture.

The present invention preferably includes, but is not limited to, the following modes.

1. A method for producing an exosome using a cell, the method comprising the steps of:

applying the cell to a cell culture module to culture the cell; and
allowing the cell to produce the exosome;
wherein the cell culture module comprises:

> a porous polymer membrane; and
> a casing having two or more medium flow inlets/outlets and containing the porous polymer membrane.

2. The method according to mode 1,

> wherein the porous polymer membrane is a three-layer structure porous polymer membrane having a surface layer A and a surface layer B, the surface layers having a plurality of pores, and a macrovoid layer sandwiched between the surface layers A and B,
> wherein an average pore diameter of the pores present in the surface layer A is smaller than an average pore diameter of the pores present in the surface layer B,
> wherein the macrovoid layer has a partition wall bonded to the surface layers A and B, and a plurality of macrovoids surrounded by the partition wall and the surface layers A and B,
> wherein the pores in the surface layers A and B communicate with the macrovoid, and
> wherein the porous polymer membrane is contained within the casing with:

>> (i) the two or more independent porous polymer membranes being aggregated;
>> (ii) the porous polymer membranes being folded up;
>> (iii) the porous polymer membranes being wound into a roll-like shape; and/or
>> (iv) the porous polymer membranes being tied together into a rope-like shape.

3. The method according to mode 1 or 2, wherein the diameter of the medium flow inlet/outlet is larger than the diameter of the cell, and smaller than the diameter at which the porous polymer membranes flow out.

4. The method according to any one of modes 1 to 3, wherein the casing has a mesh-like structure.

5. The method according to any one of modes 1 to 4, wherein the casing consists of an inflexible material.

6. The method according to any one of modes 1 to 5, wherein the porous polymer membrane has a plurality of pores having an average pore diameter of 0.01 to 100 $\mu$m.

7. The method according to any one of modes 2 to 6, wherein an average pore diameter of the surface layer A is 0.01 to 50 $\mu$m.

8. The method according to any one of modes 2 to 7, wherein an average pore diameter of the surface layer B is 20 to 100 $\mu$m.

9. The method according to any one of modes 1 to 8, wherein a total membrane thickness of the porous polymer membrane is 5 to 500 $\mu$m.

10. The method according to any one of modes 1 to 9, wherein the porous polymer membrane is a porous polyimide membrane.

11. The method according to mode 10, wherein the porous polyimide membrane is a porous polyimide membrane comprising a polyimide derived from tetracarboxylic dianhydride and diamine.

12. The method according to mode 10 or 11, wherein the porous polyimide membrane is a colored porous polyimide membrane that is obtained by molding a polyamic acid solution composition comprising a polyamic acid solution derived from tetracarboxylic dianhydride and diamine, and a coloring precursor, and subsequently heat-treating the resultant composition at 250°C or higher.

13. The method according to any one of modes 1 to 12, wherein the step of culturing a cell is performed under stationary culture conditions.

14. The method according to any one of modes 1 to 12, wherein the step of culturing a cell is performed under rotating or stirring culture conditions.

15. The method according to any one of modes 1 to 14, wherein the step of culturing a cell is performed continuously.

16. The method according to any one of modes 1 to 15,
wherein the step of culturing a cell is performed in a cell culture device placed in an incubator, the cell culture device comprising:

> a culture unit that contains the cell culture module configured to support the cell, and comprises a medium supply port and a medium discharge port; and
> a culture medium supply unit comprising:

>> a culture medium storage container;

a medium supply line; and

a liquid-transfer pump configured to liquid-transfer a culture medium via the medium supply line, wherein a first end of the medium supply line is in contact with the culture medium in the culture medium storage container, and a second end of the medium supply line communicates with the culture unit via the medium supply port of the culture unit.

17. The method according to mode 16, wherein the cell culture device does not have the medium supply line, the liquid-transfer pump, an air supply port, an air discharge port, and an oxygen permeation membrane.

18. The method according to any one of modes 1 to 17, wherein the cell is selected from the group consisting of pluripotent stem cells, tissue stem cells, somatic cells, germ cells, sarcoma cells, established cell lines, and transformants.

19. The method according to any one of modes 1 to 18, wherein the cell is selected from the group consisting of human mesenchymal stem cells, osteoblasts, chondrocytes, and cardiomyocytes.

20. The method according to any one of modes 1 to 19, wherein the step of producing an exosome is at least partially the same as the step of culturing a cell.

21. The method according to any one of modes 1 to 20, wherein the step of producing an exosome is continued over 1 month, 2 months, 3 months, 6 months, or a longer period of time.

22. An exosome production device for use in the method according to any one of modes 1 to 21, the device comprising the cell culture module.

23. A kit for use in the method according to any one of modes 1 to 21, the device comprising the cell culture module.

24. Use of the cell culture module for the method according to any one of modes 1 to 21.

25. An exosome obtained by the method according to any one of modes 1 to 21, the method comprising the cell culture module.

[ADVANTAGEOUS EFFECTS OF INVENTION]

Using the porous polymer membrane contained in the casing and modularized makes it possible that cells suspended are adsorbed efficiently, and that the cells are cultured stably for a long period of time using a conventional suspension culture vessel or the like. Enabling cells to be continuously mass-cultured stably for a long period of time under suitable conditions makes it possible to create a stable and efficient exosome production system.

BRIEF DESCRIPTION OF DRAWINGS

FIG. 1 represents an embodiment of a cell culture module. A porous polymer membrane is contained in a casing.

FIG. 2 represents an embodiment of a cell culture module. A porous polymer membrane is contained in a casing.

FIG. 3 represents an embodiment of a cell culture module. (A) A porous polymer membrane is contained in a mesh-like casing. (B) represents an embodiment of a casing composed of a mesh-like net, and a framework.

FIG. 4 represents an embodiment of a device used in combination when the cell culture module is applied in a spinner flask. (A) A rotating type device. The rotating type device to which the cell culture module is applied is placed in a spinner flask and used while rotating the device by itself. (B) Stationary type device. The device to which the cell culture module is applied is placed in a bottom part of a spinner flask. The device is used while a stirrer in a spinner flask is rotated in a central space of the device.

FIG. 5 represents an embodiment wherein culture is carried out applying a cell culture module to a flexible bag type vessel. Phenol red in a culture solution turned yellow in 1 hour, the left panel illustrating the culture solution at the beginning of the culture and the right panel illustrating the culture solution 1 hour after beginning of the culture.

FIG. 6 represents an embodiment of using a mesh-type module during shaking culture.

FIG. 7 represents a model diagram of cell culturing using a porous polyimide membrane.

FIG. 8 is a diagram illustrating a dry heat sterilization type, siphon type cell culture device (heat resistant siphon type reactor).

FIG. 9 is a diagram illustrating a siphon type cell culture device.

FIG. 10 represents a cylindrical type vapor phase culture device. (A) is a diagram illustrating a construction of a cell culture device. (B) is a diagram illustrating a cell culture unit on which a cell culture device is mounted in (A).

FIG. 11 represents a cylindrical type vapor phase culture device. The embodiment has a structure which facilitates discharge of medium owing to medium discharge ports of the respective stages being displaced in counterclockwise direction by 30 degrees, with a basic construction being common to that in FIG. 10.

FIG. 12 represents a mist/shower type culture device.

FIG. 13 represents a vapor phase exposed type rotating culture device. (A) represents a construction of the vapor phase exposed type rotating culture device. (B) represents a use aspect of the vapor phase exposed type rotating culture device.

FIG. 14 represents the cell culture unit of the vapor phase exposed type rotating culture device. (A) is a schematic view of the cell culture unit, and (B) represents a use aspect of the culture unit. (A) After two days of stationary culture, (B) After two days of shaking culture (without a mesh).

FIG. 15 represents a diagram illustrating a WAVE-type bioreactor to which a cell culture module according to an embodiment of the present invention is applied. (A) represents a bag enclosing a cell culture module, and (B) represents a step of cell adsorption to the cell culture module using WAVE 25.

FIG. 16 represents a diagram illustrating a cell culture device according to an embodiment of the present invention. (A) represents a cell culture module, (B) represents a cell culture unit, and (C) represents a cell culture device in an embodiment.

FIG. 17 represents a diagram illustrating a cell culture device in an embodiment.

FIG. 18 represents the results of an exosome production test on a cell culture using a dish, (A) represents the measurement results of the number of exosomes produced per day and contained in the cell culture solution collected on culture Day 6 and Day 13, and (B) represents the result of a particle diameter distribution measurement made on culture Day 13.

FIG. 19 represents the results of an exosome production test on a cell culture using modules in a 150-mL round storage bottle, (A) represents the measurement results of the number of exosomes produced per day and contained in the cell culture solution collected up to culture Day 27, and (B) represents the result of a particle diameter distribution measurement made on culture Day 27.

FIG. 20 represents the results of an exosome production test on a cell culture using modules in a 150-mL round storage bottle, (A) represents the measurement results of the number of exosomes produced per day and contained in the cell culture solution collected up to culture Day 27, and (B) represents the result of a particle diameter distribution measurement made on culture Day 27.

FIG. 21 represents a cell culture system (5% oxygen) using modules in an overflow reactor, (A) represents changes over time in the glucose consumption amount and the lactic acid production amount, and (B) represents an aspect of the cell culture.

FIG. 22 represents the results of an exosome production test on a cell culture (5% oxygen) using modules in an overflow reactor, (A) represents the measurement results of the number of exosomes produced per day and contained in the cell culture solution collected up to culture Day 85, and (B) represents the result of a particle diameter distribution measurement made on culture Day 85.

FIG. 23 represents a cell culture system (normal oxygen) using modules in an overflow reactor, (A) represents changes over time in the glucose consumption amount and the lactic acid production amount, and (B) represents an aspect of the cell culture.

FIG. 24 represents the results of an exosome production test on a cell culture (normal oxygen) using modules in an overflow reactor, (A) represents the measurement results of the number of exosomes produced per day and contained in the cell culture solution collected up to culture Day 85, and (B) represents the result of a particle diameter distribution measurement made on culture Day 85.

FIG. 25 (A) represents changes over time in the glucose consumption amount and the lactic acid production amount in a cell culture system using modules in a WAVE reactor. FIGs. 25 (B) and 25 (C) represent the results of an exosome production test on a cell culture using modules in a WAVE reactor, (B) represents the measurement results of the number of exosomes produced per day and contained in the cell culture solution collected up to culture Day 39, and (C) represents the result of a particle diameter distribution measurement made on culture Day 39.

FIG. 26 represents a comparison of the exosome productivity per cell on culture Day 13 among Comparative Example 1 and Examples 1 to 4.

FIG. 27 represents a comparison of the exosome production amount per cell between Comparative Example 1 (Dish_Day 6) and Example 1 (Bottle (5% oxygen)_Day 27).

FIG. 28 represents a Scatterplot of miRNA array analyses in Comparative Example 1 (Dish Day 6) and Example 1 (Bottle (5% oxygen)_Day 27).

FIG. 29 represents a Scatterplot of miRNA array analyses of hsa-miR-146a, hsa-miR-210, hsa-miR-22, and hsa-miR-24.

FIG. 30 represents the results of miRNA array analyses of hsa-miR-34b, hsa-miR-132, hsa-miR-203, hsa-miR-137, and hsa-miR-193.

FIG. 31 represents the results of miRNA array analyses of hsa-miR-26a/b, hsa-miR-23b, hsa-miR-27b, and hsa-miR-24-1.

FIG. 32 represents a comparison of the exosome production amount per cell between Comparative Example 1 (Dish_Day 13) and Example 1 (Bottle (5% oxygen)_Day 27).

FIG. 33 represents a Scatterplot of miRNA array analyses in Comparative Example 1 (Dish Day 13) and Example 1 (Bottle (5% oxygen)_Day 27).

FIG. 34 represents the results of miRNA array analyses of hsa-miR-146a, hsa-miR-210, hsa-miR-22, and hsa-miR-24.

FIG. 35 represents the results of miRNA array analyses of hsa-miR-34b, hsa-miR-132, hsa-miR-203, hsa-miR-137, and hsa-miR-193.

FIG. 36 represents the results of miRNA array analyses of hsa-miR-26a/b, hsa-miR-23b, hsa-miR-27b, and hsa-miR-24-1.

FIG. 37 represents a comparison of the exosome production amount per cell between Comparative Example 1 (Dish Day 6) and Example 2 (Bottle (Normal Oxygen)_Day 27).

FIG. 38 represents a Scatterplot of miRNA array analyses in Comparative Example 1 (Dish Day 6) and Example 2 (Bottle (Normal Oxygen)_Day 27).

FIG. 39 represents the results of miRNA array analyses of hsa-miR-146a, hsa-miR-210, hsa-miR-22, and hsa-miR-24.

FIG. 40 represents the results of miRNA array analyses of hsa-miR-34b, hsa-miR-132, hsa-miR-203, hsa-miR-137, and hsa-miR-193.

FIG. 41 represents the results of miRNA array analyses of hsa-miR-26a/b, hsa-miR-23b, hsa-miR-27b, and hsa-miR-24-1.

FIG. 42 represents a comparison of the exosome production amount per cell between Comparative Example 1 (Dish Day 13) and Example 2 (Bottle (Normal Oxygen)_Day 27).

FIG. 43 represents a Scatterplot of miRNA array analyses in Comparative Example 1 (Dish Day 13) and Example 2 (Bottle (Normal Oxygen)_Day 27).

FIG. 44 represents the results of miRNA array analyses of hsa-miR-146a, hsa-miR-210, hsa-miR-22, and hsa-miR-24.

FIG. 45 represents the results of miRNA array analyses of hsa-miR-34b, hsa-miR-132, hsa-miR-203, hsa-miR-137, and hsa-miR-193.

FIG. 46 represents the results of miRNA array analyses of hsa-miR-26a/b, hsa-miR-23b, hsa-miR-27b, and hsa-miR-24-1.

FIG. 47 represents a comparison of the exosome production amount per cell between Comparative Example 1 (Dish Day 6) and Example 3 (Reactor (5% oxygen)_Day 27).

FIG. 48 represents a Scatterplot of miRNA array analyses in Comparative Example 1 (Dish_Day 6) and Example 3 (Reactor (5% oxygen)_Day 27).

FIG. 49 represents the results of miRNA array analyses of hsa-miR-146a, hsa-miR-210, hsa-miR-22, and hsa-miR-24.

FIG. 50 represents the results of miRNA array analyses of hsa-miR-34b, hsa-miR-132, hsa-miR-203, hsa-miR-137, and hsa-miR-193.

FIG. 51 represents the results of miRNA array analyses of hsa-miR-26a/b, hsa-miR-23b, hsa-miR-27b, and hsa-miR-24-1.

FIG. 52 represents a comparison of the exosome production amount per cell between Comparative Example 1 (Dish Day 13) and Example 3 (Reactor (5% oxygen)_Day 27).

FIG. 53 represents a Scatterplot of miRNA array analyses in Comparative Example 1 (Dish Day 13) and Example 3 (Reactor (5% oxygen)_Day 27).

FIG. 54 represents the results of miRNA array analyses of hsa-miR-146a, hsa-miR-210, hsa-miR-22, and hsa-miR-24.

FIG. 55 represents the results of miRNA array analyses of hsa-miR-34b, hsa-miR-132, hsa-miR-203, hsa-miR-137, and hsa-miR-193.

FIG. 56 represents the results of miRNA array analyses of hsa-miR-26a/b, hsa-miR-23b, hsa-miR-27b, and hsa-miR-24-1.

FIG. 57 represents a comparison of the exosome production amount per cell between Comparative Example 1 (Dish Day 6) and Example 4 (Reactor (Normal Oxygen)_Day 27).

FIG. 58 represents the Scatterplot of the miRNA array analysis in Comparative Example 1 (Dish Day 6) and Example 4 (Reactor (Normal Oxygen)_Day 27).

FIG. 59 represents the results of miRNA array analyses of hsa-miR-146a, hsa-miR-210, hsa-miR-22, and hsa-miR-24.

FIG. 60 represents the results of miRNA array analyses of hsa-miR-34b, hsa-miR-132, hsa-miR-203, hsa-miR-137, and hsa-miR-193.

FIG. 61 represents the results of miRNA array analyses of hsa-miR-26a/b, hsa-miR-23b, hsa-miR-27b, and hsa-miR-24-1.

FIG. 62 represents a comparison of the exosome production amount per cell between Comparative Example 1 (Dish Day 13) and Example 4 (Reactor (Normal Oxygen)_Day 27).

FIG. 63 represents a Scatterplot of miRNA array analyses in Comparative Example 1 (Dish Day 13) and Example 4 (Reactor (Normal Oxygen)_Day 27).

FIG. 64 represents the results of miRNA array analyses of hsa-miR-146a, hsa-miR-210, hsa-miR-22, and hsa-miR-24.

FIG. 65 represents the results of miRNA array analyses of hsa-miR-34b, hsa-miR-132, hsa-miR-203, hsa-miR-137, and hsa-miR-193.

FIG. 66 represents the results of miRNA array analyses of hsa-miR-26a/b, hsa-miR-23b, hsa-miR-27b, and hsa-miR-24-1.

FIG. 67 represents changes over time in the lactic acid production amount and the glucose consumption.

FIG. 68 is a graph representing a particle size distribution of 5 kinds of exosome samples obtained.

FIG. 69 represents a transmission electron microscope image of exosomes obtained by a method of the present invention in one embodiment.

FIG. 70 is a graph representing the exosome production amounts over time in the various reactors.

FIG. 71 represents the results of miRNA array analyses (left, the overall view of the human-type miRNA; right, miR-21, 22, 23, and 24 selected).

FIG. 72 represents changes over time in the lactic acid production amount and the glucose consumption.

FIG. 73 represents the results of miRNA array analyses (left, the overall view of the human-type miRNA; right, miR-21, 22, 23, and 24 selected).

FIG. 74 represents changes over time in the lactic acid production amount and the glucose consumption.

FIG. 75 is a graph representing changes over time in the cell density (left) and total number of the human chondrocytes cultured using modules or porous membrane fragments.

FIG. 76 represents the results of miRNA array analyses (left, the overall view of the human-type miRNA; right, miR-21, 22, 23, and 24 selected).

FIG. 77 represents a use aspect of the WAVE reactor used in Example 5. Left: an HDPE cap with a liquid withdrawal tube, used for a WAVE culture bag. Right: a WAVE culture bag to which an HDPE cap with a liquid withdrawal tube is attached.

DESCRIPTION OF EMBODIMENTS

I. Method for Producing Exosome

The present invention relates to a method for producing an exosome using a cell. The method of the invention comprises: culturing a cell in a module constituted by a porous polymer membrane contained in a casing; and allowing the cell to produce an exosome.

1. Cell

A cell that can be utilized for the method of the present invention is not limited to any particular type so long as it may produce an exosome. It is known that an exosome is produced in various types of cells, and the profile of an exosome produced by a cell differs depending on the cell from which the exosome is derived and on the conditions for production. Accordingly, in the present invention, a cell that can produce an exosome with a desired quality and yield can be appropriately selected on the basis of an individual embodiment.

In one aspect in the present invention, a pluripotent stem cell is used as a culture cell. The term "pluripotent stem cells" is intended as a comprehensive term for stem cells having the ability to differentiate into cells of any tissues (pluripotent differentiating power). While not restrictive, pluripotent stem cells include embryonic stem cells (ES cells), induced pluripotent stem cells (iPS cells), embryonic germ cells (EG cells) and germ stem cells (GS cells). They are preferably ES cells or iPS cells. Particularly preferred are iPS cells, which are free of ethical problems, for example. The pluripotent stem cells used may be any publicly known ones, and for example, the pluripotent stem cells described in WO2009/123349 (PCT/JP2009/057041) may be used.

In one aspect in the present invention, a tissue stem cell is used as a culture cell. The term "tissue stem cells" refers to stem cells that are cell lines capable of differentiation but only to limited specific tissues, though having the ability to differentiate into a variety of cell types (pluripotent differentiating power). For example, hematopoietic stem cells in the bone marrow are the source of blood cells, while neural stem cells differentiate into neurons. Additional types include hepatic stem cells from which the liver is formed and skin stem cells that form skin tissue. Preferably, the tissue stem cells are selected from among mesenchymal stem cells, hepatic stem cells, pancreatic stem cells, neural stem cells, skin stem cells and hematopoietic stem cells.

In one aspect in the present invention, a somatic stem cell is used as a culture cell. The term "somatic cells" refers to cells other than germ cells, among the cells composing a multicellular organism. In sexual reproduction, these are not passed on to the next generation. Preferably, the somatic cells are selected from among hepatocytes, pancreatic cells, muscle cells, bone cells, osteoblasts, osteoclasts, chondrocytes, adipocytes, skin cells, fibroblasts, pancreatic cells, renal cells and lung cells, or blood cells such as lymphocytes, erythrocytes, leukocytes, monocytes, macrophages or megakaryocytes.

In one aspect in the present invention, a germ stem cell is used as a culture cell. The term "germ cells" refers to cells having the role of passing on genetic information to the succeeding generation in reproduction. These include, for example, gametes for sexual reproduction, i.e. the ova, egg cells, sperms, sperm cells, and spores for asexual reproduction.

The cells may also be selected from the group consisting of sarcoma cells, established cell lines and transformants. The term "sarcoma" refers to cancer occurring in non-epithelial cell-derived connective tissue cells, such as the bone, cartilage, fat, muscle or blood, and includes soft tissue sarcomas, malignant bone tumors and the like. Sarcoma cells are cells derived from sarcoma. The term "established cell line" refers to cultured cells that are maintained in vitro for long periods and reach a stabilized character and can be semi-permanently subcultured. Cell lines derived from various tissues of various species including humans exist, such as PC12 cells (from rat adrenal medulla), CHO cells (from Chinese hamster ovary), HEK293 cells (from human embryonic kidney), HL-60 cells (from human leukocytes) and HeLa cells (from human cervical cancer), Vero cells (from African green monkey kidney epithelial cells), MDCK cells (from canine renal tubular epithelial cells), and HepG2 cells (from human hepatic cancer). The term "transformants" refers to cells with an altered genetic nature by extracellularly introduced nucleic acid (DNA and the like).

It is known that an exosome is actively produced in an undifferentiated cell such as a stem cell or a cancer cell, and, if advantageous, a stem cell is used as a culture cell in the method of the invention.

2. Exosome

In the present invention, an exosome means a membranous vesicular structure, 50 to 300 nm, which a cell secretes out of the cell. The type of a cell from which an exosome is derived and the conditions for production have influence on the quality of the exosome, and thus, in the present invention, the conditions that make it possible to produce an exosome with a desired quality and yield can be selected appropriately. If advantageous, an evaluation is made on whether an exosome obtained has a quality suitable for its applications such as use as a drug delivery carrier or a bioactive substance.

3. Cell culture module

A cell culture module to be used in the present invention comprises:

a porous polymer membrane; and
a casing having two or more medium flow inlets/outlets and containing the porous polymer membrane,
wherein the porous polymer membrane is a three-layer structure porous polymer membrane having a surface layer A and a surface layer B, the surface layers having a plurality of pores, and a macrovoid layer sandwiched between the surface layers A and B,
wherein an average pore diameter of the pores present in the surface layer A is smaller than an average pore diameter of the pores present in the surface layer B,
wherein the macrovoid layer has a partition wall bonded to the surface layers A and B, and a plurality of macrovoids surrounded by the partition wall and the surface layers A and B,
wherein the pores in the surface layers A and B communicate with the macrovoid, and
wherein the porous polymer membrane is contained within the casing with:

(i) the two or more independent porous polymer membranes being aggregated;
(ii) the porous polymer membranes being folded up;
(iii) the porous polymer membranes being wound into a roll-like shape; and/or
(iv) the porous polymer membranes being tied together into a rope-like shape.

The cell culture module will be hereinafter referred to as a "cell culture module of the invention". In this specification, the phrase "a cell culture module" may be expressed simply as "a module", both expressions can be used interchangeably to indicate the same meaning.

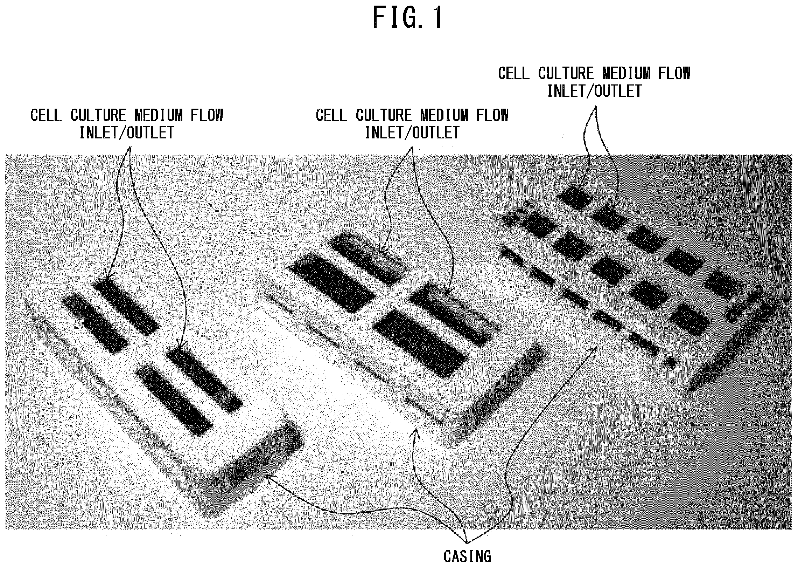

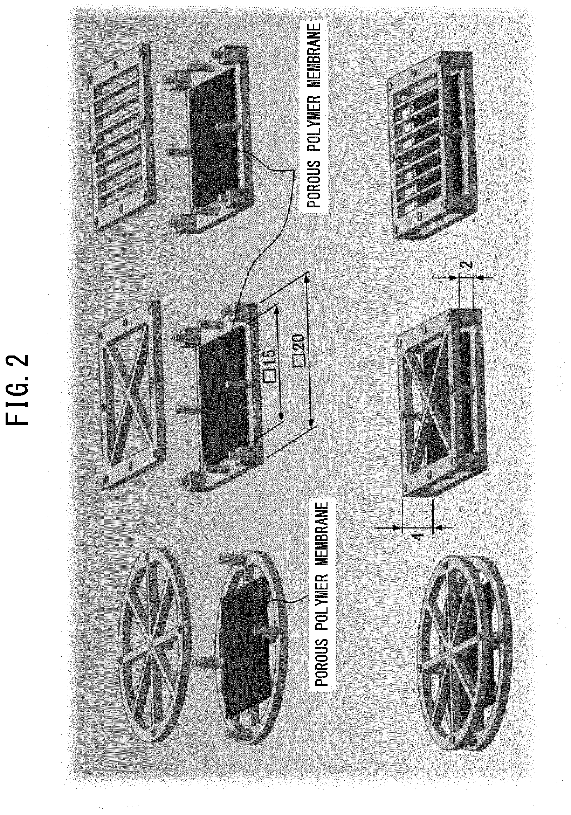

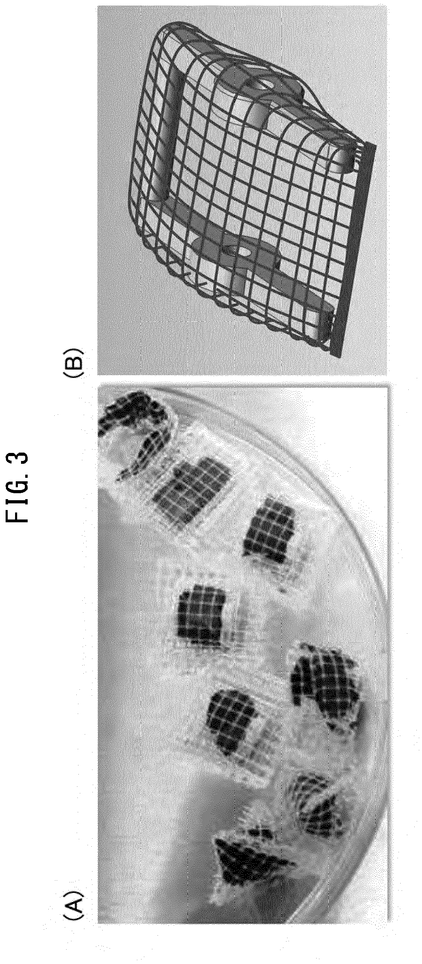

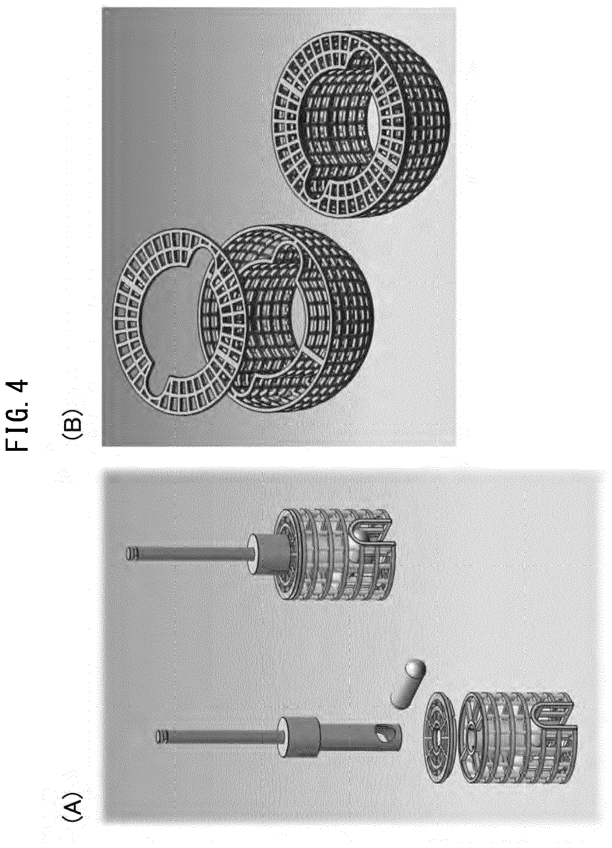

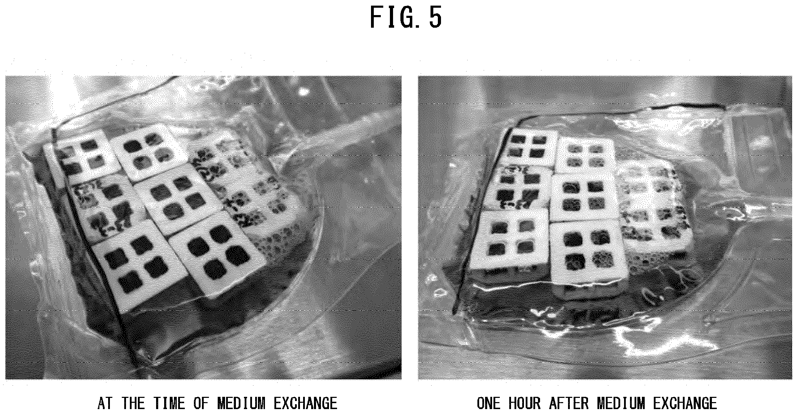

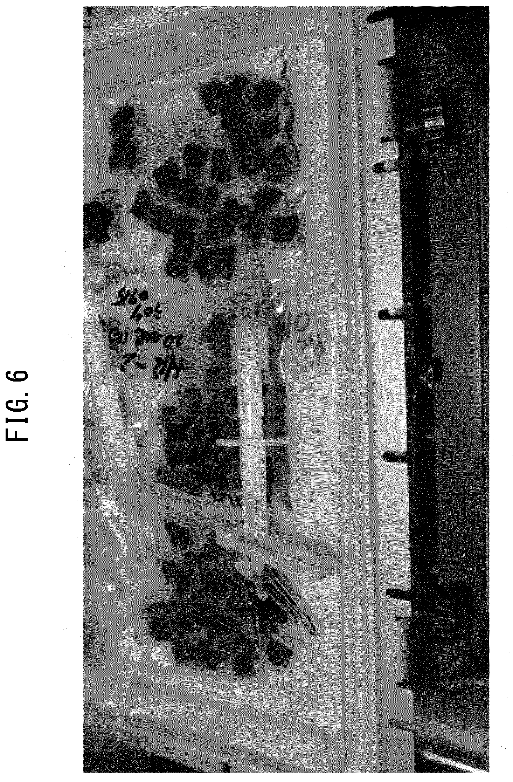

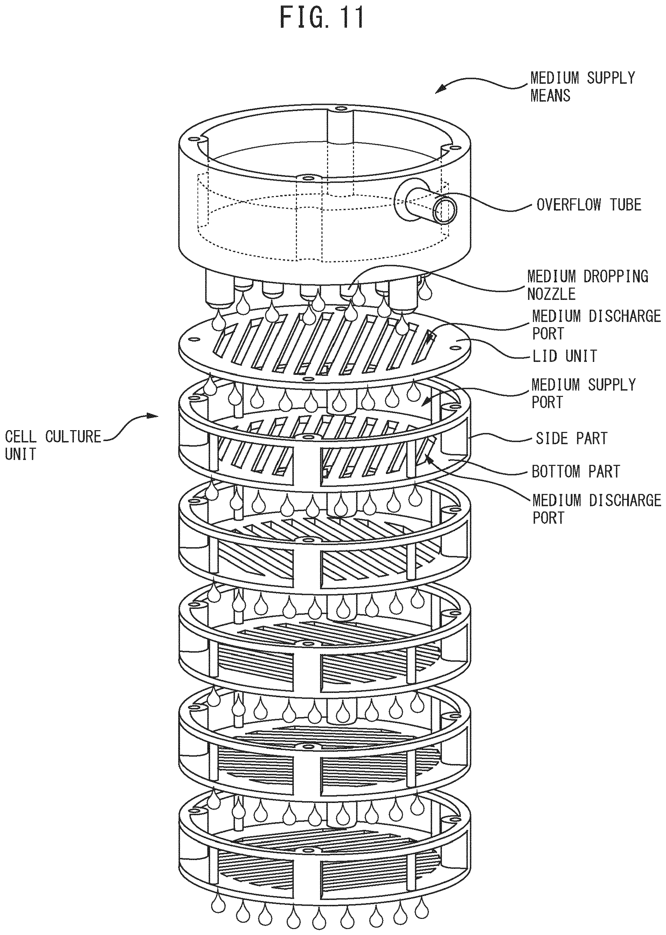

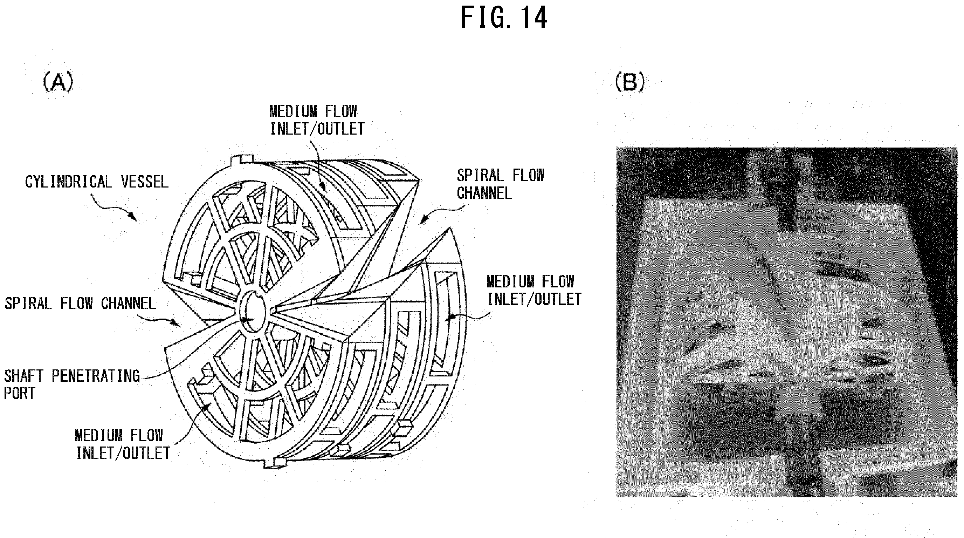

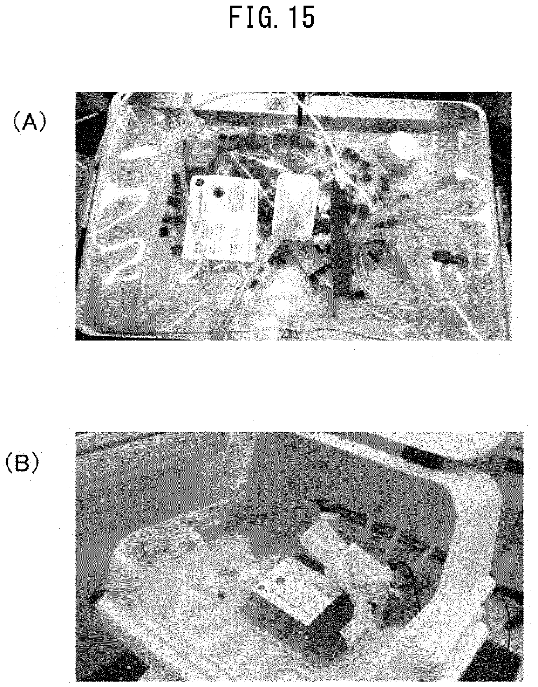

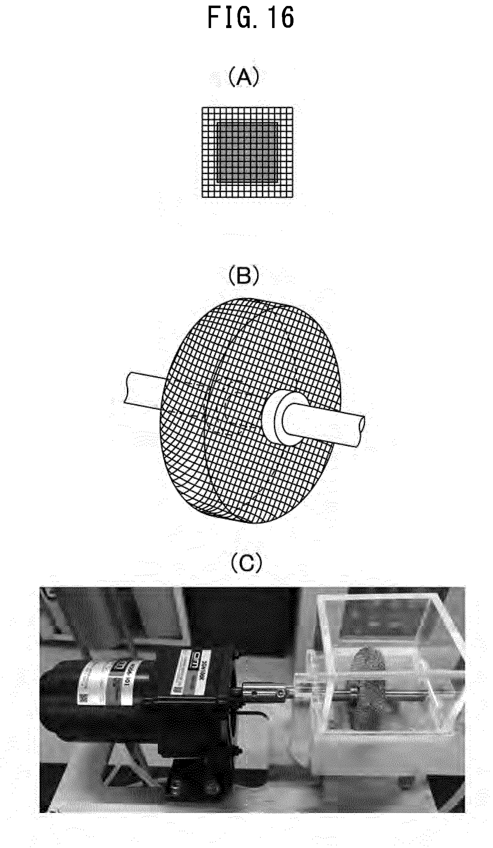

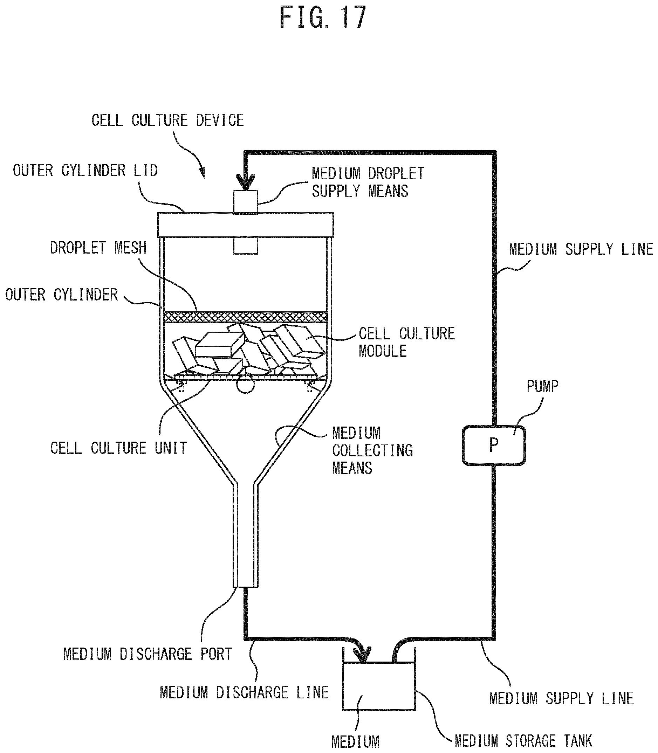

In this specification, the term "a cell culture module" refers to a cell culture substrate applicable to a cell culture vessel, cell culture device, and cell culture system, especially to a cell culture vessel, cell culture device and cell culture system which can be used for suspension culture. Several embodiments of a cell culture module are depicted in FIGs. 1 to 3, and 16 (A). The cell culture module of the invention may be used according to the embodiments such as in FIGs. 4 to 6 and 8 to 17. The cell culture module of the invention may also be used in the embodiments illustrated in Examples described below.

The cell culture module of the invention can prevent continuing morphological deformation of the membrane-like porous polymer membrane within a casing because of a porous polymer membrane being contained in the casing. This can protect cells to be grown in the porous polymer membrane from stress to be applied, resulting in suppression of apoptosis or the like and enabling a stable cell culture in a large amount.

A casing comprised in the cell culture module of the invention has two or more medium flow inlets/outlets, which let the cell culture medium be supplied into/discharge from the casing. The diameter of the medium flow inlet/outlet of the casing is preferably larger than the diameter of the cell so as to enable cell to flow into the casing. In addition, the diameter of the medium flow inlet/outlet is preferably smaller than the diameter through which the porous polymer membrane flows out from the medium flow inlet/outlet. The diameter smaller than the diameter through which the porous polymer membrane flows out may be appropriately selected depending on the shape and size of the porous polymer membrane contained in the casing. For example, when the porous polymer membrane has string-like shape, the diameter is not particularly limited so long as it is smaller than the width of the shorter side of the porous polymer membrane so that the porous polymer membrane is prevented from flowing out. It is preferred to provide as many medium flow inlets/outlets as possible so that the cell culture medium may be easily supplied into and/or discharged from the casing. It is preferably 5 or more, preferably 10 or more, preferably 20 or more, preferably 50 or more, and preferably 100 or more. As for the medium flow inlet/outlet, the casing may have a mesh-like structure in part or as a whole. Moreover, the casing itself may be mesh-like. In the present invention, examples of mesh-like structure include, but not limited to, those including longitudinal, transverse, and/or oblique elements wherein individual apertures form medium flow inlets/outlets which allow the fluid to pass therethrough.

It is preferred that the casing contained in the cell culture module of the invention has enough strength not to be deformed by movement of the culture medium under agitation culture, shaking culture conditions, and that casing is formed of a non-flexible material. Moreover, it is preferred that the casing is formed of a material which does not affect the growth of cells in cell culture. Examples of such materials include, for example, polymers such as polyethylene, polypropylene, nylon, polyester, polystyrene, polycarbonate, polymethyl methacrylate, polyethylene terephthalate; metals such as stainless steel, titanium, but not limited thereto. Having some strength in the casing prevents the shape of the porous polymer membrane inside the casing from continually being changed, and thus the effect of the present invention will be better exhibited. In this specification, "the casing is not deformed" means that the casing is not absolutely undeformable but is not deformed under load experienced in the ordinary culture environment.

The cell culture module of the present invention is contained within the casing with:

  (i) the two or more independent porous polymer membranes being aggregated;
  (ii) the porous polymer membranes being folded up;
  (iii) the porous polymer membranes being wound into a roll-like shape; and/or
  (iv) the porous polymer membranes being tied together into a rope-like shape.

In this specification, "two or more independent porous polymer membranes are aggregated and contained within a casing" means that two or more independent porous polymer membranes are aggregated and contained in a predetermined space surrounded by a casing. According to the present invention, the two or more independent porous polymer membranes may be immovably fixed by fixing at least one point of the porous polymer membrane to at least one point of the casing by an arbitrary method. In addition, the two or more independent porous polymer membranes may be fragments. The fragments may take any shape such as a circle, an ellipse, a square, a triangle, a polygon, a string, etc., but a substantially square shape is preferred. In the present invention, the fragments may be any size. When it has a substantially square shape, the side may be any length, but, for example, preferably 80 mm or less, preferably 50 mm or less, more preferably 30 mm or less, still more preferably 20 mm or less, and may be 10 mm or less. In addition, when the fragments of the porous polymer membrane are substantially square, it may be formed so that length of each side may match the inner wall or may be shorter than each side of the inner wall (e.g. shorter by about 0.1 mm to 1 mm), rendering the porous polymer membrane immovable in the casing. This can protect cells to be grown in the porous polymer membrane from stress to be applied, resulting in suppression of apoptosis or the like and enabling a stable cell culture in a large amount. The string-like porous polymer membrane may be contained within the casing, with: (ii) the porous polymer membranes being folded up; (iii) the porous polymer membranes being wound into a roll-like shape; and/or (iv) the porous polymer membrane being tied together into a rope-like shape, as described below. In addition, any number of the porous polymer membranes may be stacked to aggregate and contain the two or more independent porous polymer membranes in the casing. In this case, a liner may be provided between the porous polymer membranes. Providing a liner may enable efficient supply of a medium between the stacked porous polymer membranes. The liner may be not particularly limited so long as it may have function to form an arbitrary space between the stacked porous polymer membranes to efficiently supply medium. For example, a planar construct having a mesh structure may be used. As for material of the liner, for example, a mesh made of polystyrene, polycarbonate, polymethyl methacrylate, polyethylene terephthalate, stainless steel or the like may be used without limitation. When there is a liner having a mesh structure, the material may be appropriately selected so long as the mesh may have openings such that a medium may be supplied between the stacked porous polymer membranes.

In this specification, "the porous polymer membranes being folded up" means a porous polymer membrane which is folded up in the casing, and thus it is rendered immovable in the casing by frictional force between each surfaces of the porous polymer membrane and/or the inner surface of the casing. In this specification, "being folded" may indicate the pours polymer membrane being creased or creaseless.

In this specification, "the porous polymer membranes being wound into a roll-like shape" means the porous polymer membrane being wound into a roll-like shape and thus it is rendered immovable in the casing by frictional force between each surfaces of the porous polymer membrane and/or the inner surface of the casing. Moreover, in the present invention, the porous polymer membrane being tied together into a rope-like shape means, for example, more than one porous polymer membranes in rectangle strip shape are knitted into a rope-shape by arbitrary method, rendering the porous polymer membranes immovable by the mutual frictional force of the porous polymer membranes. It is also possible that (i) the two or more independent porous polymer membranes being aggregated; (ii) the porous polymer membranes being folded up; (iii) the porous polymer membranes being wound into a roll-like shape; and/or (iv) the porous polymer membrane being tied together into a rope-like shape may be combined and contained within a casing.

In this specification, "the porous polymer membrane being immovable in the casing" means that the porous polymer membrane is contained in the casing so that the porous polymer membrane is continually morphologically unchanged during culturing the cell culture module in the cell culture medium. In other words, the porous polymer membrane itself is continually prevented from waving by fluid. Since the porous polymer membrane is kept immovable in the casing, the cell growing in the porous polymer membrane is protected from stress to be applied, enabling stable cell culture without cells being killed by apoptosis.

As for the cell culture module of the invention, the commercially available product may be applied so long as it is a culture device, system etc. which may culture cells. For example, it is applicable to a culture device wherein a culture vessel is composed of a flexible bag, and can be used while it is suspended in the culture vessel. In addition, the cell culture module of the invention can be applied to and cultured in an agitating culture type vessel such as a spinner flask. In addition, as for a culture vessel, it may be applicable to an open type vessel, or it may be applicable to a closed type vessel. For example, any of a dish, a flask, plastic bag, test tube and large tank for cell culture may be used, as appropriate. These include, for example, Cell Culture Dish manufactured by BD Falcon, and Nunc Cell Factory manufactured by Thermo Scientific.

4. Application of cell culture module to cell culture device

In this specification, "cell culture device" is a term generally used synonymously for a cell culture system, bioreactor or reactor, and interchangeably used. The cell culture module of the invention is applicable to the cell culture device illustrated below. In addition, it is applicable to the commercially available devices other than devices illustrated below.

(1) Siphon type culture device

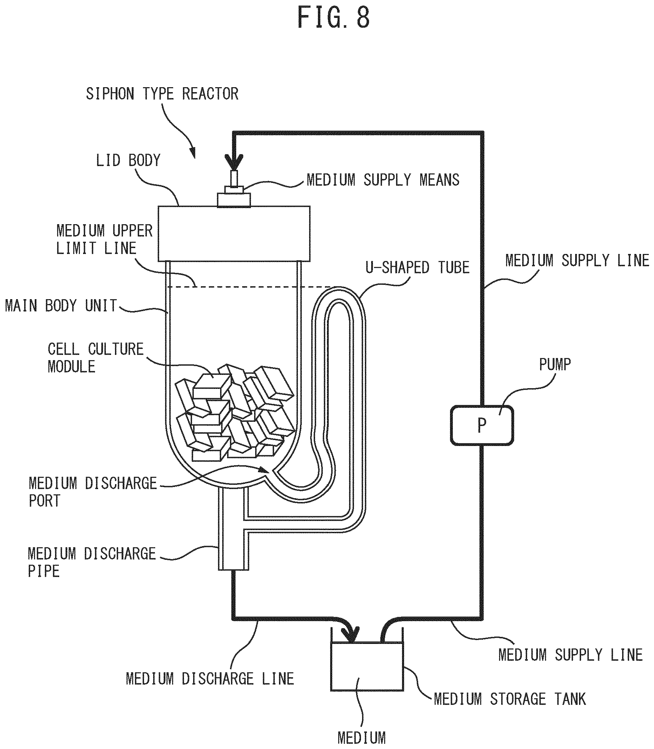

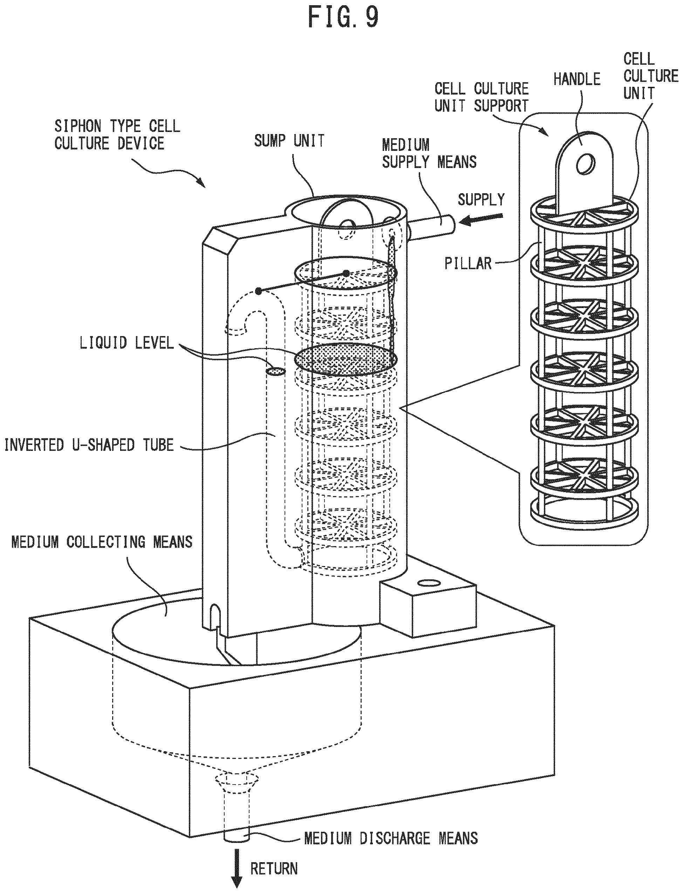

The cell culture module of the invention is applicable to a siphon type culture device depicted in FIGs. 8 and 9. A siphon type culture device is a cell culture device which is characterized by including a porous polymer membrane, a cell culture unit containing the porous polymer membrane, a sump unit containing the cell culture unit therein, a medium supply means placed at the upper part of the sump unit, an inverted U-shaped tube communicating with the bottom of the sump unit, a medium collecting means placed at the lower part of the other end of the inverted U-shaped tube, and a medium discharge means placed in the medium collecting means; wherein the porous polymer membrane is a three-layer structure porous polymer membrane having a surface layer A and a surface layer B, the surface layers having a plurality of pores, and a macrovoid layer sandwiched between the surface layers A and B; wherein an average pore diameter of the pores present in the surface layer A is smaller than an average pore diameter of the pores present in the surface layer B; wherein the macrovoid layer has a partition wall bonded to the surface layers A and B, and a plurality of macrovoids surrounded by the partition wall and the surface layers A and B; wherein the pores in the surface layers A and B communicate with the macrovoid; and wherein when the liquid level of the medium supplied into the sump unit from the medium supply means reaches the top of the inverted U-shape tube, the medium is intermittently discharged into the medium collecting means by the principle of siphon. The device can be used wherein the porous polymer membrane is exchanged with the cell culture module.

(2) Cylindrical type vapor phase culture device

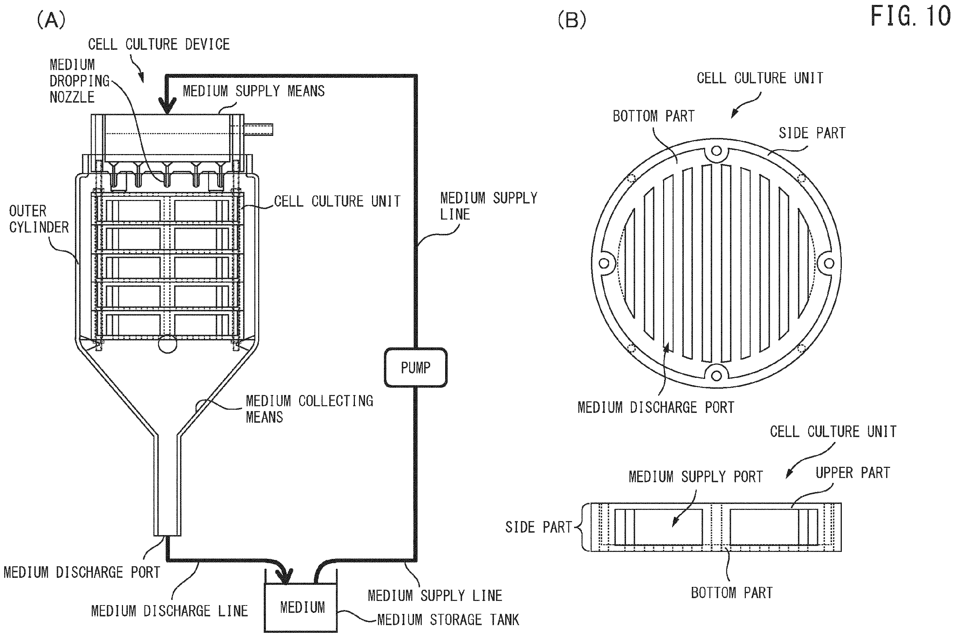

The cell culture module of the present invention is applicable to a cylindrical type vapor phase culture device depicted in FIGs. 10, 11 and 17. In an embodiment, a cylindrical vapor phase culture device is a cell culture device which includes a porous polymer membrane, a cell culture unit containing the porous polymer membrane, a medium supply means placed at the upper part of the cell culture unit, and a medium collecting means placed at the lower part of the cell culture unit; wherein the porous polymer membrane is a three-layer structure porous polymer membrane having a surface layer A and a surface layer B, the surface layers having a plurality of pores, and a macrovoid layer sandwiched between the surface layers A and B; wherein an average pore diameter of the pores present in the surface layer A is smaller than an average pore diameter of the pores present in the surface layer B; wherein the macrovoid layer has a partition wall bonded to the surface layers A and B, and a plurality of macrovoids surrounded by the partition wall and the surface layers A and B; wherein the cell culture unit is provided with a bottom part having one or more medium discharge port(s) and a side part arranged substantially vertical to the bottom part. In addition, in an embodiment, a cylindrical vapor phase culture device is a cell culture device which includes a porous polymer membrane, a cell culture unit containing the porous polymer membrane, a medium supply means placed at the upper part of the cell culture unit, and a medium collecting means placed at the lower part of the cell culture unit; wherein the porous polymer membrane is a three-layer structure porous polymer membrane having a surface layer A and a surface layer B, the surface layers having a plurality of pores, and a macrovoid layer sandwiched between the surface layers A and B; wherein an average pore diameter of the pores present in the surface layer A is smaller than an average pore diameter of the pores present in the surface layer B; wherein the macrovoid layer has a partition wall bonded to the surface layers A and B, and a plurality of macrovoids surrounded by the partition wall and the surface layers A and B; wherein the pores in the surface layers A and B communicate with the macrovoid; and wherein the medium collecting means is a part of the outer cylinder containing the cell culture unit. The device can be used wherein the porous polymer membrane is exchanged with the cell culture module.

(3) Mist/shower type culture device

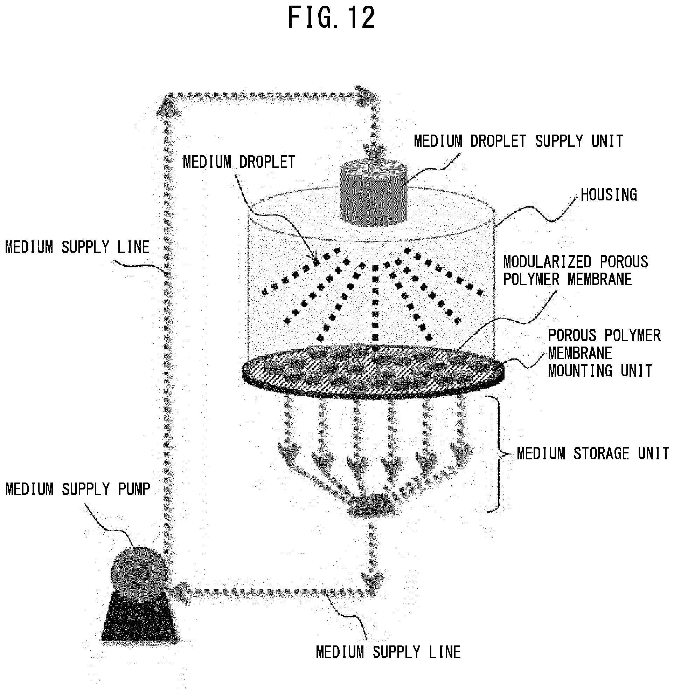

The cell culture module of the present invention is applicable to a mist/shower type culture device depicted in FIG. 12. A mist/shower type culture device is a cell culture device which includes: a porous polymer membrane, a porous polymer membrane mounting unit on which the porous polymer membrane is mounted, a housing containing the porous polymer membrane mounting unit, a medium droplet supply unit placed in the housing, a medium supply line communicating with the medium droplet supply unit, a medium storage unit communicating with the medium supply line, and a pump provided on a part of the medium supply line; wherein the porous polymer membrane is a three-layer structure porous polymer membrane having a surface layer A and a surface layer B, the surface layers having a plurality of pores, and a macrovoid layer sandwiched between the surface layers A and B; wherein an average pore diameter of the pores present in the surface layer A is smaller than an average pore diameter of the pores present in the surface layer B; wherein the macrovoid layer has a partition wall bonded to the surface layers A and B, and a plurality of macrovoids surrounded by the partition wall and the surface layers A and B; wherein the pores in the surface layers A and B communicate with the macrovoid; and wherein the porous polymer membrane mounting unit includes a plurality of slit- or mesh-like medium discharge ports. The device can be used wherein the porous polymer membrane is exchanged with the cell culture module.

(4) Vapor phase exposed type rotating culture device

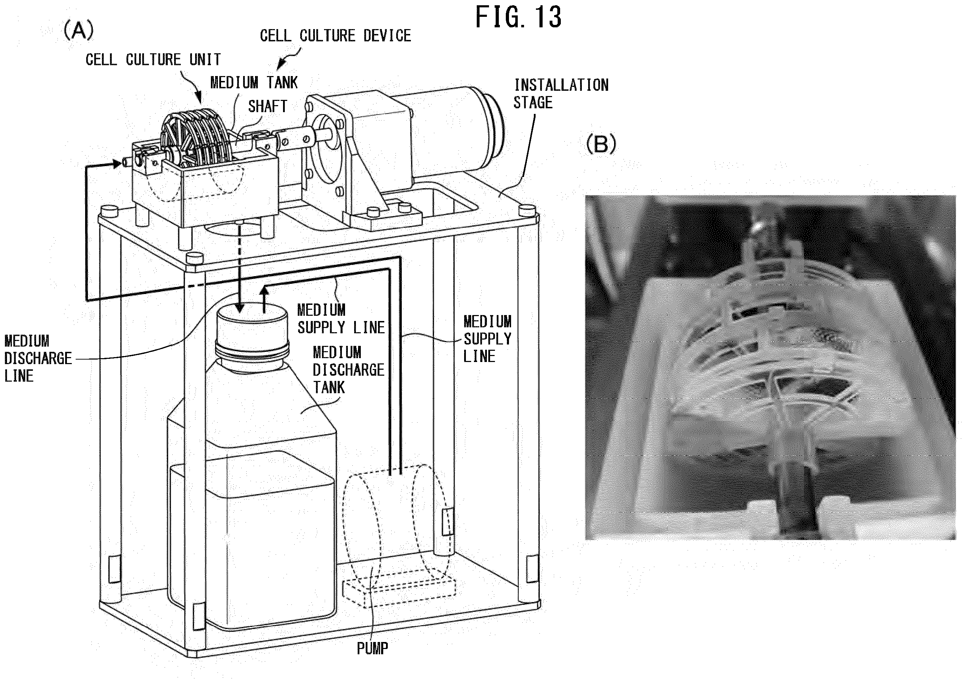

The cell culture module of the invention is applicable to a rotating culture device depicted in FIGs. 13, 14 and 16. The vapor phase exposed type rotating culture device is a cell culture device including a porous polymer membrane, a cell culture unit having the porous polymer membrane, a shaft penetrating the cell culture unit, a rotating motor to rotate the shaft; and a medium tank immersing at least a part of the cell culture unit; wherein the porous polymer membrane is a three-layer structure porous polymer membrane having a surface layer A and a surface layer B, the surface layers having a plurality of pores, and a macrovoid layer sandwiched between the surface layers A and B; wherein an average pore diameter of the pores present in the surface layer A is smaller than an average pore diameter of the pores present in the surface layer B; wherein the macrovoid layer has a partition wall bonded to the surface layers A and B, and a plurality of macrovoids surrounded by the partition wall and the surface layers A and B; wherein the pores in the surface layers A and B communicate with the macrovoid; and wherein the cell culture unit rotates around the shaft, and the cells supported on the porous polymer membrane are cultured alternately in a vapor phase and a liquid phase. The device can be used wherein the porous polymer membrane is exchanged with the cell culture module.

5. Porous polymer membrane

An average pore diameter of the pore present on a surface layer A (hereinafter referred to as "surface A" or "mesh surface") in the porous polymer membrane used for the present invention is not particularly limited, but is, for example, 0.01 $\mu$m or more and less than 200 $\mu$m, 0.01 to 150 $\mu$m, 0.01 to 100 $\mu$m, 0.01 to 50 $\mu$m, 0.01 to 40 $\mu$m, 0.01 to 30 $\mu$m, 0.01 to 20 $\mu$m, or 0.01 to 15 $\mu$m, preferably 0.01 to 15 $\mu$m.

The average pore diameter of the pore present on a surface layer B (hereinafter referred to as "surface B" or "large pore surface") in the porous polymer membrane used for the present invention is not particularly limited so long as it is larger than the average pore diameter of the pore present on the surface layer A, but is, for example, greater than 5 $\mu$m and 200 $\mu$m or less, 20 $\mu$m to 100 $\mu$m, 30 $\mu$m to 100 $\mu$m, 40 $\mu$m to 100 $\mu$m, 50 $\mu$m to 100 $\mu$m, or 60 $\mu$m to 100 $\mu$m, preferably 20 $\mu$m to 100 $\mu$m.

The average pore diameter on the surface of the porous polymer membrane can be determined by measuring pore area for 200 or more open pore portions, and calculated an average diameter according to the following Equation (1) from the average pore area assuming the pore shape as a perfect circle.

[Math. 1]

$$\text{Average Pore Diameter} = 2 \times \sqrt{(Sa/\pi)} \qquad (1)$$

(wherein Sa represents the average value for the pore areas)

The thicknesses of the surface layers A and B are not particularly limited, but is, for example, 0.01 to 50 $\mu$m, preferably 0.01 to 20 $\mu$m.

The average pore diameter of macrovoids in the planar direction of the membrane in the macrovoid layer in the porous polymer membrane is not particularly limited but is, for example, 10 to 500 $\mu$m, preferably 10 to 100 $\mu$m, and more preferably 10 to 80 $\mu$m. The thicknesses of the partition wall in the macrovoid layer are not particularly limited, but is, for example, 0.01 to 50 $\mu$m, preferably 0.01 to 20 $\mu$m. In an embodiment, at least one partition wall in the macrovoid layer has one or two or more pores connecting the neighboring macrovoids and having the average pore diameter of 0.01 to 100 $\mu$m, preferably 0.01 to 50 $\mu$m. In another embodiment, the partition wall in the macrovoid layer has no pore.

The total membrane thickness of the porous polymer membrane used for the invention is not particularly limited, but may be 5 $\mu$m or more, 10 $\mu$m or more, 20 $\mu$m or more or 25 $\mu$m or more, and 500 $\mu$m or less, 300 $\mu$m or less, 100 $\mu$m or less, 75 $\mu$m or less, or 50 $\mu$m or less. It is preferably 5 to 500 $\mu$m, and more preferably 25 to 75 $\mu$m.

The membrane thickness of the porous polymer membrane used for the invention can be measured using a contact thickness gauge.

The porosity of the porous polymer membrane used in the present invention is not particularly limited but is, for example, 40% or more and less than 95%.

The porosity of the porous polymer membrane used for the invention can be determined by measuring the membrane thickness and mass of the porous membrane cut out to a prescribed size, and performing calculation from the basis weight according to the following Equation (2).

[Math. 2]

$$\text{Porosity (\%)} = (1-w/(S \times d \times D)) \times 100 \qquad (2)$$

(wherein S represents the area of the porous membrane, d represents the total membrane thickness, w represents the measured mass, and D represents the polymer density. The density is defined as 1.34 g/cm$^3$ when the polymer is a polyimide.)

The porous polymer membrane used for the present invention is preferably a porous polymer membrane which includes a three-layer structure porous polymer membrane having a surface layer A and a surface layer B, the surface layers having a plurality of pores, and a macrovoid layer sandwiched between the surface layers A and B; wherein the average pore diameter of the pore present on the surface layer A is 0.01 $\mu$m to 15 $\mu$m, and the average pore diameter of the pore present on the surface layer B is 20 $\mu$m to 100 $\mu$m; wherein the macrovoid layer has a partition wall bonded to the surface layers A and B, and a plurality of macrovoids surrounded by the partition wall and the surface layers A and B, the thickness of the macrovoid layer, and the surface layers A and B is 0.01 to 20 $\mu$m; wherein the pores on the surface layers A and B communicate with the macrovoid, the total membrane thickness is 5 to 500 $\mu$m, and the porosity is 40% or more and less than 95%. In an embodiment, at least one partition wall in the macrovoide layer has one or two or more pores connecting the neighboring macrovoids with each other and having the average pore diameter of 0.01 to 100 $\mu$m, preferably 0.01 to 50 $\mu$m. In another embodiment, the partition wall does not have such pores.

The porous polymer membrane used for the present invention is preferably sterilized. The sterilization treatment is not particularly limited, but any sterilization treatment such as dry heat sterilization, steam sterilization, sterilization with a disinfectant such as ethanol, electromagnetic wave sterilization such as ultraviolet rays or gamma rays, and the like can be mentioned.

The porous polymer membrane used for the present invention is not particularly limited so long as it has the structural features described above and includes, preferably a porous polyimide membrane or porous polyethersulfone membrane.

5-1. Porous polyimide membrane

Polyimide is a general term for polymers containing imide bonds in the repeating unit, and usually it refers to an aromatic polyimide in which aromatic compounds are directly linked by imide bonds. An aromatic polyimide has an aromatic-aromatic conjugated structure via an imide bond, and therefore has a strong rigid molecular structure, and since the imide bonds provide powerful intermolecular force, it has very high levels of thermal, mechanical and chemical properties.

The porous polyimide membrane usable for the present invention is a porous polyimide membrane preferably containing polyimide (as a main component) obtained from tetracarboxylic dianhydride and diamine, more preferably a porous polyimide membrane composed of tetracarboxylic dianhydride and diamine. The phrase "including as the main component" means that it essentially contains no components other than the polyimide obtained from a tetracarboxylic dianhydride and a diamine, as constituent components of the porous polyimide membrane, or that it may contain them but they are additional components that do not affect the properties of the polyimide obtained from the tetracarboxylic dianhydride and diamine.

In an embodiment, the porous polyimide membrane usable for the present invention includes a colored porous polyimide membrane obtained by forming a polyamic acid solution composition including a polyamic acid solution obtained from a tetracarboxylic acid component and a diamine component, and a coloring precursor, and then heat treating it at 250°C or higher.

A polyamic acid is obtained by polymerization of a tetracarboxylic acid component and a diamine component. A polyamic acid is a polyimide precursor that can be cyclized to a polyimide by thermal imidization or chemical imidization.

The polyamic acid used may be any one that does not have an effect on the invention, even if a portion of the amic acid is imidized. Specifically, the polyamic acid may be partially thermally imidized or chemically imidized.

When the polyamic acid is to be thermally imidized, there may be added to the polyamic acid solution, if necessary, an imidization catalyst, an organic phosphorus-containing compound, or fine particles such as inorganic fine particles or organic fine particles. In addition, when the polyamic acid is to be chemically imidized, there may be added to the polyamic acid solution, if necessary, a chemical imidization agent, a dehydrating agent, or fine particles such as inorganic fine particles or organic fine particles. Even if such components are added to the polyamic acid solution, they are preferably added under conditions that do not cause precipitation of the coloring precursor.

In this specification, a "coloring precursor" is a precursor that generates a colored substance by partial or total carbonization under heat treatment at 250°C or higher.

Coloring precursors usable for the production of the porous polyimide membrane are preferably uniformly dissolved or dispersed in a polyamic acid solution or polyimide solution and subjected to thermal decomposition by heat treatment at 250°C or higher, preferably 260°C or higher, even more preferably 280°C or higher and more preferably 300°C or higher, and preferably heat treatment in the presence of oxygen such as air, at 250°C or higher, preferably 260°C or higher, even more preferably 280°C or higher and more preferably 300°C or higher, for carbonization to produce a colored substance, more preferably producing a black colored substance, with carbon-based coloring precursors being most preferred.

The coloring precursor, when being heated, first appears as a carbonized compound, but compositionally it contains other elements in addition to carbon, and also includes layered structures, aromatic crosslinked structures and tetrahedron carbon-containing disordered structures.

Carbon-based coloring precursors are not particularly restricted, and for example, they include tar or pitch such as petroleum tar, petroleum pitch, coal tar and coal pitch, coke, polymers obtained from acrylonitrile-containing monomers, ferrocene compounds (ferrocene and ferrocene derivatives), and the like. Of these, polymers obtained from acrylonitrile-containing monomers and/or ferrocene compounds are preferred, with polyacrylonitrile being preferred as a polymer obtained from an acrylonitrile-containing monomer.

Moreover, in another embodiment, examples of the porous polyimide membrane which may be used for the present invention also include a porous polyimide membrane which can be obtained by molding a polyamic acid solution derived from a tetracarboxylic acid component and a diamine component followed by heat treatment without using the coloring precursor.

The porous polyimide membrane produced without using the coloring precursor may be produced, for example, by casting a polyamic acid solution into a membrane, the polyamic acid solution being composed of 3 to 60% by mass of polyamic acid having an intrinsic viscosity number of 1.0 to 3.0 and 40 to 97% by mass of an organic polar solvent, immersing or contacting in a coagulating solvent containing water as an essential component, and imidating the porous membrane of the polyamic acid by heat treatment. In this method, the coagulating solvent containing water as an essential component may be water, or a mixed solution containing 5% by mass or more and less than 100% by mass of water and more than 0% by mass and 95% by mass or less of an organic polar solvent. Further, after the imidation, one surface of the resulting porous polyimide membrane may be subjected to plasma treatment.

The tetracarboxylic dianhydride which may be used for the production of the porous polyimide membrane may be any tetracarboxylic dianhydride, selected as appropriate according to the properties desired. Specific examples of tetracarboxylic dianhydrides include biphenyltetracarboxylic dianhydrides such as pyromellitic dianhydride, 3,3',4,4'-biphenyltetracarboxylic dianhydride (s-BPDA) and 2,3,3',4'-biphenyltetracarboxylic dianhydride (a-BPDA), oxydiphthalic dianhydride, diphenylsulfone-3,4,3',4'-tetracarboxylic dianhydride, bis(3,4-dicarboxyphenyl)sulfide dianhydride, 2,2-bis(3,4-dicarboxyphenyl)-1,1,1,3,3,3-hexafluoropropane dianhydride, 2,3,3',4'-benzophenonetetracarboxylic dianhydride, 3,3',4,4'-benzophenonetetracarboxylic dianhydride, bis(3,4-dicarboxyphenyl)methane dianhydride, 2,2-bis(3,4-dicarboxyphenyl)propane dianhydride, p-phenylenebis(trimellitic acid monoester acid anhydride), p-biphenylenebis(trimellitic acid monoester acid anhydride), m-terphenyl-3,4,3',4'-tetracarboxylic dianhydride, p-terphenyl-3,4,3',4'-tetracarboxylic dianhydride, 1,3-bis(3,4-dicarboxyphenoxy)benzene dianhydride, 1,4-bis(3,4-dicarboxyphenoxy)benzene dianhydride, 1,4-bis(3,4-dicarboxyphenoxy)biphenyl dianhydride, 2,2-bis[(3,4-dicarboxyphenoxy)phenyl]propane dianhydride, 2,3,6,7-naphthalenetetracarboxylic dianhydride, 1,4,5,8-naphthalenetetracarboxylic dianhydride, 4,4'-(2,2-hexafluoroisopropylidene)diphthalic dianhydride, and the like. Also preferably used is an aromatic tetracarboxylic acid such as 2,3,3',4'-diphenylsulfonetetracarboxylic acid. These may be used alone or in appropriate combinations of two or more.

Particularly preferred among these are at least one type of aromatic tetracarboxylic dianhydride selected from the group consisting of biphenyltetracarboxylic dianhydride and pyromellitic dianhydride. As a biphenyltetracarboxylic dianhydride there may be suitably used 3,3',4,4'-biphenyltetracarboxylic dianhydride.

As diamine which may be used for the production of the porous polyimide membrane, any diamine may be used. Specific examples of diamines include the following.

1) Benzenediamines with one benzene nucleus, such as 1,4-diaminobenzene(paraphenylenediamine), 1,3-diaminobenzene, 2,4-diaminotoluene and 2,6-diaminotoluene;

2) diamines with two benzene nuclei, including diaminodiphenyl ethers such as 4,4'-diaminodiphenyl ether and 3,4'-diaminodiphenyl ether, and 4,4'-diaminodiphenylmethane, 3,3'-dimethyl-4,4'-diaminobiphenyl, 2,2'-dimethyl-4,4'-diaminobiphenyl, 2,2'-bis(trifluoromethyl)-4,4'-diaminobiphenyl, 3,3'-dimethyl-4,4'-diaminodiphenylmethane, 3,3'-dicarboxy-4,4'-diaminodiphenylmethane, 3,3',5,5'-tetramethyl-4,4'-diaminodiphenylmethane, bis(4-aminophenyl)sulfide, 4,4'-diaminobenzanilide, 3,3'-dichlorobenzidine, 3,3'-dimethylbenzidine, 2,2'-dimethylbenzidine, 3,3'-dimethoxybenzidine, 2,2'-dimethoxybenzidine, 3,3'-diaminodiphenyl ether, 3,4'-diaminodiphenyl ether, 4,4'-diaminodiphenyl ether, 3,3'-diaminodiphenyl sulfide, 3,4'-diaminodiphenyl sulfide, 4,4'-diaminodiphenyl sulfide, 3,3'-diaminodiphenylsulfone, 3,4'-diaminodiphenylsulfone, 4,4'-diaminodiphenylsulfone, 3,3'-diaminobenzophenone, 3,3'-diamino-4,4'-dichlorobenzophenone, 3,3'-diamino-4,4'-dimethoxybenzophenone, 3,3'-diaminodiphenylmethane, 3,4'-diaminodiphenylmethane, 4,4'-diaminodiphenylmethane, 2,2-bis(3-aminophenyl)propane, 2,2-bis(4-aminophenyl)propane, 2,2-bis(3-aminophenyl)-1,1,1,3,3,3-hexafluoropropane, 2,2-bis(4-aminophenyl)-1,1,1,3,3,3-hexafluoropropane, 3,3'-diaminodiphenyl sulfoxide, 3,4'-diaminodiphenyl sulfoxide and 4,4'-diaminodiphenyl sulfoxide;

3) diamines with three benzene nuclei, including 1,3-bis(3-aminophenyl)benzene, 1,3-bis(4-aminophenyl)benzene, 1,4-bis(3-aminophenyl)benzene, 1,4-bis(4-aminophenyl)benzene, 1,3-bis(4-aminophenoxy)benzene, 1,4-bis(3-aminophenoxy)benzene, 1,4-bis(4-aminophenoxy)benzene, 1,3-bis(3-aminophenoxy)-4-trifluoromethylbenzene, 3,3'-diamino-4-(4-phenyl)phenoxybenzophenone, 3,3'-diamino-4,4'-di(4-phenylphenoxy)benzophenone, 1,3-bis(3-aminophenyl sulfide)benzene, 1,3-bis(4-aminophenyl sulfide)benzene, 1,4-bis(4-aminophenyl sulfide)benzene, 1,3-bis(3-aminophenylsulfone)benzene, 1,3-bis(4-aminophenylsulfone)benzene, 1,4-bis(4-aminophenylsulfone)benzene, 1,3-bis[2-(4-aminophenyl)isopropyl]benzene, 1,4-bis[2-(3-aminophenyl)isopropyl]benzene and 1,4-bis[2-(4-aminophenyl)isopropyl]benzene;

4) diamines with four benzene nuclei, including 3,3'-bis(3-aminophenoxy)biphenyl, 3,3'-bis(4-aminophenoxy)biphenyl, 4,4'-bis(3-aminophenoxy)biphenyl, 4,4'-bis(4-aminophenoxy)biphenyl, bis[3-(3-aminophenoxy)phenyl]ether, bis[3-(4-aminophenoxy)phenyl]ether, bis[4-(3-aminophenoxy)phenyl]ether, bis[4-(4-aminophenoxy)phenyl]ether, bis[3-(3-aminophenoxy)phenyl]ketone, bis[3-(4-aminophenoxy)phenyl]ketone, bis[4-(3-aminophenoxy)phenyl]ketone, bis[4-(4-aminophenoxy)phenyl]ketone, bis[3-(3-aminophenoxy)phenyl]sulfide, bis[3-(4-aminophenoxy)phenyl]sulfide, bis[4-(3-aminophenoxy)phenyl]sulfide, bis[4-(4-aminophenoxy)phenyl]sulfide, bis[3-(3-aminophenoxy)phenyl]sulfone, bis[3-(4-aminophenoxy)phenyl]sulfone, bis[4-(3-aminophenoxy)phenyl]sulfone, bis[4-(4-aminophenoxy)phenyl] sulfone, bis[3-(3-aminophenoxy)phenyl]methane, bis[3-(4-aminophenoxy)phenyl]methane, bis[4-(3-aminophenoxy)phenyl]methane, bis[4-(4-aminophenoxy)phenyl]methane, 2,2-bis[3-(3-aminophenoxy)phenyl]propane, 2,2-bis[3-(4-aminophenoxy)phenyl]propane, 2,2-bis[4-(3-aminophenoxy)phenyl]propane, 2,2-bis[4-(4-aminophenoxy)phenyl]propane, 2,2-bis[3-(3-aminophenoxy)phenyl]-1,1,1,3,3,3-hexafluoropropane, 2,2-bis[3-(4-aminophenoxy)phenyl]-1,1,1,3,3,3-hexafluoropropane, 2,2-bis[4-(3-aminophenoxy)phenyl]-1,1,1,3,3,3-hexafluoropropane and 2,2-bis[4-(4-aminophenoxy)phenyl]-1,1,1,3,3,3-hexafluoropropane.

These may be used alone or in mixtures of two or more. The diamine used may be appropriately selected according to the properties desired.

Preferred among these are aromatic diamine compounds, with 3,3'-diaminodiphenyl ether, 3,4'-diaminodiphenyl ether, 4,4'-diaminodiphenyl ether, paraphenylenediamine, 1,3-bis(3-aminophenyl)benzene, 1,3-bis(4-aminophenyl)benzene, 1,4-bis(3-aminophenyl)benzene, 1,4-bis(4-aminophenyl)benzene, 1,3-bis(4-aminophenoxy)benzene and 1,4-bis(3-aminophenoxy)benzene being preferred for use. Particularly preferred is at least one type of diamine selected from the group consisting of benzenediamines, diaminodiphenyl ethers and bis(aminophenoxy)phenyl.

From the viewpoint of heat resistance and dimensional stability under high temperature, the porous polyimide membrane which may be used for the invention is preferably formed from a polyimide obtained by combination of a tetracarboxylic dianhydride and a diamine, having a glass transition temperature of 240°C or higher, or without a distinct transition point at 300°C or higher.

From the viewpoint of heat resistance and dimensional stability under high temperature, the porous polyimide membrane which may be used for the invention is preferably a porous polyimide membrane comprising one of the following aromatic polyimides.

(i) An aromatic polyimide comprising at least one tetracarboxylic acid unit selected from the group consisting of biphenyltetracarboxylic acid units and pyromellitic acid units, and an aromatic diamine unit,
(ii) an aromatic polyimide comprising a tetracarboxylic acid unit and at least one type of aromatic diamine unit selected from the group consisting of benzenediamine units, diaminodiphenyl ether units and bis(aminophenoxy)phenyl units,

and/or,

(iii) an aromatic polyimide comprising at least one type of tetracarboxylic acid unit selected from the group consisting of biphenyltetracarboxylic acid units and pyromellitic acid units, and at least one type of aromatic diamine unit selected from the group consisting of benzenediamine units, diaminodiphenyl ether units and bis(aminophenoxy)phenyl units.

The porous polyimide membrane used in the present invention is preferably a three-layer structure porous polyimide membrane having a surface layer A and a surface layer B, the surface layers having a plurality of pores, and a macrovoid layer sandwiched between the surface layers A and B; wherein an average pore diameter of the pores present in the surface layer A is 0.01 $\mu$m to 15 $\mu$m, and the mean pore diameter present on the surface layer B is 20 $\mu$m to 100 $\mu$m; wherein the macrovoid layer has a partition wall bonded to the surface layers A and B, and a plurality of macrovoids surrounded by the partition wall and the surface layers A and B; wherein the thickness of the macrovoid layer, and the surface layers A and B is 0.01 to 20 $\mu$m, wherein the pores on the surface layers A and B communicate with the macrovoid, the total membrane thickness is 5 to 500 $\mu$m, and the porosity is 40% or more and less than 95%. In this case, at least one partition wall in the macrovoid layer has one or two or more pores connecting the neighboring macrovoids and having the average pore diameter of 0.01 to 100 $\mu$m, preferably 0.01 to 50 $\mu$m.

For example, porous polyimide membranes described in WO2010/038873, Japanese Unexamined Patent Publication (Kokai) No. 2011-219585 or Japanese Unexamined Patent Publication (Kokai) No. 2011-219586 may be used for the present invention.

5-2. Porous polyethersulfone membrane (Porous PES membrane)

The porous polyethersulfone membrane which may be used for the present invention contains polyethersulfone and typically consists substantially of polyethersulfone. Polyethersulfone may be synthesized by the method known to those skilled in the art. For example, it may be produced by a method wherein a dihydric phenol, an alkaline metal compound and a dihalogenodiphenyl compound are subjected to polycondensation reaction in an organic polar solvent, a method wherein an alkaline metal di-salt of a dihydric phenol previously synthesized is subjected to polycondensation reaction dihalogenodiphenyl compound in an organic polar solvent or the like.

Examples of an alkaline metal compound include alkaline metal carbonate, alkaline metal hydroxide, alkaline metal hydride, alkaline metal alkoxide and the like. Particularly, sodium carbonate and potassium carbonate are preferred.

Examples of a dihydric phenol compound include hydroquinone, catechol, resorcin, 4,4'-biphenol, bis (hydroxyphenyl)alkanes (such as 2,2-bis(hydroxyphenyl)propane, and 2,2-bis(hydroxyphenyl)methane), dihydroxydiphenylsulfones, dihydroxydiphenyl ethers, or those mentioned above having at least one hydrogen on the benzene rings thereof substituted with a lower alkyl group such as a methyl group, an ethyl group, or a propyl group, or with a lower alkoxy group such as a methoxy group, or an ethoxy group. As the dihydric phenol compound, two or more of the aforementioned compounds may be mixed and used.

Polyethersulfone may be a commercially available product. Examples of a commercially available product include SUMIKAEXCEL 7600P, SUMIKAEXCEL 5900P (both manufactured by Sumitomo Chemical Company, Limited).

The logarithmic viscosity of the polyethersulfone is preferably 0.5 or more, more preferably 0.55 or more from the viewpoint of favorable formation of a macrovoid of the porous polyethersulfone membrane; and it is preferably 1.0 or less, more preferably 0.9 or less, further preferably 0.8 or less, particularly preferably 0.75 or less from the viewpoint of the easy production of a porous polyethersulfone membrane.

Further, from the viewpoints of heat resistance and dimensional stability under high temperature, it is preferred that the porous polyethersulfone membrane or polyethersulfone as a raw material thereof has a glass transition temperature of 200°C or higher, or that a distinct glass transition temperature is not observed.

The method for producing the porous polyethersulfone membrane which may be used for the present invention is not particularly limited.

For example, the membrane may be produced by a method including the following steps: a step in which polyethersulfone solution containing 0.3 to 60% by mass of polyethersulfone having logarithmic viscosity of 0.5 to 1.0 and 40 to 99.7% by mass of an organic polar solvent is casted into a membrane, immersed in or contacted with a coagulating solvent containing a poor solvent or non-solvent of polyethersulfone to produce a coagulated membrane having pores; and

a step in which the coagulated membrane having pores obtained in the above-mentioned step is heat-treated for coarsening of the aforementioned pores to obtain a porous polyethersulfone membrane;

wherein the heat treatment includes the temperature of the coagulated membrane having the pores is raised higher than the glass transition temperature of the polyethersulfone, or up to 240°C or higher.

The porous polyethersulfone membrane which can be used in the present invention is preferably a porous polyethersulfone membrane having a surface layer A, a surface layer B, and a macrovoid layer sandwiched between the surface layers A and B,

wherein the macrovoid layer has a partition wall bonded to the surface layers A and B, and a plurality of macrovoids surrounded by the partition wall and the surface layers A and B, the macrovoids having the average pore diameter in the planar direction of the membrane of 10 to 500 μm;
wherein the thickness of the macrovoid layer is 0.1 to 50 μm,
each of the surface layers A and B has a thickness of 0.1 to 50 μm,
wherein one of the surface layers A and B has a plurality of pores having the average pore diameter of more than 5 μm and 200 μm or less, while the other has a plurality of pores having the average pore diameter of 0.01 μm or more and less than 200 μm,
wherein one of the surface layers A and B has a surface aperture ratio of 15% or more while other has a surface aperture ratio of 10% or more,
wherein the pores of the surface layers A and B communicate with the macrovoids,
wherein the porous polyethersulfone membrane has total membrane thickness of 5 to 500 μm and a porosity of 50 to 95%.

6. Applying cell to cell culture module

A specific step of applying a cell to a cell culture module is not particularly limited. It is possible to adopt the step described in this specification, or any approach suitable for applying a cell to a carrier in membrane form. In a method of the present invention, applying a cell to a cell culture module includes, but is not limited to, the following aspect.

(1) applying a first medium containing a cell in a cell suspension to a cell culture module;
(2) maintaining the cell culture module at a temperature at which the cell can be cultured, and allowing the cell to be adsorbed onto the porous polymer membrane in the cell culture module, and
(3) culturing the cell culture module having the cell adsorbed thereto, in a second medium in a culture vessel.

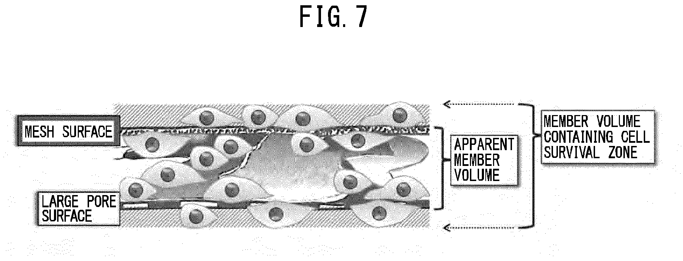

FIG. 7 represents a model diagram of cell culturing using a cell culture module. FIG. 7 serves merely for illustration and the elements are not drawn to their actual dimensions. In the cell culture method of the invention, application of cells and culturing are carried out on a cell culture module, thereby allowing simple culturing of large volumes of cells to be accomplished since large numbers of cells grow on the multisided connected pore sections on the inside, and the surfaces on the porous polymer membrane. Moreover, in the cell culture method of the invention, it is possible to culture large volumes of cells while drastically reducing the amount of medium used for cell culturing compared to the prior art. For example, large volumes of cells can be cultured over a long period of time even when all or a portion of the porous polymer membrane is not in contact with the liquid phase of the cell culture medium. In addition, the total volume of the cell culture medium in the cell culture vessel, with respect to the total porous polymer membrane volume including the cell survival zone, can be significantly reduced.

Throughout the present specification, the volume of the porous polymer membrane without cells, that occupies the space including the volume between the interior gaps, will be referred to as the "apparent porous polymer membrane volume" (see, FIG. 7). In the state where the cells are applied to the porous polymer membrane and the cells have been supported on the surface and the interior of the porous polymer membrane, the total volume of the porous polymer membrane, the cells and the medium that has wetted the porous polymer membrane interior, which is occupying the space therein, will be referred to as the "porous polymer membrane volume including the cell survival zone" (see, FIG. 1). When the porous polymer membrane has a membrane thickness of 25 $\mu$m, the porous polymer membrane volume including the cell survival zone is a value of at maximum about 50% larger than the apparent porous polymer membrane volume. In the method of the invention, a plurality of porous polymer membranes may be housed in a single cell culture vessel for culturing, in which case the total sum of the porous polymer membrane volume including the cell survival zone for each of the plurality of porous polymer membranes supporting the cells may be referred to simply as the "total sum of the porous polymer membrane volume including the cell survival zone".

Using the cell culture module, cells can be satisfactorily cultured for a long period of time even under conditions in which the total volume of the cell culture medium in the cell culture vessel is up to 10,000 times the total sum of the porous polymer membrane volume including the cell survival zone. Moreover, cells can be satisfactorily cultured for a long period of time even under conditions in which the total volume of the cell culture medium in the cell culture vessel is up to 1,000 times the total sum of the porous polymer membrane volume including the cell survival zone. In addition, cells can be satisfactorily cultured for a long period of time even under conditions in which the total volume of the cell culture medium in the cell culture vessel is up to 100 times the total sum of the porous polymer membrane volume including the cell survival zone. In addition, cells can be satisfactorily cultured for a long period of time even under conditions in which the total volume of the cell culture medium in the cell culture vessel is up to 10 times the total sum of the porous polymer membrane volume including the cell survival zone.

In other words, according to the invention, the space (vessel) used for cell culturing can be reduced to an absolute minimum, compared to a conventional cell culture device for performing two-dimensional culture. Furthermore, when it is desired to increase the number of cells cultured, the cell culturing volume can be flexibly increased by a convenient procedure including increasing the number of layered porous polymer membranes. In a cell culture device comprising a porous polymer membrane to be used for the invention, the space (vessel) in which cells are cultured and the space (vessel) in which the cell culture medium is stored can be separate, and the necessary amount of cell culture medium can be prepared according to the number of cells to be cultured. The space (vessel) in which the cell culture medium is stored can be increased or decreased according to the purpose, or it may be a replaceable vessel, with no particular restrictions.

In the method of the invention, culture can be performed until the number of cells in the cell culture vessel after culturing using the porous polymer membrane reaches $1.0 \times 10^5$ or more, $1.0 \times 10^6$ or more, $2.0 \times 10^6$ or more, $5.0 \times 10^6$ or more, $1.0 \times 10^7$ or more, $2.0 \times 10^7$ or more, $5.0 \times 10^7$ or more, $1.0 \times 10^8$ or more, $2.0 \times 10^8$ or more, $5.0 \times 10^8$ or more, $1.0 \times 10^9$ or more, $2.0 \times 10^9$ or more, or $5.0 \times 10^9$ or more per milliliter of medium, assuming that all of the cells are evenly dispersed in the cell culture medium in the cell culture vessel.

In this specification, a "medium" refers to a cell culture medium for culturing cells, especially animal cells. The term "medium" is interchangeably used as "cell culture solution". Accordingly, the medium used in the invention refers to a liquid medium. As for types of a medium, the conventionally used medium may be used and appropriately selected depending on the types of cells to be cultured.

In the cell culture in the invention, the first medium used in the step (1) is not particularly limited so long as it may culture cells. For example, in the case of culturing CHO cell, BalanCD (Trademark) CHO GROWH A (manufactured by Fujifilm Irvine Scientific, Inc.) may be used.

In the cell culture in the invention, a temperature at which cell culture may be performed in the step (2) may be any temperature at which cells may be adsorbed onto a porous polymer membrane, for example 10 to 45°C, preferably 15 to 42°C, more preferably 20 to 40°C, still more preferably 25 to 39°C. In addition, in the cell culture method of the invention, a time for cells to be adsorbed in the step (2) is, for example, 5 minutes to 24 hours, preferably 10 minutes to 12 hours, more preferably 15 minutes to 500 minutes.

In the cell culture in the invention, in the step (2), the cells may be adsorbed to the porous polymer membrane of the cell culture module with shaking and/or stirring, or cells may be adsorbed to the porous polymer membrane of the cell culture module while being stood still. The method for shaking is not particularly limited. For example, a culture vessel containing the cell culture module of the invention and cells is mounted and shaken on a commercially available shaking device. Shaking may be performed continuously or intermittently. For example, shaking and standing still are alternately repeated and adjusted as appropriate. The method for stirring is not particularly limited. For example, the cell culture module of the invention and cells are placed in a commercially available spinner flask and stirred by rotating a stirrer. Stirring may be performed continuously or intermittently. For example, stirring and standing still are alternately repeated and adjusted as appropriate.

In the cell culture in the invention, as the second medium used in the step (3), a medium used for culturing of adherent cells may be selected. For example, D-MEM, E-MEM, IMDM, Ham's F-12 and the like may be used, but not limited to them. The second medium is preferably a medium free from a component which prevents a cell from adhering to a substrate, such as a surfactant. The second medium used may be appropriately selected depending on the types of cells. In the step (3), culturing in the second medium facilitates the cells which is adsorbed onto the porous polymer membrane in the step (2) to adhere in the porous polymer membrane. Accordingly, cells may be stably cultured without being detached from the porous polymer membrane. In addition, the cell culture method of the invention utilizes the cell culture module provided with the porous polymer membrane described above. The cell culture module used in the cell culture method of the present invention can prevent continuing morphological deformation of the membrane-like porous polymer membrane within a casing because of containing a porous polymer membrane in the casing. This can protect cells to be grown in the porous polymer membrane from stress to be applied, resulting in suppression of apoptosis or the like and enabling a stable cell culture in a large amount.

In the cell culture in the invention, the step (3) may utilize any commercially available product so long as it is a culture device and system which may culture cells. For example, the culture vessel may be a flexible bag-type culture vessel. In addition, culture may be performed in a stirring type culture vessel such as spinner flask as a culture vessel. In addition, an open type vessel may be applicable, and a closed type vessel may be applicable, as a culture vessel. For example, any of a dish, flask, plastic bag, test tube and large tank for cell culturing may be used, as appropriate. These include, for example, Cell Culture Dish manufactured by BD Falcon, and Nunc Cell Factory manufactured by Thermo Scientific. In addition, in the cell culture method of the present invention, by using a cell culture module, it has become possible to carry out culturing even of cells that have not been capable of natural suspension culture, using a device intended for suspension culture, in a state similar to suspension culturing. The device for suspension culture that is used may be, for example, a spinner flask or rotating culturing flask manufactured by Corning, Inc. In addition, the step (3) may be performed in a cell culture device described in this specification.

In the cell culture in the invention, the step (3) may be performed using a continuously circulating type device in which a medium is continuously added to and collected from a culture vessel containing the cell culture module.

In the cell culture in the present invention, the step (3) may be a system in a system in which a cell culture medium is continuously or intermittently supplied to a cell culture vessel from cell culture medium supply means installed outside of the culture vessel containing the cell culture module. In this case, the system may be such that the cell culture medium is circulated between the cell culture medium supply means and the cell culture vessel.

7. Cell-culture/exosome-production system and culture conditions

In the present invention, a cell-culture/exosome-production system and culture conditions can be appropriately selected in accordance with, for example, the type of a cell. A culture method suitable for each type of cell is known, and a person skilled in the art can use any known method to culture cells applied to a cell culture module. A cell culture medium can also be prepared appropriately in accordance with the type of the cell.

In the present invention, a system used for culturing is not limited to any particular shape or scale, and any of a dish, flask, plastic bag, test tube and large tank for cell culturing may be utilized, as appropriate. These include, for example, Cell Culture Dish manufactured by BD Falcon, and Nunc Cell Factory manufactured by Thermo Scientific. In this regard, in the present invention, by using a porous polyimide membrane, it has become possible to carry out culturing even of cells that have not been capable of natural suspension culture, using a device intended for suspension culture, in a state similar to suspension culturing. The device for suspension culture that is used may be, for example, a spinner flask or rotating culturing flask manufactured by Corning, Inc.

In the present invention, a cell may be cultured under stationary culture conditions. Exchanging a culture medium intermittently makes it possible to isolate the exosome produced. Alternatively, in the present invention, a cell may be cultured under rotating or stirring culture conditions. Stirring culture with a spinner flask for rotating culture is also possible. In addition, it is possible to combine each of these methods with a continuous or intermittent culture medium exchange system, aiming for long-term culture.

In the present invention, a cell may be cultured continuously. For example, culture in the method of the invention may also be performed using a continuously circulating type or open type device in which a medium is continuously added to and collected from a cell culture module and in which a porous polymer membrane is exposed to the air.

In the present invention, cell culturing may be performed in a system in which a cell culture medium is continuously or intermittently supplied to a cell culture vessel from cell culture medium supply means installed outside of the cell culture vessel. In this case, the system may be such that the cell culture medium is circulated between the cell culture medium supply means and the cell culture vessel.

The present invention includes an aspect including placing a cell culture device in an incubator, where a cell is cultured. In cases where culture is performed in a system in which a cell culture medium is continuously or intermittently supplied to a cell culture vessel from cell culture medium supply means installed outside of the cell culture vessel, the system may be a cell culture device comprising a culture unit as the cell culture vessel and a culture medium supply unit as the cell culture medium supply means; wherein the culture unit contains one or more cell culture modules configured to support the cell, and includes a medium supply port and a medium discharge port; wherein the culture medium supply unit includes: a culture medium storage container; a medium supply line; and a liquid-transfer pump configured to liquid-transfer a culture medium via the medium supply line; and wherein a first end of the medium supply line is in contact with the culture medium in the culture medium storage container, and a second end of the medium supply line communicates with the culture unit via the medium supply port of the culture unit.

Additionally, in the cell culture device, the culture unit may be a culture unit that does not comprise a medium supply line, liquid-transfer pump, air supply port, and air discharge port, or may be a culture unit that comprises a medium supply line, liquid-transfer pump, air supply port, and air discharge port. The culture unit may be that which comprises neither an air supply port nor an air discharge port. Furthermore, in the cell culture device, the culture unit may further comprise a culture medium discharge line, wherein a first end of the culture medium discharge line is connected to a culture medium storage container, a second end of the culture medium discharge line communicates with the culture unit via a medium discharge port of the culture unit, and the culture medium can be circulated between the culture medium supply unit and the culture unit.

8. Production of exosome from cell

In the present invention, culturing a cell as above-mentioned allows the cell to produce an exosome. The exosome produced can be collected by a known method. The exosome is secreted from the cell, and thus, the substance can be collected from the cell culture medium.

In the method of the invention, the cell culture step and the exosome production step may be separate or otherwise. For example, a cell is already producing an exosome at the point of time when the cell is applied to a cell culture module to start cell culture. Accordingly, a production system designed to collect an exosome from a cell continuously from the start to termination of culture results in performing the cell culture step and the exosome production step simultaneously. Alternatively, in cases where the composition of the culture medium, the settings of the culture device, and/or the like is/are different between the cell culture and the exosome production, the termination of the cell culture step is followed by switching the culture conditions over to the conditions designed for the exosome production, and accordingly both of the steps are separate.

In the method of the invention, a culture cell can produce a uniform-quality exosome sustainably over a long period of time. In the method of the invention, the step of producing an exosome is preferably continued over 1 month, 2 months, 3 months, 6 months, or a longer period of time. In the method of the invention, it is preferable that the molecular profile and bioactivity of an exosome produced by a cell are evaluated during the exosome production period.

II. Exosome Production Device

The present invention also relates to a device for producing an exosome by cell culture, wherein the device is for use in the method of the invention, and comprises a cell culture module. In the exosome production device of the invention, the cell culture module may be used in a fixed manner, may be used as suspended in a cell culture medium, may be placed in the culture medium, or may be exposed out of the culture medium.

The exosome production device for cell culture in the invention may take any form so long as it comprises a cell culture module, and it is possible to use a known cell culture device. The culture device is not limited to any particular shape or scale, and any of a dish, a test tube, and a large tank may be utilized, as appropriate. These include, for example, Cell Culture Dish manufactured by BD Falcon, and Nunc Cell Factory manufactured by Thermo Scientific. In this regard, in the present invention, by using a porous polymer membrane, it has become possible to carry out culturing even of cells that have not been capable of natural suspension culture, using a device intended for suspension culture, in a state similar to suspension culturing. The device for suspension culture that is used may be, for example, a spinner flask or rotating culturing flask manufactured by Corning, Inc.

The exosome production device based on a cultured cell according to the invention may also be operated using a continuously circulating type or open type device in which a medium is continuously added to and collected from a cell culture module and in which a porous polymer membrane sheet is exposed to the air.

The method of the invention may further include a means for collecting a higher concentration of exosomes produced by cells. For example, connecting a semipermeable membrane and the like directly to a circulating culture medium makes it possible to remove an undesired substance such as lactic acid, and at the same time, add carbohydrate or an amino acid, and thus, makes it possible to create a combination of an efficient and long-time culture method and an undesired substance removal method.

III. Kit

The present invention further relates to a kit for use in the method of the invention, wherein the kit comprises a cell culture module. The kit of the invention can appropriately comprise a constituent to be used for cell culture, exosome production, and exosome collection, besides the cell culture module. Examples of such constituents include: a cell to be applied to a cell culture module; a cell culture medium; a continuous culture medium supply device; a continuous culture medium circulating device; a cell culture device; an evaluation means for verifying exosome production; an exosome collection means (for example, ultracentrifugation, ultrafiltration, immunoprecipitation, chromatography, and the like), an instruction manual for the kit, and the like.

IV. Use

The present invention further comprises use of a cell culture module in the method of the present invention.

V. Exosome

The present invention further comprises an exosome obtained by the method of the invention.
Below, the present invention will be described in detail with reference to Examples, and the invention is not limited to these Examples. A person skilled in the art may easily implement modifications and changes to the invention based on the description in the present specification, and these are also encompassed within the technical scope of the invention.

EXAMPLES

Porous polymer membranes used in the following Examples were porous polyimide membranes, and prepared by forming a polyamic acid solution composition including a polyamic acid solution obtained from 3,3',4,4'-biphenyltetracarboxylic dianhydride (s-BPDA) as a tetracarboxylic acid component and 4,4'-diaminodiphenyl ether (ODA) as a diamine component, and polyacrylamide as a coloring precursor, and performing heat treatment at 250°C or higher. The resulting porous polyimide membrane was a three-layer structure porous polyimide membrane having a surface layer A and a surface layer B, the surface layers having a plurality of pores, and a macrovoid layer sandwiched between the surface layers A and B; wherein the average pore diameter of the pore present on the surface layer A was 19 $\mu$m, the average pore diameter of the pore present on the surface layer B was 42 $\mu$m, and the membrane thickness was 25 $\mu$m, and the porosity was 74%.
Additionally, in the below-mentioned Examples, a modularized porous polymer membrane (referred to as a "module" in the below-mentioned Examples) was used, wherein the module included a polyethylene-made casing having sixteen 2 mm $\times$ 2 mm medium flow inlets/outlets on one surface thereof, and wherein the casing contained the following: six 1.0 $\times$ 1.0 cm square porous polyimide membranes, a 1.0 $\times$ 1.0 cm liner (polypropylene/polyethylene, product number: ESP10TC, manufactured by NBC Meshtec Inc.), six 1.0 $\times$ 1.0 cm square porous polyimide membranes, a 1.0 $\times$ 1.0 cm liner (polypropylene/polyethylene, product number: ESP10TC, manufactured by NBC Meshtec Inc.), and six 1.0 $\times$ 1.0 cm square porous polyimide membranes, which are stacked in this order. The module means this structure unless otherwise specified.
Additionally, in Examples in which a "type 2 module" is specified, a polyethylene-made casing having sixteen 2 mm $\times$ 2 mm medium flow inlets/outlets on one surface thereof was used, wherein the casing contained the following: four 1.0 $\times$ 1.0 cm square porous polyimide membranes, a 1.0 $\times$ 1.0 cm liner (polypropylene/polyethylene, product number: ESP10TC, manufactured by NBC Meshtec Inc.), four 1.0 $\times$ 1.0 cm square porous polyimide membranes, a 1.0 $\times$ 1.0 cm liner (polypropylene/polyethylene, product number: ESP10TC, manufactured by NBC Meshtec Inc.), four 1.0 $\times$ 1.0 cm square porous polyimide membranes, a 1.0 $\times$ 1.0 cm liner (polypropylene/polyethylene, product number: ESP10TC, manufactured by NBC Meshtec Inc.), four 1.0 $\times$ 1.0 cm square porous polyimide membranes, a 1.0 $\times$ 1.0 cm liner (polypropylene/ polyethylene, product number: ESP10TC, manufactured by NBC Meshtec Inc.), and four 1.0 $\times$ 1.0 cm square porous polyimide membranes, which are stacked in this order.

Comparative Example 1: Cell culture method using dish

Passage 2 mesenchymal stem cells (Y25 male _451491) cultured in an IWAKI Collagen I Coat dish were peeled off using TrypLE (trademark) Select manufactured by Thermo Fisher Scientific Inc., and suspended in a culture medium (Xeno-free culture medium, KBM ADSC-4, manufactured by Kohjin Bio Co., Ltd.). A cell suspension (3.0 $\times$ 10$^5$ cells in total) was disseminated in an IWAKI Collagen I Coat dish, 60 cm$^2$, and left to stand in a CO$_2$ incubator set to 5% CO$_2$ and 37°C. The cells were left adhered for approximately 16 hours, and then, the culture medium was exchanged for 12 mL of culture medium (KBM ADSC-4). Then, the culture medium was exchanged on the 6th day after the start of the culture, and the culture was continued for 13 days.

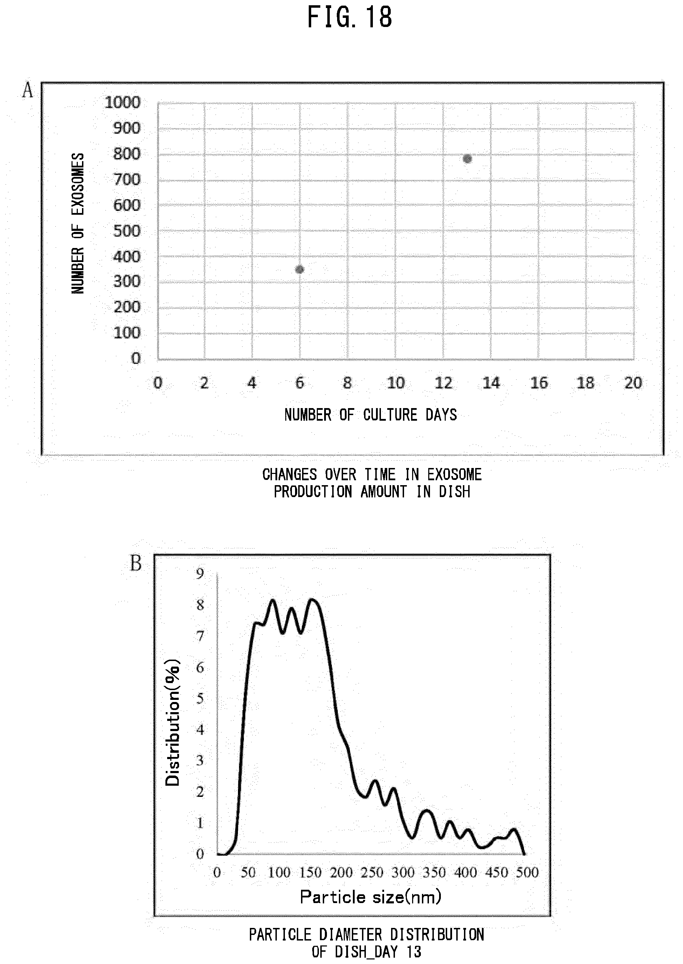

The number of exosomes produced per day and contained in the cell culture solution collected on culture Day 6 and Day 13 was measured using the procedures in the below-mentioned "Exosome Amount Measurement Using Zeta-potential/Particle Diameter Distribution Measurement Device", and the measurement results are illustrated in FIG. 18A. The particle diameter distribution measurement results on culture Day 13 are illustrated in FIG. 18B.

Example 1: Cell culture method using modules in 150-mL round storage bottle manufactured by Corning, Inc. (Bottle (5% Oxygen))

Into a 150-mL round storage bottle manufactured by Corning, Inc. and containing 5 modules, 50 mL of culture medium (Xeno-free culture medium, KBM ADSC-4, manufactured by Kohjin Bio Co., Ltd.) was poured, and then the bottle was placed in an incubator shaker (Lab-Therm LT-XC) manufactured by Kuhner AG and set to 5% $CO_2$, 5% $O_2$, and 37°C. Then, the resulting mixture was stirred at a rotation speed of 100 rpm for approximately 1 hour to wet the modules.

Passage 2 mesenchymal stem cells (Y25 male _451491) cultured in an IWAKI Collagen I Coat dish were peeled off using TrypLE (trademark) Select manufactured by Thermo Fisher Scientific Inc., and suspended in a culture medium (KBM ADSC-4). The cell suspension containing $2.0 \times 10^4$ cells ($1.80 \times 10^6$ cells in total) per $cm^2$ of the area of the porous polyimide membrane as a culture substrate in the module was poured into a storage bottle. The cells were allowed to be adsorbed onto the modules for approximately 16 hours through stirring at a rotation speed of 100 rpm, and then, the culture medium was exchanged for 50 mL of culture medium (KBM ADSC-4). The culture medium was exchanged 6 days after the start of the culture, and then, the culture medium was exchanged once every 3 days or 4 days.

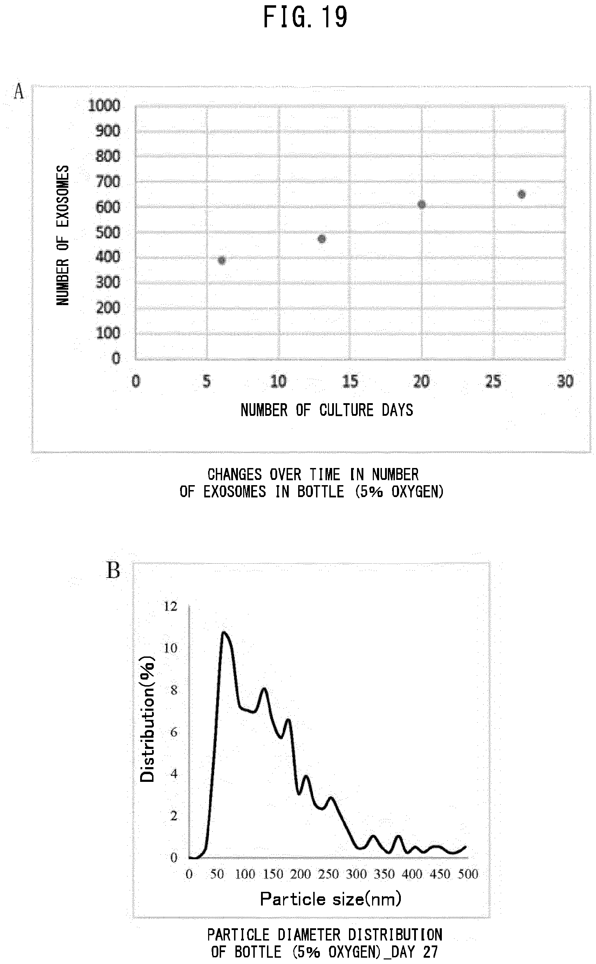

The number of exosomes produced per day and contained in the cell culture solution collected up to culture Day 27 after the start of the culture was measured using the procedures in the below-mentioned "Exosome Amount Measurement Using Zeta-potential/Particle Diameter Distribution Measurement Device", and the measurement results are illustrated in FIG. 19A. The particle diameter distribution measurement results on culture Day 27 are illustrated in FIG. 19B. Stable exosome production was verified even in a long-term continuous culture performed over approximately 1 month.

Example 2: Cell culture method using modules in 150-mL round storage bottle manufactured by Corning, Inc. (Bottle (Normal Oxygen))

Cell culture was performed by the same operation as in Example 1 (Bottle (5% oxygen)) except that a 150-mL round storage bottle manufactured by Corning, Inc. was placed in a $CO_2$ incubator (aniCell) manufactured by N-BIOTEK, having a built-in humidification-handling shaker, and set to 5% $CO_2$ and 37°C.

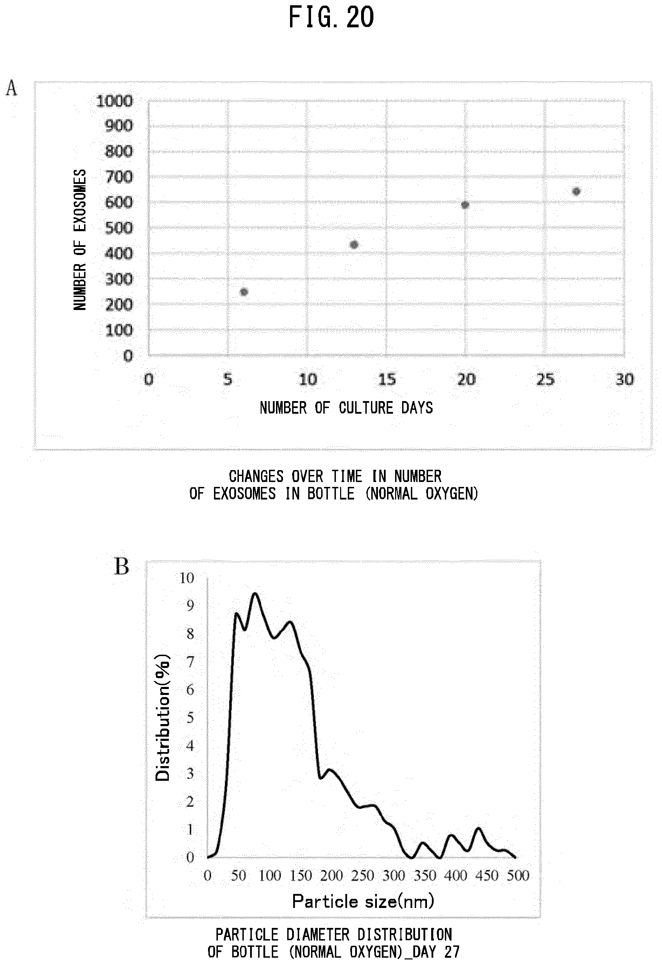

The number of exosomes produced per day and contained in the cell culture solution collected up to culture Day 27 after the start of the culture was measured using the procedures in the below-mentioned "Exosome Amount Measurement Using Zeta-potential/Particle Diameter Distribution Measurement Device", and the measurement results are illustrated in FIG. 20A. The particle diameter distribution measurement results on culture Day 27 are illustrated in FIG. 20B. Stable exosome production was verified even in a long-term continuous culture performed over approximately 1 month.

Example 3: Cell culture method using modules in overflow reactor (Reactor (5% Oxygen))

Into an overflow reactor culture vessel containing 40 modules, 40 mL of culture medium (Xeno-free culture medium, KBM ADSC-4, manufactured by Kohjin Bio Co., Ltd.) was poured, and then the vessel was placed in an incubator shaker (Lab-Therm LT-XC) manufactured by Kuhner AG and set to 5% $CO_2$, 5% $O_2$, and 37°C. Then, the resulting mixture was stirred at a rotation speed of 80 rpm for approximately 1 hour to wet the modules.

Passage 2 mesenchymal stem cells (Y25 male _451491) cultured in an IWAKI Collagen I Coat dish were peeled off using TrypL (trademark) Select manufactured by Thermo Fisher Scientific Inc., and suspended in a culture medium (KBM ADSC-4). The cell suspension containing $2.0 \times 10^4$ cells ($1.44 \times 10^7$ cells in total) per $cm^2$ of the area of the porous polyimide membrane as a culture substrate in the module was poured into an overflow reactor culture vessel. A culture medium was added in such a manner that the amount of the solution in the overflow reactor culture vessel became 80 mL. Then, the cells were allowed to be adsorbed for approximately 26 hours under stirring conditions at a rotation speed of 80 rpm.

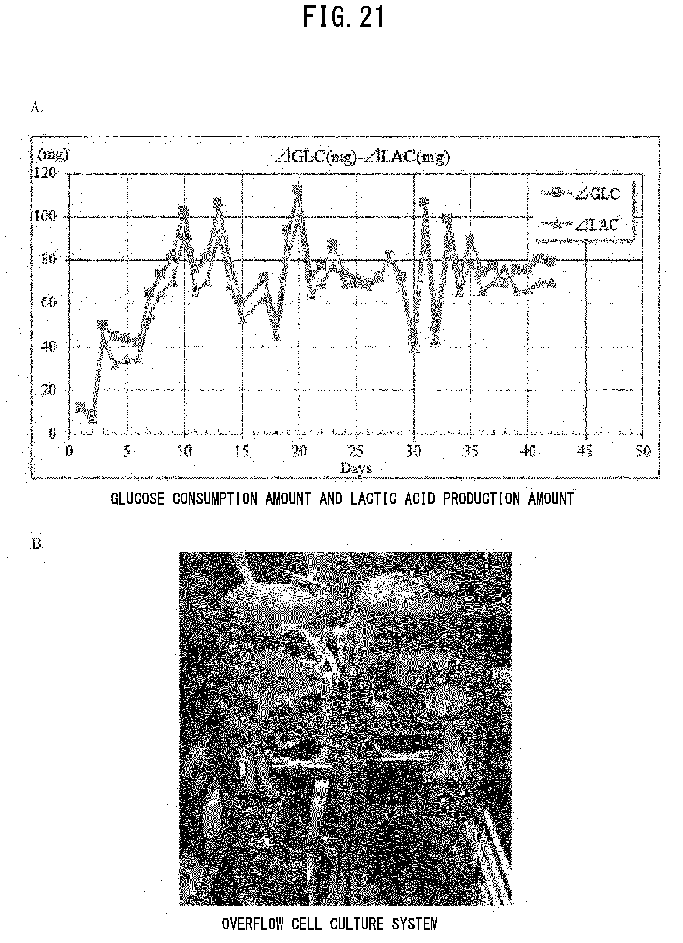

After the adsorption of the cells, a glucose solution (45 w/v% D(+)-glucose solution manufactured by Fujifilm Wako Pure Chemical Corporation) was poured into the culture medium (KBM ADSC-4). The solution adjusted to have a total glucose concentration of 2000 mg/L (glucose-adjusted KBM ADSC-4) started to be transferred into an overflow reactor culture vessel using a tube pump at a speed of 40 mL/day. The culture medium overflown through the culture medium collection outlet disposed in the wall face of the culture vessel was collected into a collection liquid pool bottle. A long-term cell culture was performed as follows: using CedexBio manufactured by F. Hoffmann-La Roche, Ltd., the cell growth behavior of the culture medium collected was observed with reference to changes in the metabolism parameters of the medium collected; and the medium-feeding speed and the rotation speed were changed, as appropriate, in accordance with the consumption of glucose and the like of the cell. Changes over time in the glucose consumption amount and the lactic acid production amount are illustrated in FIG. 21A, and how the cell culture using an overflow reactor was is illustrated in FIG. 21B. Stable cell proliferation and growth over a long period of time was observed.

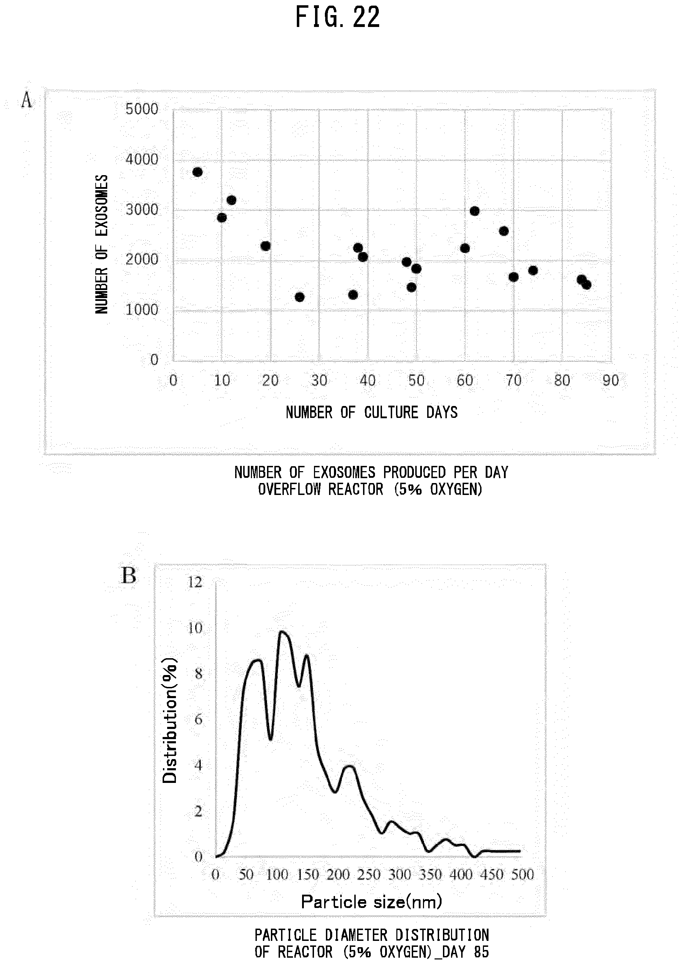

The number of exosomes produced per day and contained in the cell culture solution collected was measured using the procedures in the below-mentioned "Exosome Amount Measurement Using Zeta-potential/Particle Diameter Distribution Measurement Device", and the measurement results are illustrated in FIG. 22A. The particle diameter distribution measurement results on culture Day 85 are illustrated in FIG. 22B. Stable exosome production was verified even in a long-term continuous culture carried out over approximately 3 months.

Example 4: Cell culture method using modules in overflow reactor (Reactor (Normal Oxygen))

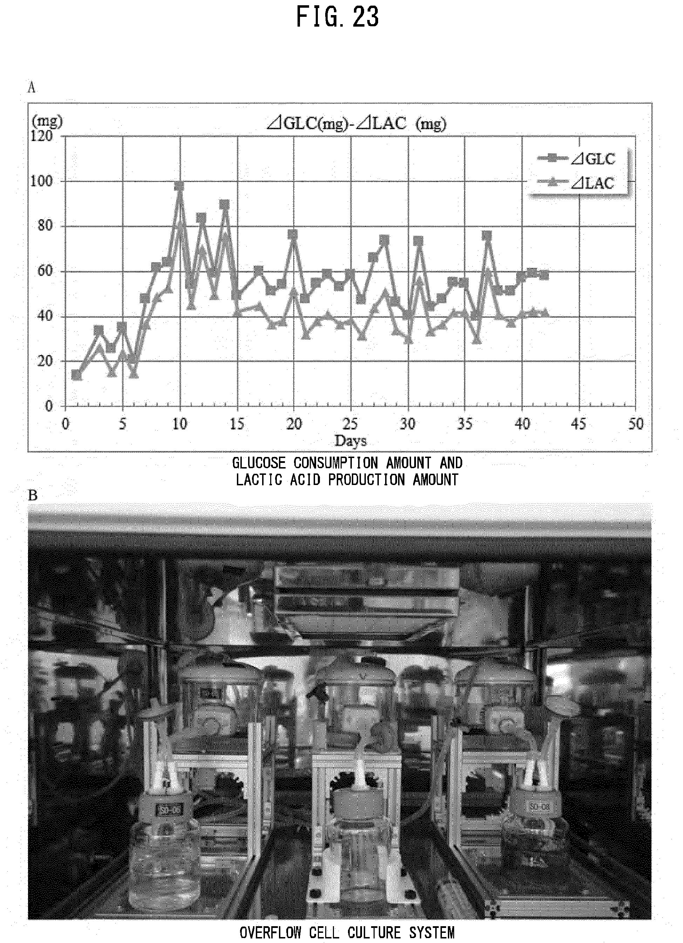

Cell culture was performed by the same operation as in Example 3 (Reactor (5% oxygen)) except that an overflow reactor was placed in a $CO_2$ incubator (aniCell) manufactured by N-BIOTEK, having a built-in humidification-resistant shaker, and set to 5% $CO_2$ and 37°C. Changes over time in the glucose consumption amount and the lactic acid production amount are illustrated in FIG. 23A, and how the cell culture using an overflow reactor was is illustrated in FIG. 23B. Stable cell proliferation and growth over a long period of time was observed.

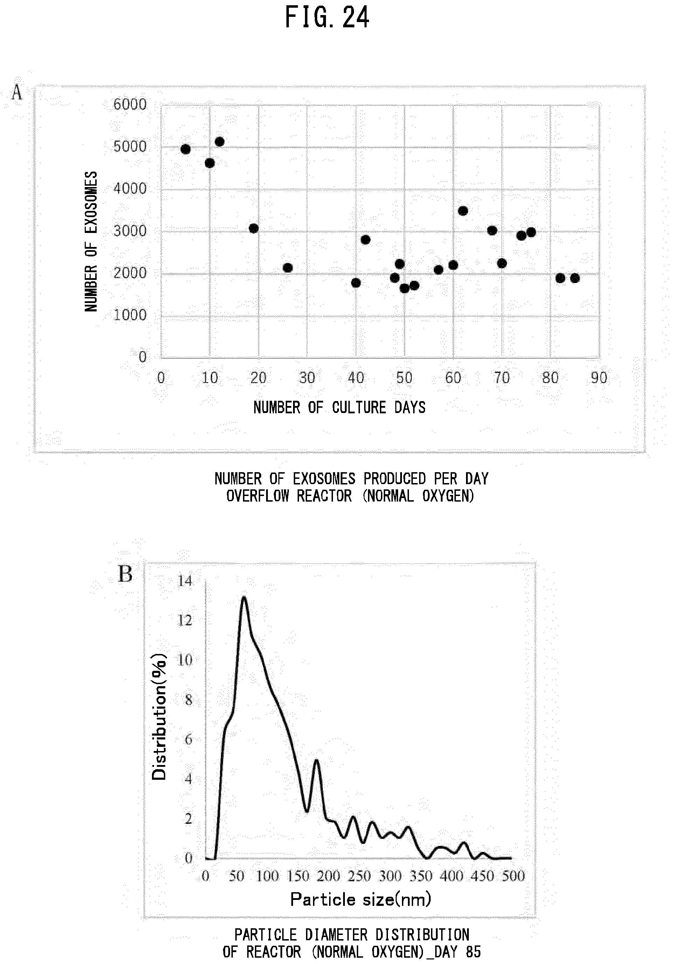

The number of exosomes produced per day and contained in the cell culture solution collected was measured using the procedures in the below-mentioned "Exosome Amount Measurement Using Zeta-potential/Particle Diameter Distribution Measurement Device", and the measurement results are illustrated in FIG. 24A. The particle diameter distribution measurement results on culture Day 85 are illustrated in FIG. 24B. Stable exosome production was verified even in a long-term continuous culture carried out over approximately 3 months.

Example 5: Cell culture method using modules in WAVE reactor

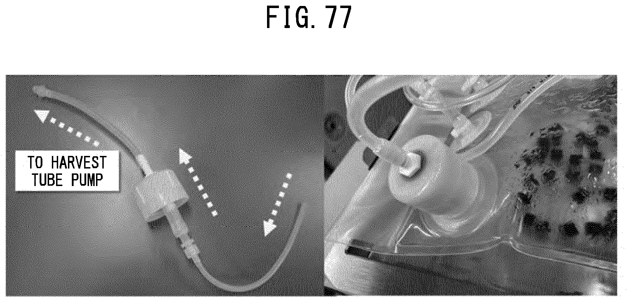

To perform culture medium withdrawal continuously, a liquid withdrawal tube was provided on a cap portion of a WAVE culture bag (CELLBAG DISPOSABLE BIOREACTOR Part # CB0002L11-33) manufactured by GE. With a γ-beam-sterilized Nalgene HDPE cap (product number: 342151-0384) mounted in exchange in a sterilizing manner, the tube was connected to a Harvest tube pump (FIG. 77). In this regard, in the below-mentioned Examples, culture was performed with the same withdrawal line arranged, in cases where continuous culture was performed using a WAVE reactor.

Preliminarily, 200 modules were wetted for 24 hours or more with a Ham's F-12 culture medium manufactured by Fujifilm Wako Pure Chemical Corporation and containing glutamine and phenol red, and the modules were transferred into the above-mentioned bag in a sterilizing manner. Into the bag, 200 mL of culture medium (Xeno-free culture medium, KBM ADSC-4, manufactured by Kohjin Bio Co., Ltd.) was poured. The bag was placed in a WAVE reactor (ReadyTo-Process WAVE 25) manufactured by GE, and then the modules were wetted through stirring for approximately 1 hour under conditions: 5% $CO_2$, a temperature of 37°C, a vibration speed of 15 rpm, and a vibration angle of 9°.

Passage 3 mesenchymal stem cells (Y25 male_18TL262066) cultured in a dish were peeled off using "Trypsin-EDTA (0.05%), phenol red" manufactured by Gibco, and suspended with a culture medium (DMEM manufactured by Fujifilm Wako Pure Chemical Corporation). The cell suspension containing $2.0 \times 10^4$ cells ($7.20 \times 10^7$ cells in total) per $cm^2$ of the area of the porous polyimide membrane as a culture substrate in the module was disseminated, and then, a culture medium (Xeno-free culture medium, KBM ADSC-4, manufactured by Kohjin Bio Co., Ltd.) was immediately added in such a manner that the amount of the solution in the WAVE culture bag manufactured by GE became 300 mL. Then, the cells were allowed to be adsorbed for approximately 27 hours under the same vibration conditions as during the module wetting.

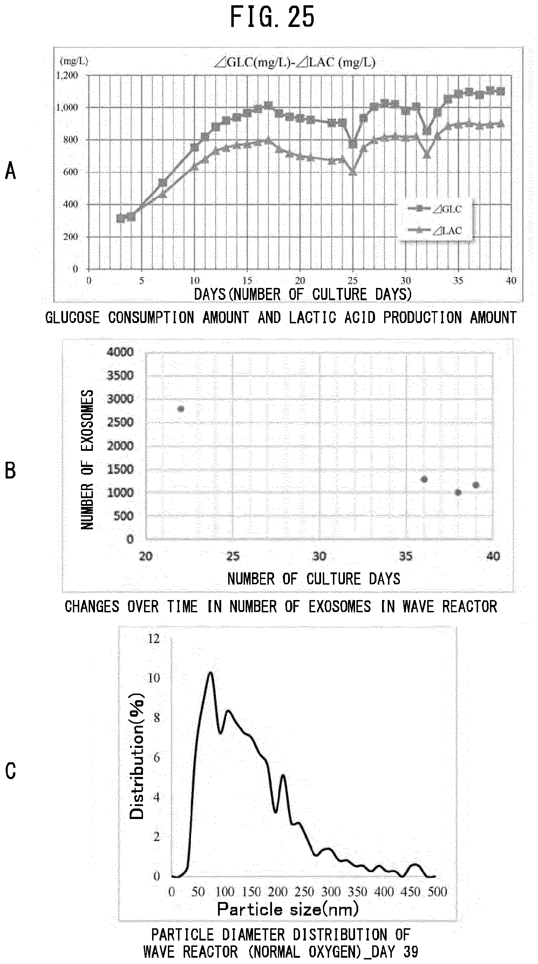

Then, the perfusion function (bag weight control function) of the reactor was utilized to withdraw the cell culture solution from the WAVE culture bag manufactured by GE, via the Harvest tube pump at a speed of 350 mL/day, and the culture medium (KBM ADSC-4) having the same volume as the above-mentioned culture medium withdrawn was liquid-transferred into the WAVE culture bag manufactured by GE, via the Feed tube pump. Using CedexBio manufactured by F. Hoffmann-La Roche, Ltd., the cell growth behavior of the culture medium collected was observed with reference to changes in the metabolism parameters, with the result that stable cell proliferation and growth over a long period of time was observed. Changes over time in the glucose consumption amount and the lactic acid production amount are illustrated in FIG. 25A.

The number of exosomes produced per day and contained in the cell culture solution collected was measured using the procedures in the below-mentioned "Exosome Amount Measurement Using Zeta-potential/Particle Diameter Distribution Measurement Device", and the measurement results are illustrated in FIG. 25B. The particle diameter distribution measurement results on culture Day 39 are illustrated in FIG. 25C. Stable exosome production was verified even in a long-term continuous culture performed over approximately 1 month.

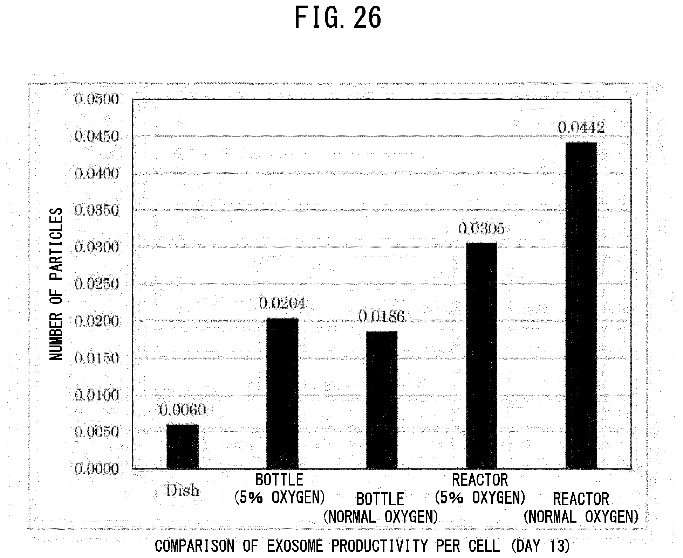

FIG. 26 illustrates a comparison of the number of exosomes produced per day by 1 cell on cell culture Day 13 among the various culture methods, as demonstrated in Comparative Example and Examples. The module culture, compared with the Dish culture, verified a very high exosome productivity per cell.

Exosome amount measurement using zeta-potential/particle diameter distribution measurement device

The cell culture solution collected under the culture conditions of Comparative Example 1 and Examples 1 to 5 was centrifuged using a high-speed cooling centrifuge (Model 6000, manufactured by Kubota Corporation) at 10,000 g at 4°C for 30 minutes to remove debris (prepared solution 1). The "prepared solution 1" was diluted to a predetermined concentration using PBS(-) (distributor's code: 166-23555, manufactured by Fujifilm Wako Pure Chemical Corporation) from which fine particles had been removed by suction filtration using a 0.025 $\mu$m filter (product name: MF-Millipore membrane filter, model number: VSWP04700, manufactured by Merck & Co., Inc.) (prepared solution 2). Then, 200 nm or larger particles were removed from the "prepared solution 2" using a 0.2 $\mu$m syringe filter (product name: MINISART, model number: 16534K, manufactured by Sartorius AG) (prepared solution 3). A zeta-potential/particle diameter distribution measurement device (ZEECOM ZC-3000, manufactured by Microtec Co., Ltd.) was used to measure, by Brownian motion track analysis, the particle diameter distribution of the particles contained in the "prepared solution 3" and the number of the particles in the exosome fraction.

At the time when the culture medium for measurement of an exosome was collected, Cell Counting Kit-8 manufactured by Dojindo Laboratories was used to measure the number of cells. The amount of exosomes produced per day by one cell was calculated according to the following "Equation 1". The exosome production performance of the cell was compared among the different cell culture conditions.

Equation 1:

$$\text{Number of particles} \times \text{Dilution magnification ratio of sample} \times \text{Ratio of 150 nm or smaller particles} \times \text{Amount of culture medium collected} / \text{total number of cells} / \text{Number of days of culture medium pooling}$$

The changes over time in the exosome produce amount per day under the different cell culture conditions were measured according to the following "Equation 2".

Equation 2:

$$\text{Number of particles} \times \text{Dilution ratio of sample} \times \text{Ratio of 150 nm or smaller particles} / \text{Number of days of culture medium pooling}$$

miRNA Array Analysis Using GeneChip (Trademark) miRNA 4.0 Array

The cell culture solution collected under the culture conditions of Comparative Example 1 and Examples 1 to 5 was centrifuged using a high-speed cooling centrifuge (Model 6000, manufactured by Kubota Corporation) at 10,000 g at 4°C for 30 minutes to remove debris. From 8 mL of the supernatant obtained, exosomes were extracted by a polymer precipitation method using Total Exosome Isolation Regent (from cell culture media) (Cat. # 4478359, manufactured by Thermo Fisher Scientific Inc.). Then, using Total Exosome RNA and Protein Isolation Kit (Cat. # 4478545, manufactured by Thermo Fisher Scientific Inc.), Total RNA was extracted from the exosome-extracted solution and purified, and then, the concentration of the Total RNA solution was measured using Nano Drop 2000 (manufactured by Thermo Fisher Scientific Inc.). Using 390 ng of the Total RNA obtained, a human-type miRNA array analysis was performed with GeneChip (trademark) miRNA 4.0 Array (Cat. # 902445, manufactured by Thermo Fisher Scientific Inc.).

Comparison of exosome production amount between Comparative Example 1 (Dish_Day 6) and Example 1 (Bottle (5% Oxygen)_Day 27), and miRNA array analysis

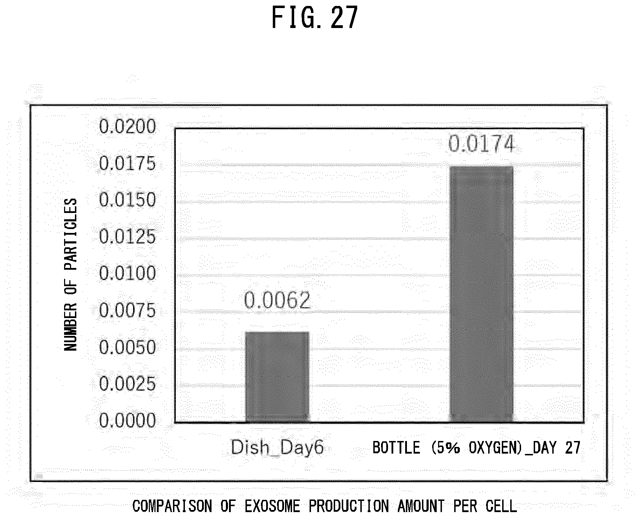

FIG. 27 represents the comparison of the exosome production amount per cell between Comparative Example 1 (Dish Day 6) and Example 1 (Bottle (5% Oxygen)_Day 27). With the Bottle (5% oxygen) culture in which the porous polyimide membrane modules were used, the exosome production performance was found to be 2.8 times higher than with Dish_Day 6 even in a long-term continuous stable culture state.

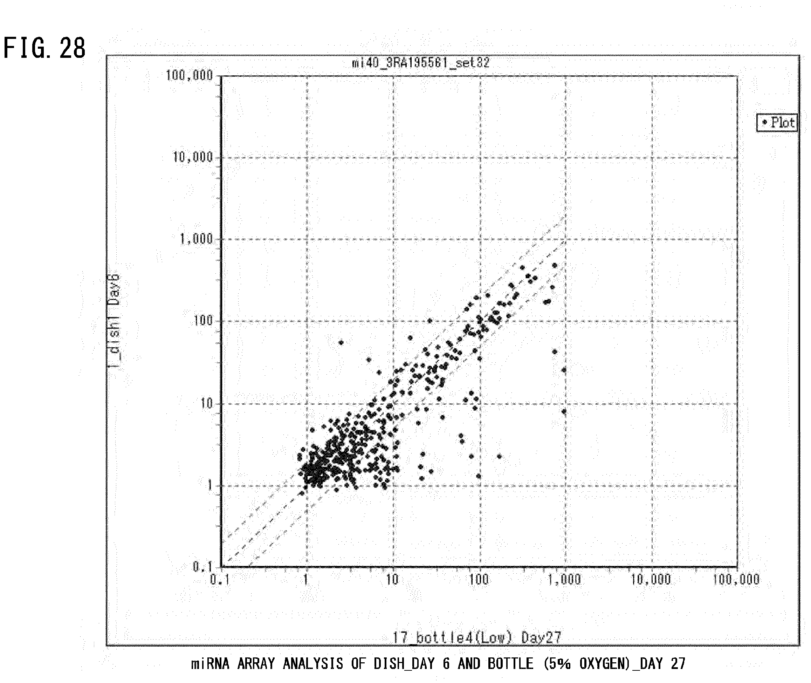

FIG. 28 represents a Scatterplot of the miRNA array analyses in Comparative Example 1 (Dish_Day 6) and Example 1 (Bottle (5% oxygen)_Day 27), and Table 1 lists the miRNAs the expression ratio of which exhibited a tenfold or more difference.

[Table 1]

Table 1. miRNAs the expression ratio of which exhibited a tenfold or more difference

| microRNA Types | Expression Amount in Bottle (5% Oxygen)_Day 27 Assuming that Expression Amount in Dish_Day 6 is 1 |
|---|---|
| hsa-miR-23a-3p | 38.85 |
| hsa-miR-24a-3p | 17.54 |
| hsa-miR-26a-5 | 36.57 |
| hsa-miR-31a-5p | 12.37 |
| hsa-miR-214-3p | 75.00 |
| hsa-miR-23b-3p | 15.36 |
| hsa-miR-320c | 10.19 |
| hsa-miR-1290 | 77.69 |
| hsa-miR-1246 | 121.72 |
| hsa-miR-320d | 18.89 |
| hsa-miR-3124-5p | 18.83 |
| hsa-miR-3128 | 17.72 |
| hsa-miR-7641 | 1/22.29 |

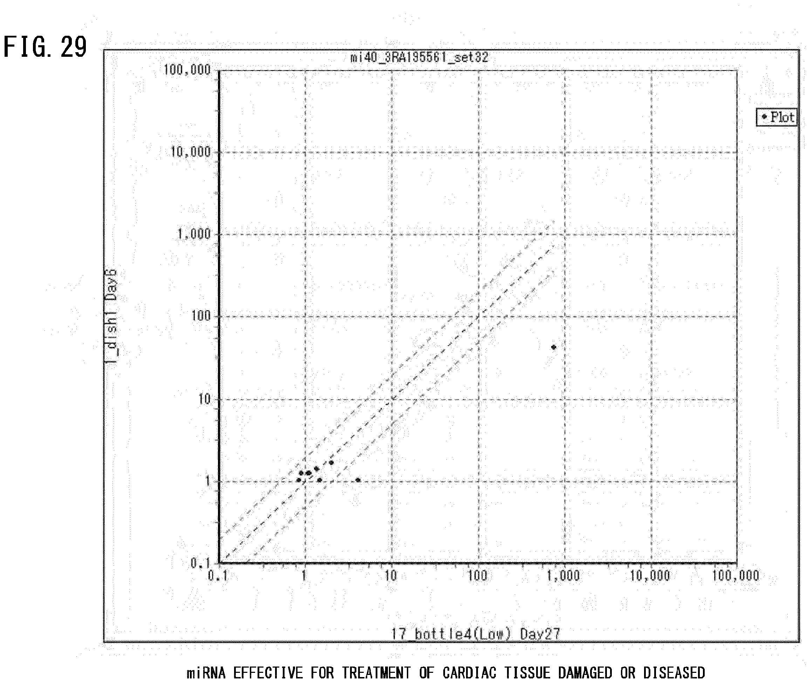

FIG. 29 represents a Scatterplot of the miRNA array analyses of hsa-miR-146a, hsa-miR-210, hsa-miR-22, and hsa-miR-24, which are miRNAs described in PTL 2 and effective for treatment of a cardiac tissue damaged or diseased. With the Bottle (5% oxygen) culture in which the porous polyimide membrane modules were used, the production amounts of hsa-miR-210-3p and hsa-miR-24a-3p were found to be 4.0 times and 17.5 times respectively higher than with Dish Day 6 even in a long-term continuous stable culture state, and the expression amount of each of the other miRNAs was approximately the same.

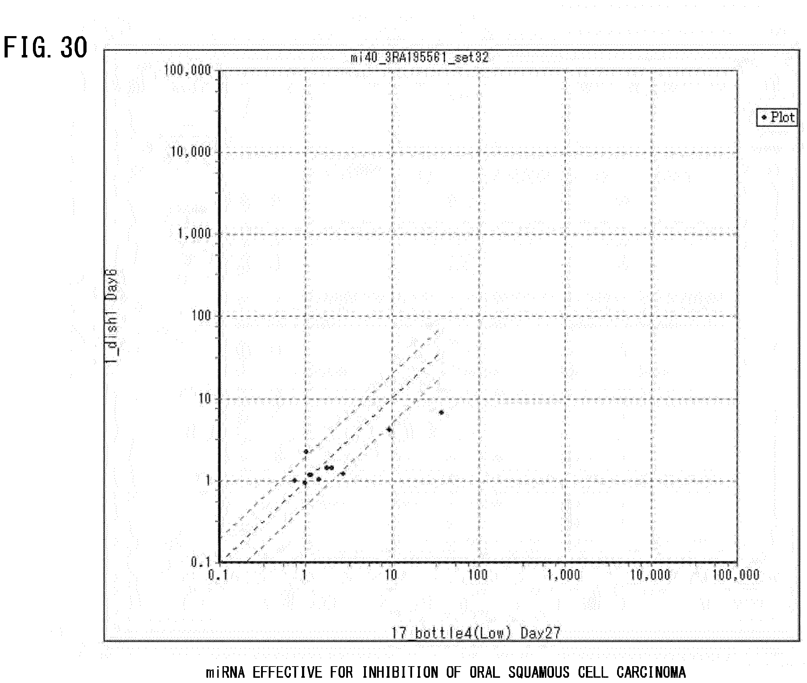

FIG. 30 represents the results of the miRNA array analyses of hsa-miR-34b, hsa-miR-132, hsa-miR-203, hsa-miR-137, and hsa-miR-193, which are miRNAs described in PTL 3 and effective for inhibition of oral squamous cell carcinoma. With the Bottle (5% oxygen) culture in which the porous polyimide membrane modules were used, the production amount of hsa-miR-193a-5p was found to be 5.8 times higher than with Dish_Day 6 even in a long-term continuous stable culture state, and the expression amount of each of the other miRNAs was approximately the same.

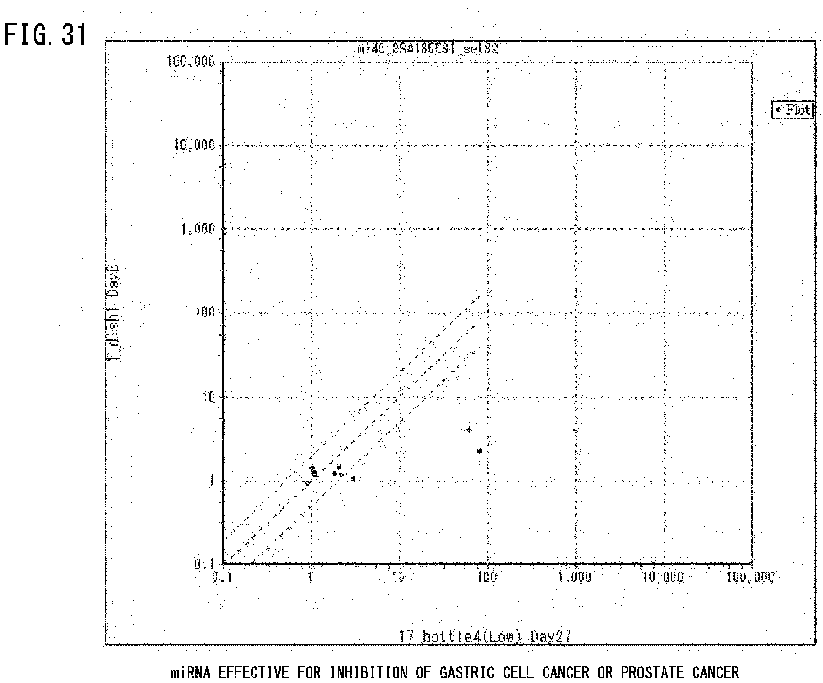

FIG. 31 represents the results of the miRNA array analyses of hsa-miR-26a/b, hsa-miR-23b, hsa-miR-27b, and hsa-miR-24-1, which are miRNAs described in NPL 6 and NPL 7 and effective for inhibition of gastric cell cancer or a prostate cancer cell. With the Bottle (5% oxygen) culture in which the porous polyimide membrane modules were used, the production amounts of hsa-miR-26a-5p and hsa-miR-23b-3p were found to be 36.6 times and 15.4 times respectively higher than with Dish_Day 6 even in a long-term continuous stable culture state, and the expression amount of each of the other miRNAs was approximately the same.

Comparison of exosome production amount between Comparative Example 1 (Dish Day 13) and Example 1 (Bottle (5% Oxygen)_Day 27), and miRNA array analysis

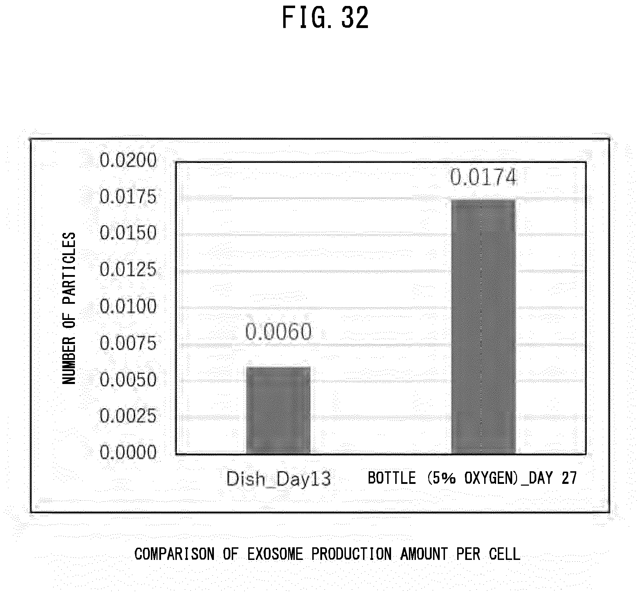

FIG. 32 represents a comparison of the exosome production amount per cell between Comparative Example 1 (Dish Day 13) and Example 1 (Bottle (5% oxygen)_Day 27). With the Bottle (5% oxygen) culture in which the porous polyimide membrane modules were used, the exosome production performance was found to be 2.9 times higher than with Dish_Day 13 even in a long-term continuous stable culture state.

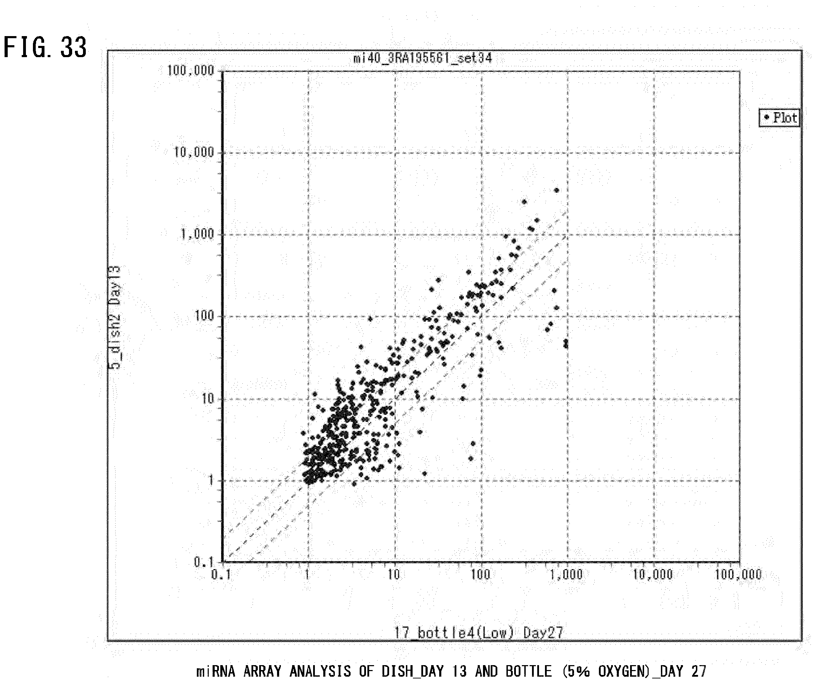

FIG. 33 represents a Scatterplot of the miRNA array analyses in Comparative Example 1 (Dish Day 13) and Example 1 (Bottle (5% oxygen)_Day 27), and Table 2 lists the miRNAs the expression ratio of which exhibited a tenfold or more difference.

[Table 2]

Table 2. miRNAs the expression ratio of which exhibited a tenfold or more difference

| microRNA Types | Expression Amount in Bottle (5% Oxygen)_Day 27 Assuming that Expression Amount in Dish_Day 13 is 1 |
| --- | --- |
| hsa-miR-23a-3p | 21.90 |
| hsa-miR-26a-5p | 29.14 |
| hsa-miR-199a-3p | 41.33 |
| hsa-miR-199b-3p | 41.33 |
| hsa-miR-1246 | 19.04 |
| hsa-miR-3201 | 18.11 |
| hsa-miR-1273g-3p | 1/17.75 |
| hsa-miR-6780b-5p | 1/10.17 |

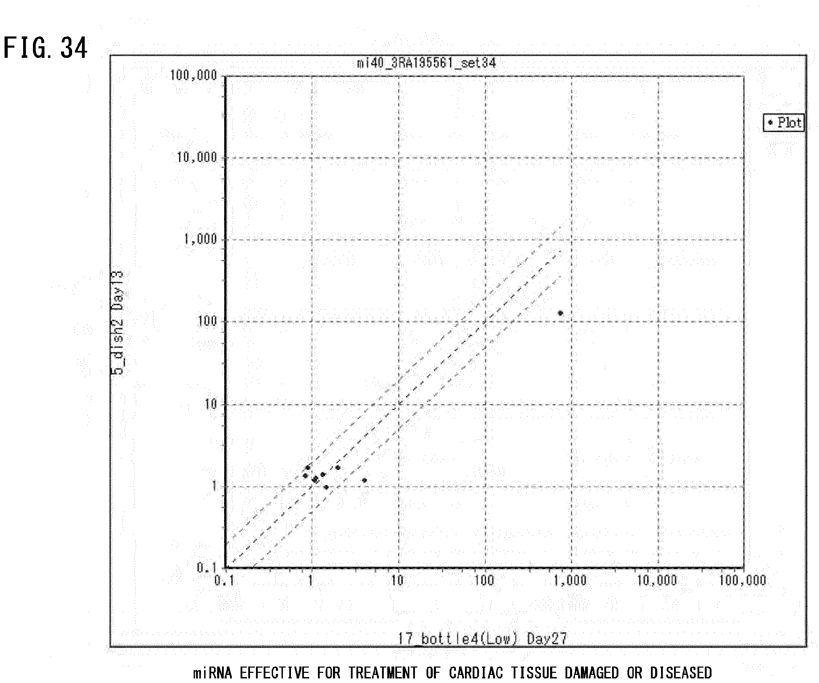

FIG. 34 represents the results of the miRNA array analyses of hsa-miR-146a, hsa-miR-210, hsa-miR-22, and hsa-miR-24, which are miRNAs described in PTL 2 and effective for treatment of a cardiac tissue damaged or diseased. With the Bottle (5% oxygen) culture in which the porous polyimide membrane modules were used and which was in a long-term continuous stable culture state, the production amount of hsa-miR-24-3p was found to be 5.3 times higher than with Dish_Day 13 that was just about to reach confluence, and the expression amount of each of the other miRNAs was approximately the same.

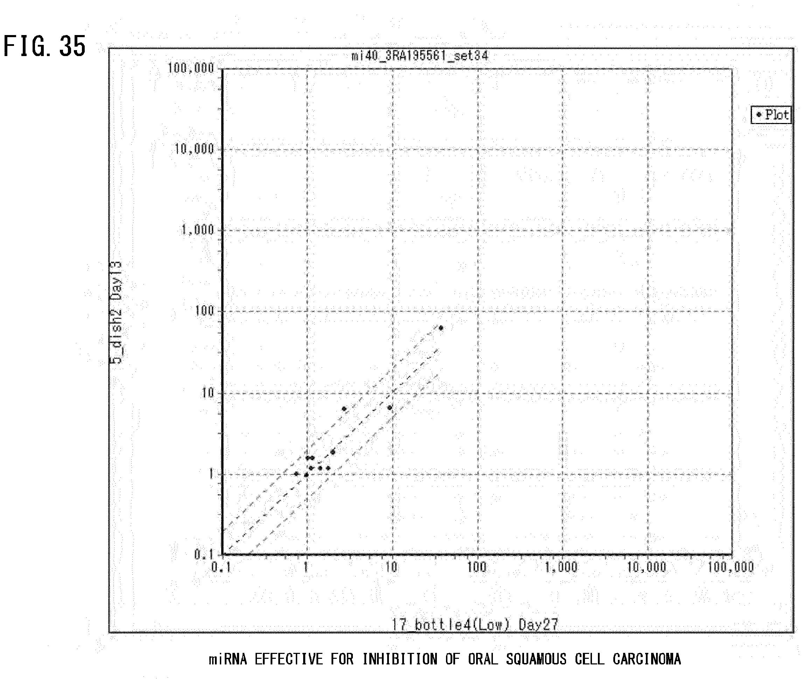

FIG. 35 represents the results of the miRNA array analyses of hsa-miR-34b, hsa-miR-132, hsa-miR-203, hsa-miR-137, and hsa-miR-193, which are miRNAs described in PTL 3 and effective for inhibition of oral squamous cell carcinoma. With the Bottle (5% oxygen) culture in which the porous polyimide membrane modules were used and which was in a long-term continuous stable culture state, the same results were demonstrated as with Dish_Day 13 that was just about to reach confluence.

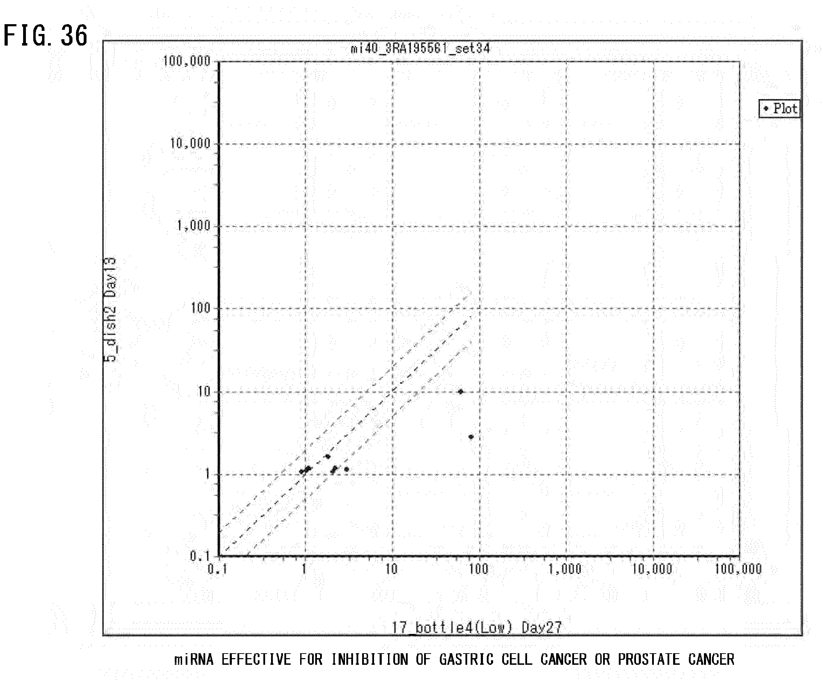

FIG. 36 represents the results of the miRNA array analyses of hsa-miR-26a/b, hsa-miR-23b, hsa-miR-27b, and hsa-miR-24-1, which are miRNAs described in NPL 6 and NPL 7 and effective for inhibition of gastric cell cancer or a prostate cancer cell. With the Bottle (5% oxygen) culture in which the porous polyimide membrane modules were used, the production amounts of hsa-miR-26a-5p and hsa-miR-23b-3p were found to be 29.1 times and 6.1 times respectively higher than with Dish_Day 13 that was just about to reach confluence, even in a long-term continuous stable culture state, and the expression amount of each of the other miRNAs was approximately the same.

Comparison of exosome production amount between Comparative Example 1 (Dish Day 6) and Example 2 (Bottle (Normal Oxygen)_Day 27), and miRNA analysis

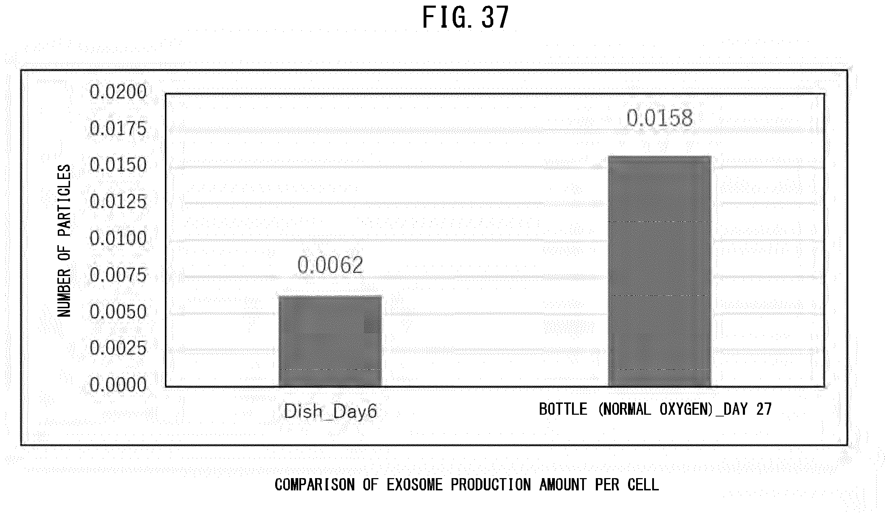

FIG. 37 represents a comparison of the exosome production amount per cell between Comparative Example 1 (Dish Day 6) and Example 2 (Bottle (Normal Oxygen)_Day 27). With the Bottle (Normal Oxygen) culture in which the porous polyimide membrane modules were used, the exosome production performance was found to be 2.5 times higher than with Dish_Day 6 even in a long-term continuous stable culture state.

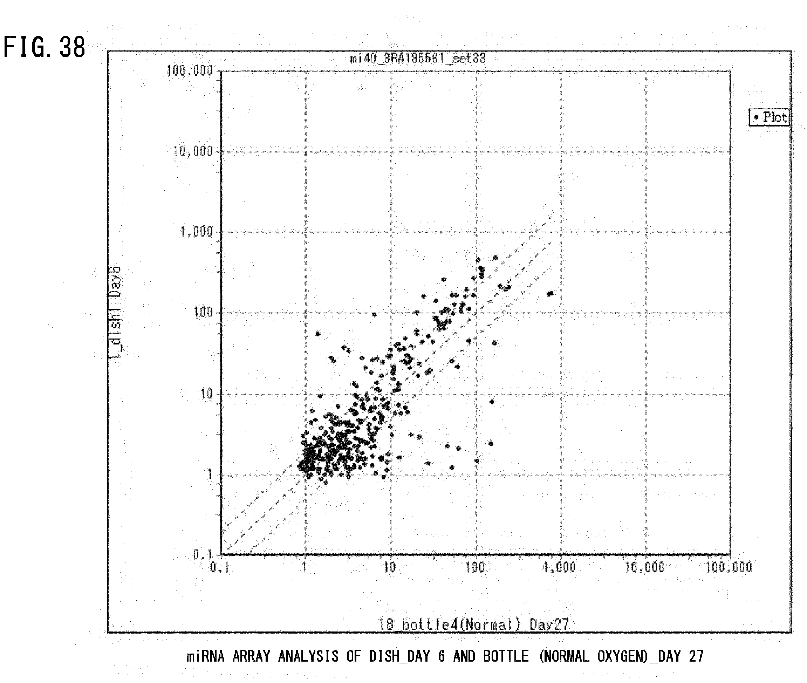

FIG. 38 represents a Scatterplot of the miRNA array analyses in Comparative Example 1 (Dish Day 6) and Example 2 (Bottle (Normal Oxygen)_Day 27), and Table 3 lists the miRNAs the expression ratio of which exhibited a tenfold or more difference.

[Table 3]

Table 3. miRNAs the expression ratio of which exhibited a tenfold or more difference

| microRNA Types | Expression Amount in Bottle (Normal Oxygen) _Day 27 Assuming that Expression Amount in Dish Day 6 is 1 |
|---|---|
| hsa-let-7a-5p | 1/13.61 |
| hsa-let-7c-5p | 1/148.62 |
| hsa-miR-214-3p | 21.03 |
| hsa-miR-1246 | 19.99 |
| hsa-miR-3124-5p | 70.38 |
| hsa-miR-3128 | 42.78 |
| has-miR-3201 | 64.28 |
| hsa-miR-4423-3p | 20.25 |
| hsa-miR-4484 | 1/12.12 |
| hsa-miR-1273g-3p | 1/10.52 |
| hsa-miR-6126 | 1/13.36 |
| hsa-miR-7641 | 1/40.37 |
| has-miR-8084 | 30.04 |

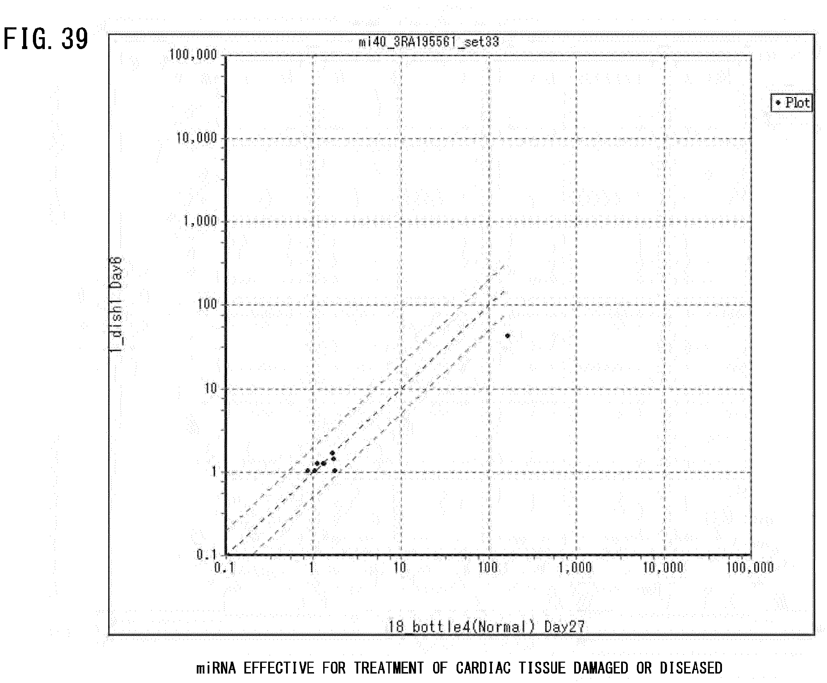

FIG. 39 represents the results of the miRNA array analyses of hsa-miR-146a, hsa-miR-210, hsa-miR-22, and hsa-miR-24, which are miRNAs described in PTL 2 and effective for treatment of a cardiac tissue damaged or diseased. With the Bottle (Normal Oxygen) culture in which the porous polyimide membrane modules were used, the production amount of hsa-miR-24-3p was found to be 3.9 times higher than with Dish_Day 6 even in a long-term continuous stable culture state, and the expression amount of each of the other miRNAs was approximately the same.

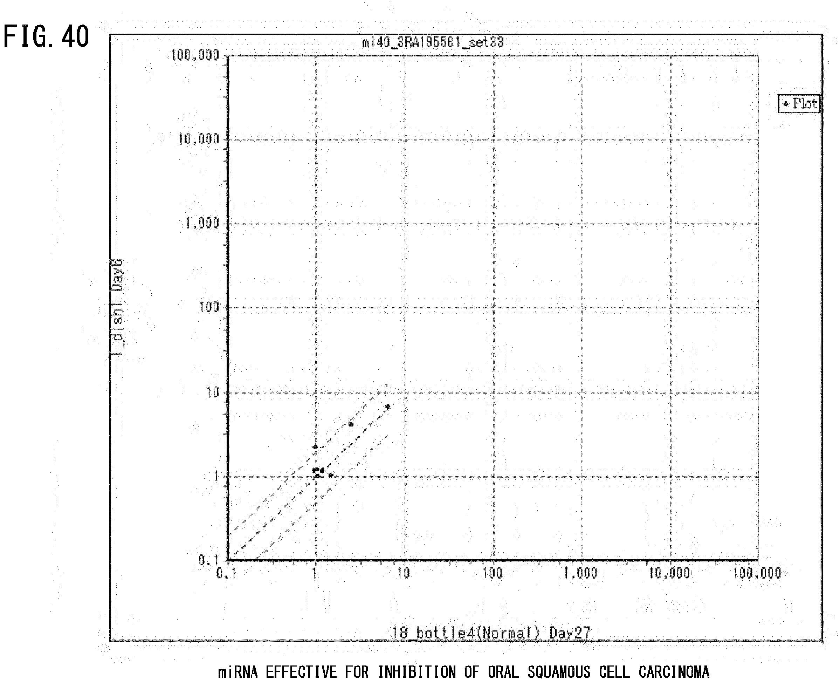

FIG. 40 represents the results of the miRNA array analyses of hsa-miR-34b, hsa-miR-132, hsa-miR-203, hsa-miR-137, and hsa-miR-193, which are miRNAs described in PTL 3 and effective for inhibition of oral squamous cell carcinoma. With the Bottle (Normal Oxygen) culture in which the porous polyimide membrane modules were used, the production amount was found to be the same as with Dish Day 6 even in a long-term continuous stable culture state.

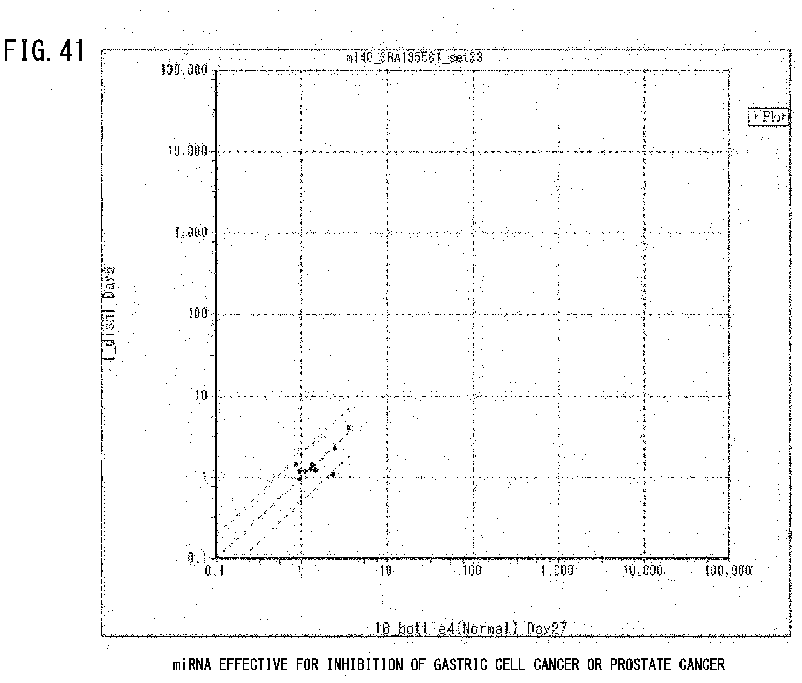

FIG. 41 represents the results of the miRNA array analyses of hsa-miR-26a/b, hsa-miR-23b, hsa-miR-27b, and hsa-miR-24-1, which are miRNAs described in NPL 6 and NPL 7 and effective for inhibition of gastric cell cancer or a prostate cancer cell. With the Bottle (Normal Oxygen) culture in which the porous polyimide membrane modules were used, the expression amount was found to be the same as with Dish_Day 6 even in a long-term continuous stable culture state.

Comparison of exosome production amount between Comparative Example 1 (Dish Day 13) and Example 2 (Bottle (Normal Oxygen)_Day 27), and miRNA array analysis

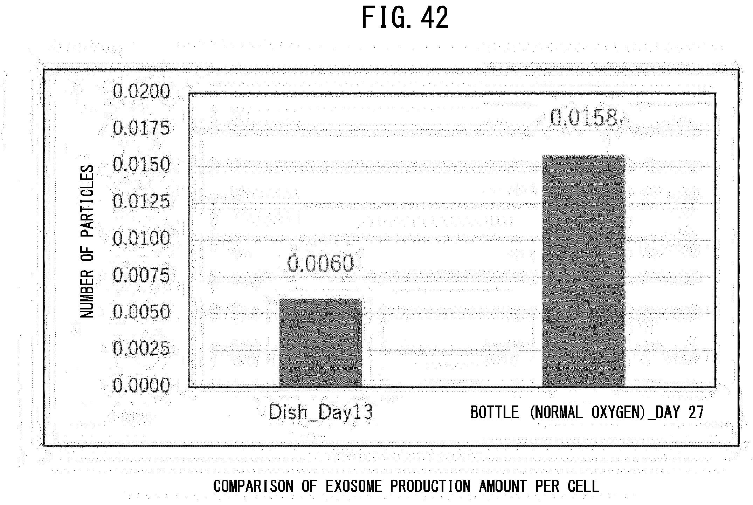

FIG. 42 represents a comparison of the exosome production amount per cell between Comparative Example 1 (Dish Day 13) and Example 2 (Bottle (Normal Oxygen)_Day 27). With the Bottle (Normal Oxygen) culture in which the porous polyimide membrane modules were used, the exosome production performance was found to be 2.6 times higher than with Dish_Day 13 even in a long-term continuous stable culture state.

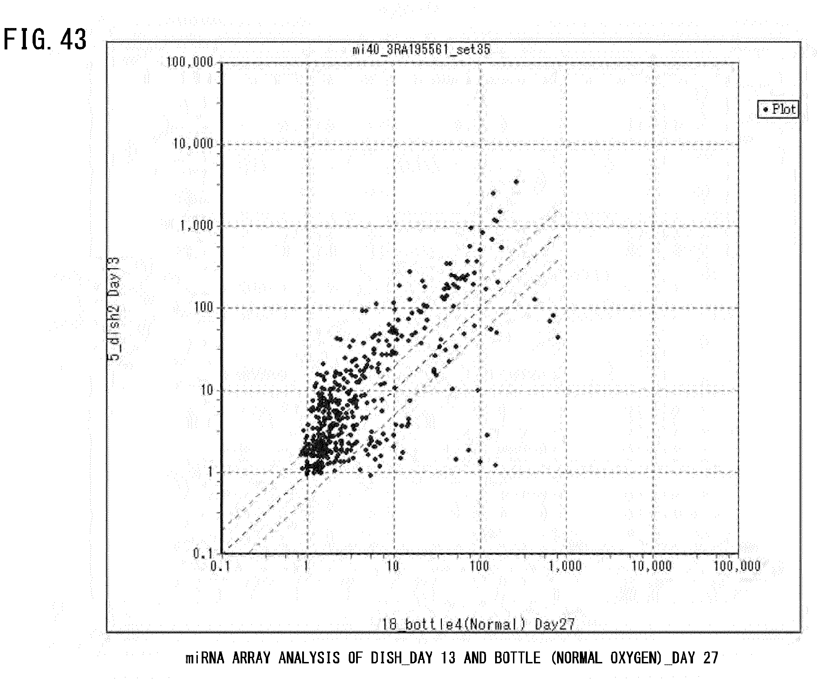

FIG. 43 represents a Scatterplot of the miRNA array analyses in Comparative Example 1 (Dish_Day 13) and Example 2 (Bottle (Normal Oxygen)_Day 27), and Table 4 lists the miRNAs the expression ratio of which exhibited a tenfold or more difference.

[Table 4]

Table 4. miRNAs the expression ratio of which exhibited a tenfold or more difference

| microRNA Types | Expression Amount in Bottle (Normal Oxygen) _Day 27 Assuming that Expression Amount in Dish Day 13 is 1 |
|---|---|
| hsa-miR-21-5p | 37.23 |
| hsa-miR-23a-3p | 18.07 |
| hsa-miR-26a-5p | 43.80 |
| hsa-miR-199a-3p | 39.87 |
| hsa-miR-199b-3p | 39.87 |
| has-miR-320a | 1/16.21 |
| hsa-miR-423-5p | 1/10.48 |
| hsa-miR-92b-5p | 1/16.97 |
| hsa-miR-320c | 1/11.52 |
| hsa-miR-1207-5p | 1/10.73 |
| hsa-miR-3201 | 125.77 |
| hsa-miR-4270 | 1/17.88 |
| hsa-miR-4532 | 1/13.67 |
| hsa-miR-3960 | 1/13.24 |
| hsa-miR-4739 | 1/21.41 |
| hsa-miR-1273g-3p | 1/19.38 |
| hsa-miR-6087 | 1/17.19 |
| hsa-miR-6126 | 1/18.14 |
| hsa-miR-6780b-5p | 1/19.16 |
| hsa-miR-7704 | 1/11.96 |
| hsa-miR-4433b-3p | 1/11.71 |
| hsa-miR-7845-5p | 1/12.01 |
| hsa-miR-8084 | 76.74 |

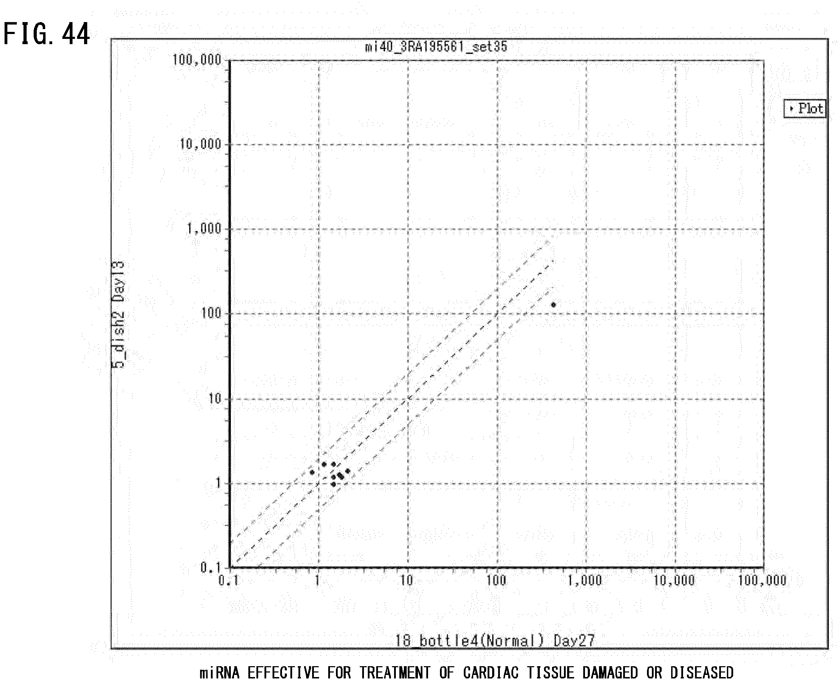

FIG. 44 represents the results of the miRNA array analyses of hsa-miR-146a, hsa-miR-210, hsa-miR-22, and hsa-miR-24, which are miRNAs described in PTL 2 and effective for treatment of a cardiac tissue damaged or diseased. With the Bottle (Normal Oxygen) culture in which the porous polyimide membrane modules were used, the production amount of hsa-miR-24-3p was found to be 3.4 times higher than with Dish_Day 13 that was just about to reach confluence, even in a long-term continuous stable culture state, and the expression amount of each of the other miRNAs was approximately the same.

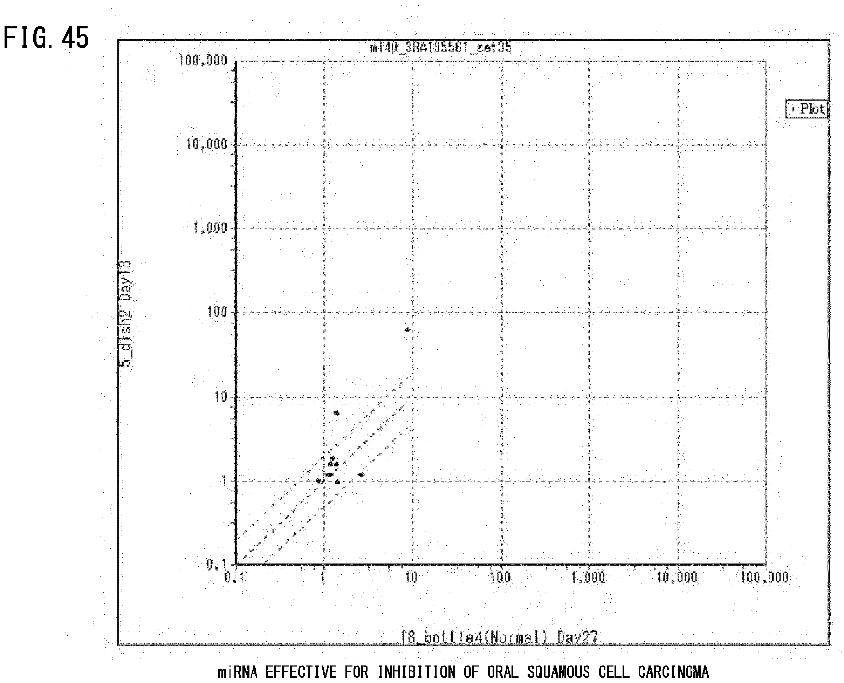

FIG. 45 represents the results of the miRNA array analyses of hsa-miR-34b, hsa-miR-132, hsa-miR-203, hsa-miR-137, and hsa-miR-193, which are miRNAs described in PTL 3 and effective for inhibition of oral squamous cell carcinoma. With Dish_Day 13 that was just about to reach confluence, the production amounts of hsa-miR-193a-5p, hsa-miR-193b-5p, and hsa-miR-193b-3p were found to be 7.0 times, 4.6 times, and 4.4 times respectively higher than with the Bottle (Normal Oxygen) culture in which the porous polyimide membrane modules were used, even in a long-term continuous stable culture state, and the expression amount of each of the other miRNAs was approximately the same.

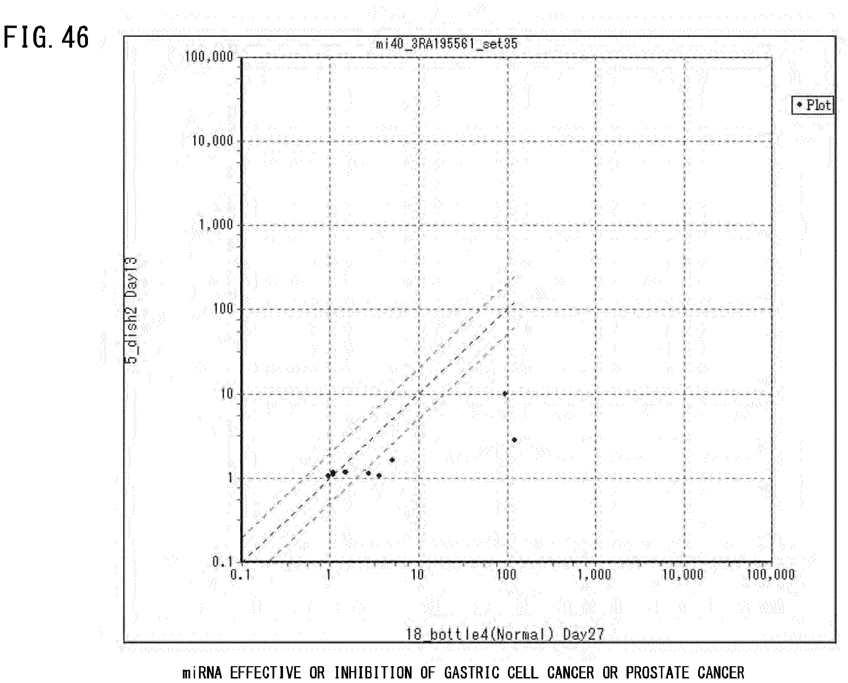

FIG. 46 represents the results of the miRNA array analyses of hsa-miR-26a/b, hsa-miR-23b, hsa-miR-27b, and hsa-miR-24-1, which are miRNAs described in NPL 6 and NPL 7 and effective for inhibition of gastric cell cancer or a prostate cancer cell. With the Bottle (Normal Oxygen) culture in which the porous polyimide membrane modules were used, the production amounts of hsa-miR-26a-5p, hsa-miR-23b-3p, hsa-miR-26a-3p, and hsa-miR-27b-3p were found to be 43.8 times, 9.7 times, 3.4 times, and 3.1 times respectively higher than with Dish Day 13 that was just about to reach confluence, even in a long-term continuous stable culture state, and the expression amount of each of the other miRNAs was approximately the same.

Comparison of exosome production amount between Comparative Example 1 (Dish Day 6) and Example 3 (Reactor (5% Oxygen)_Day 27), and miRNA array analysis

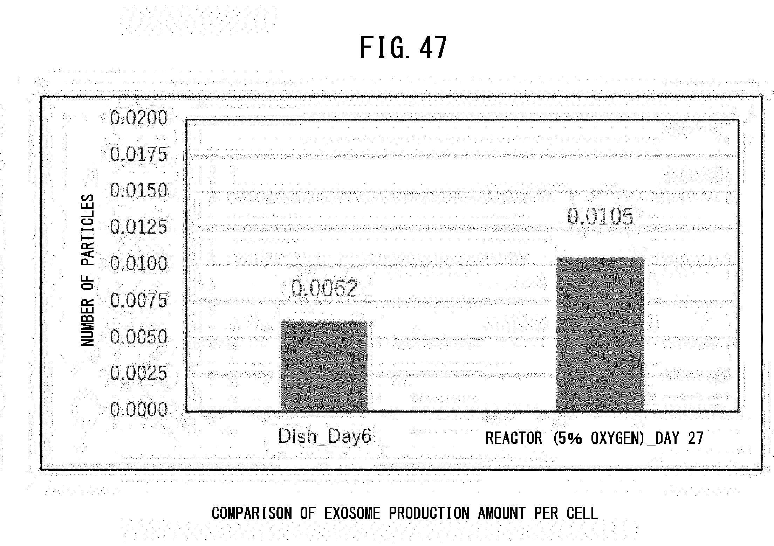

FIG. 47 represents a comparison of the exosome production amount per cell between Comparative Example 1 (Dish Day 6) and Example 3 (Reactor (5% oxygen)_Day 27). With the Reactor (5% oxygen) culture in which the porous polyimide membrane modules were used, the exosome production performance was found to be 1.7 times higher than with Dish_Day 6 even in a long-term continuous stable culture state.

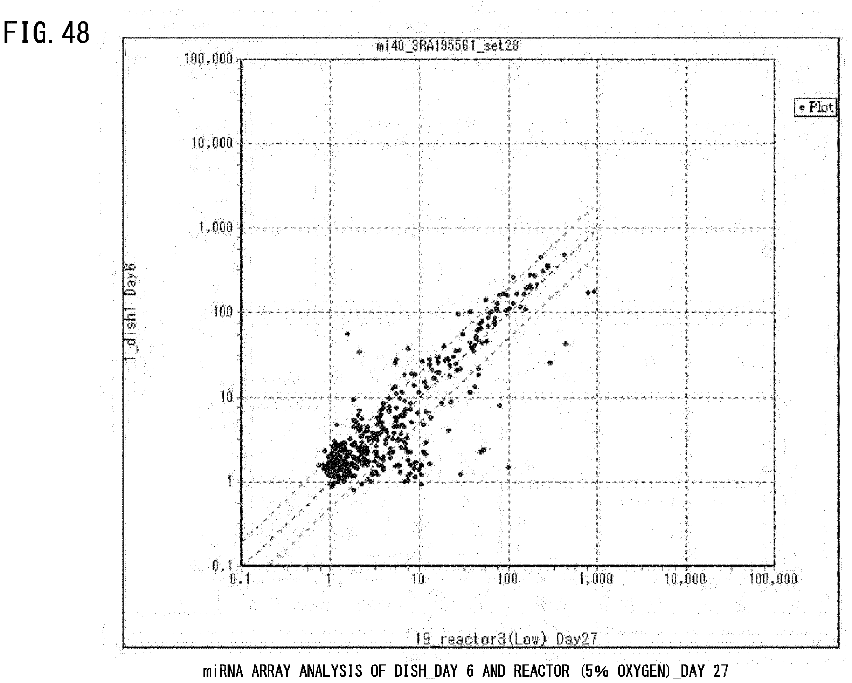

FIG. 48 represents a Scatterplot of the miRNA array analyses in Comparative Example 1 (Dish Day 6) and Example 3 (Reactor (5% oxygen)_Day 27), and Table 5 lists the miRNAs the expression ratio of which exhibited a tenfold or more difference.

[Table 5]

Table 5. miRNAs the expression ratio of which exhibited a tenfold or more difference

| microRNA Types | Expression Amount in Reactor (5% Oxygen)_Day 27 Assuming that Expression Amount in Dish_Day 6 is 1 |
|---|---|
| hsa-miR-23a-3p | 11.83 |
| hsa-miR-24a-3p | 10.32 |
| hsa-miR-214-3p | 22.27 |
| hsa-miR-1246 | 10.46 |
| hsa-miR-3124-5p | 67.93 |
| hsa-miR-3128 | 24.38 |
| hsa-miR-3201 | 22.57 |
| hsa-miR-1273g-3p | 1/15.73 |
| hsa-miR-5681 | 11.29 |
| hsa-miR-7641 | 1/35.73 |

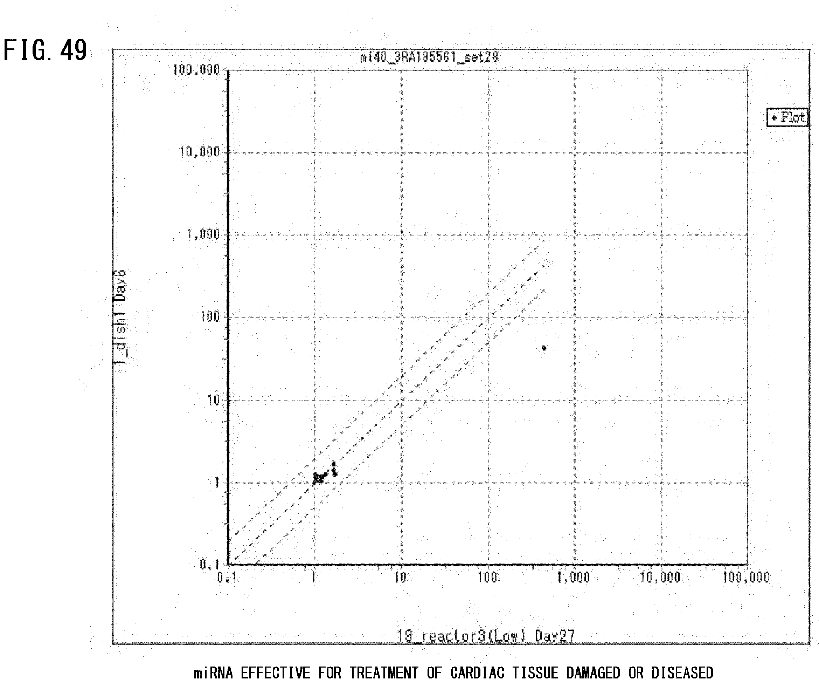

FIG. 49 represents the results of the miRNA array analyses of hsa-miR-146a, hsa-miR-210, hsa-miR-22, and hsa-miR-24, which are miRNAs described in PTL 2 and effective for treatment of a cardiac tissue damaged or diseased. With the Reactor (5% oxygen) culture in which the porous polyimide membrane modules were used, the production amount of hsa-miR-24a-3p was found to be 10.3 times higher than with Dish_Day 6 even in a long-term continuous stable culture state, and the expression amount of each of the other miRNAs was approximately the same.

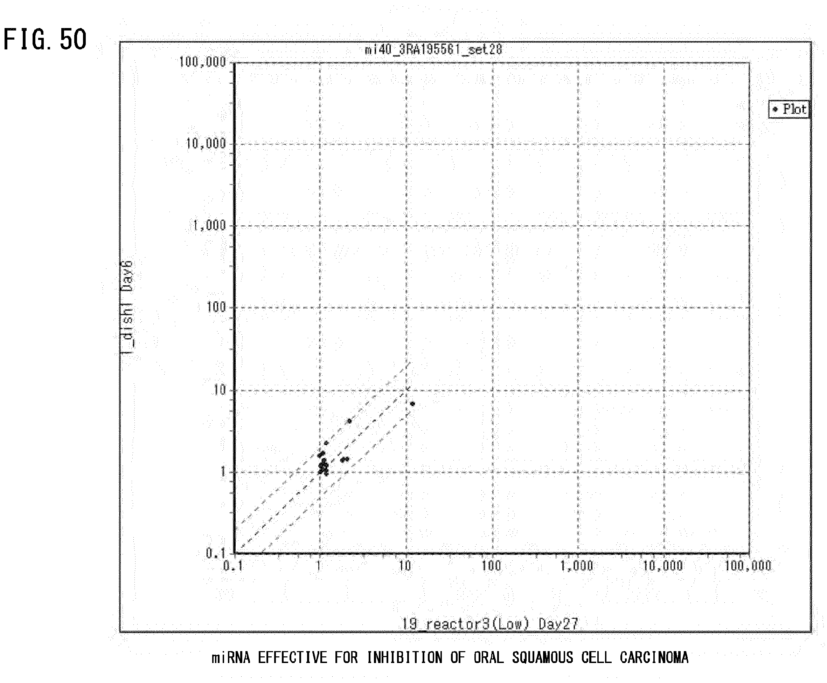

FIG. 50 represents the results of the miRNA array analyses of hsa-miR-34b, hsa-miR-132, hsa-miR-203, hsa-miR-137, and hsa-miR-193, which are miRNAs described in PTL 3 and effective for inhibition of oral squamous cell carcinoma. With the Reactor (5% oxygen) culture in which the porous polyimide membrane modules were used, the production amount was found to be the same as with Dish Day 6 even in a long-term continuous stable culture state.

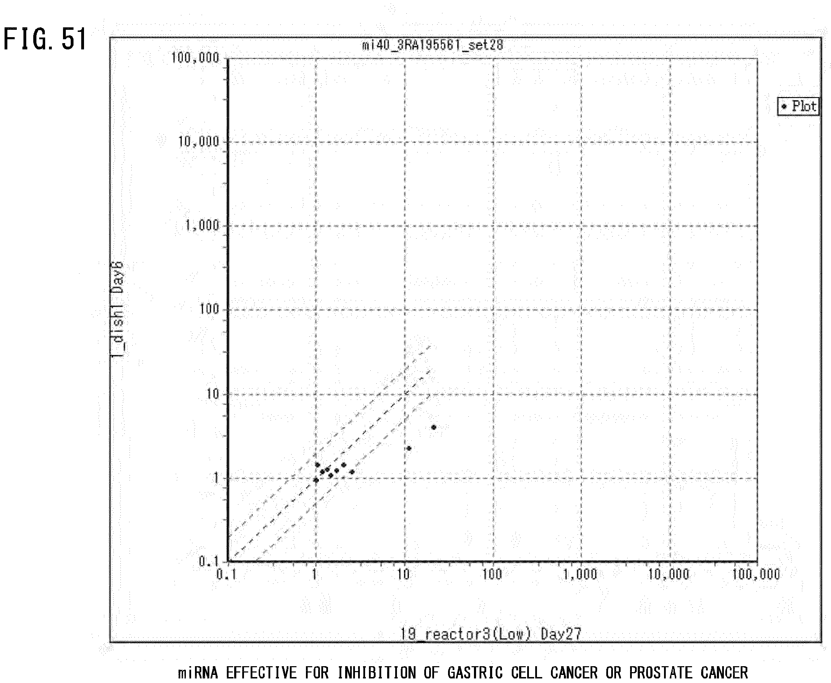

FIG. 51 represents the results of the miRNA array analyses of hsa-miR-26a/b, hsa-miR-23b, hsa-miR-27b, and hsa-miR-24-1, which are miRNAs described in NPL 6 and NPL 7 and effective for inhibition of gastric cell cancer or a prostate cancer cell. With the Reactor (5% oxygen) culture in which the porous polyimide membrane modules were used, the production amounts of hsa-miR-26a-5p and hsa-miR-23b-3p were found to be 5.0 times and 5.4 times respectively higher than with Dish_Day 6 even in a long-term continuous stable culture state, and the expression amount of each of the other miRNAs was approximately the same.

Comparison of exosome production amount between Comparative Example 1 (Dish Day 13) and Example 3 (Reactor (5% Oxygen)_Day 27), and miRNA array analysis

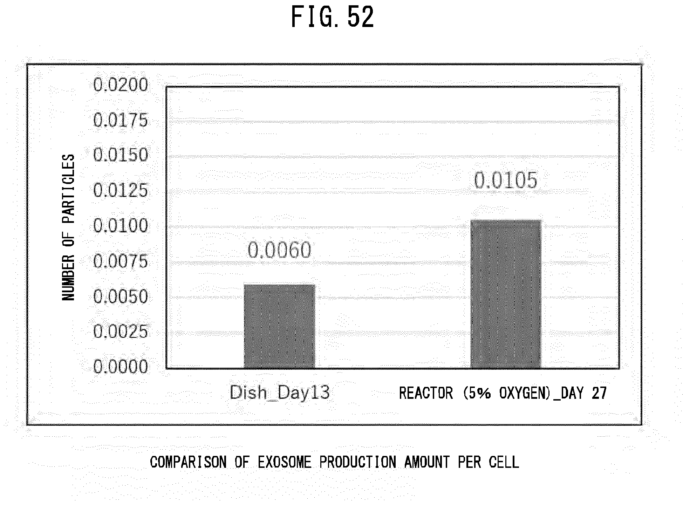

FIG. 52 represents a comparison of the exosome production amount per cell between Comparative Example 1 (Dish Day 13) and Example 3 (Reactor (5% oxygen)_Day 27). With the Reactor (5% oxygen) culture in which the porous polyimide membrane modules were used, the exosome production performance was found to be 1.8 times higher than with Dish_Day 13 even in a long-term continuous stable culture state.

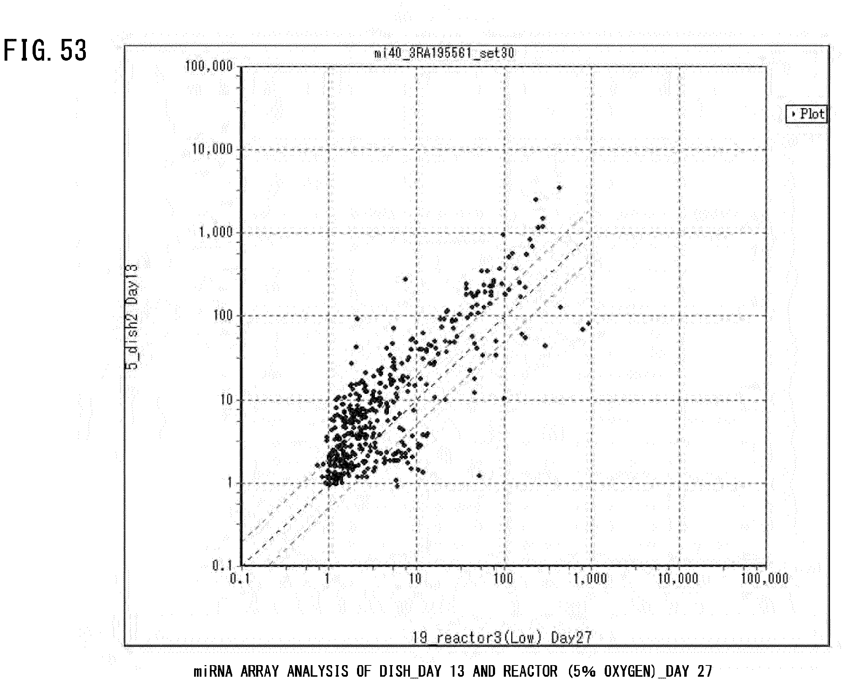

FIG. 53 represents a Scatterplot of the miRNA array analyses in Comparative Example 1 (Dish Day 13) and Example 3 (Reactor (5% oxygen)_Day 27), and Table 6 lists the miRNAs the expression ratio of which exhibited a tenfold or more difference.

[Table 6]

Table 6. miRNAs the expression ratio of which exhibited a tenfold or more difference

| microRNA Types | Expression Amount in Reactor(5% Oxygen)_Day 27 Assuming that Expression Amount in Dish_Day 13 is 1 |
| --- | --- |
| hsa-let-7e-5p | 1/14.59 |
| hsa-miR-92a-3p | 1/12.44 |
| hsa-miR-3201 | 43.49 |
| hsa-miR-3613-3p | 11.35 |
| hsa-miR-4668-5p | 11.42 |
| hsa-miR-1273g-3p | 1/43.27 |
| hsa-miR-6087 | 1/10.47 |
| hsa-miR-6126 | 1/36.77 |
| hsa-miR-6780b-5p | 1/20.50 |

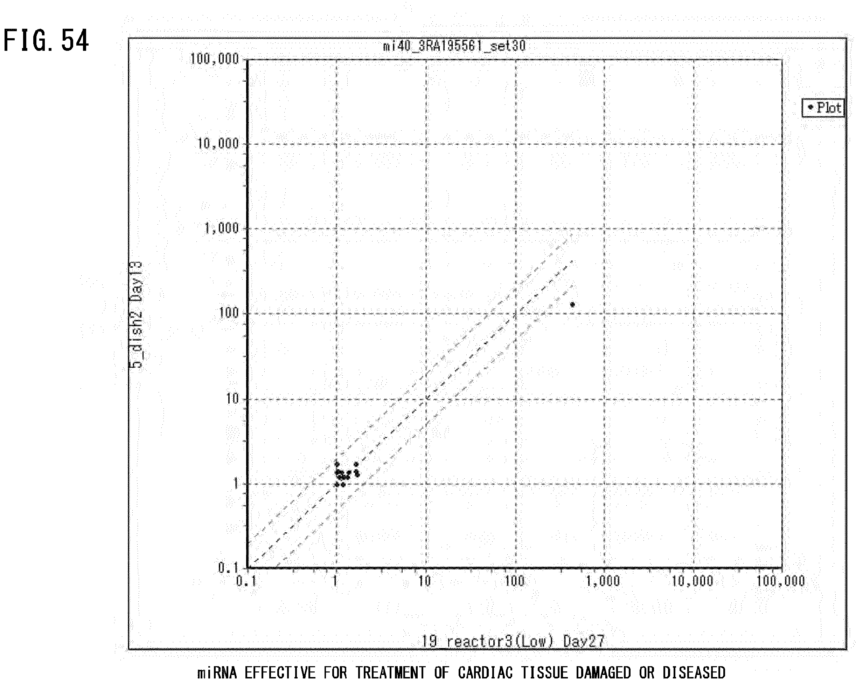

FIG. 54 represents the results of the miRNA array analyses of hsa-miR-146a, hsa-miR-210, hsa-miR-22, and hsa-miR-24, which are miRNAs described in PTL 2 and effective for treatment of a cardiac tissue damaged or diseased. With the Reactor (5% oxygen) culture in which the porous polyimide membrane modules were used, the production amount of hsa-miR-24-3p was found to be 3.4 times higher than with Dish_Day 13 that was just about to reach confluence, even in a long-term continuous stable culture state, and the expression amount of each of the other miRNAs was approximately the same.

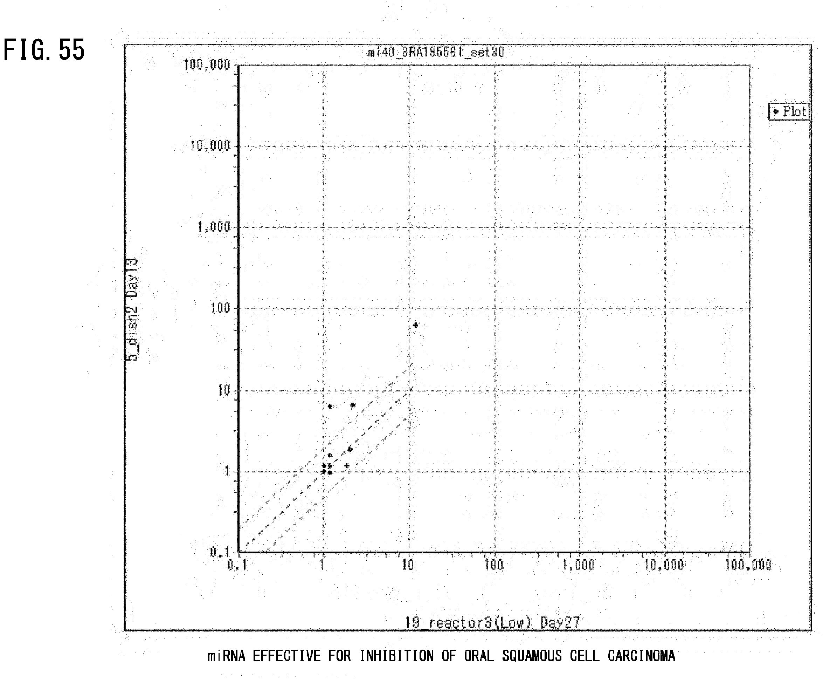

FIG. 55 represents the results of the miRNA array analyses of hsa-miR-34b, hsa-miR-132, hsa-miR-203, hsa-miR-137, and hsa-miR-193, which are miRNAs described in PTL 3 and effective for inhibition of oral squamous cell carcinoma. With Dish_Day 13 that was just about to reach confluence, the production amounts of hsa-miR-193a-5p, hsa-miR-193b-5p, and hsa-miR-193b-3p were found to be 5.2 times, 2.9 times, and 5.4 times respectively higher than with the Reactor (5% oxygen) culture in which the porous polyimide membrane modules were used, even in a long-term continuous stable culture state, and the expression amount of each of the other miRNAs was approximately the same.

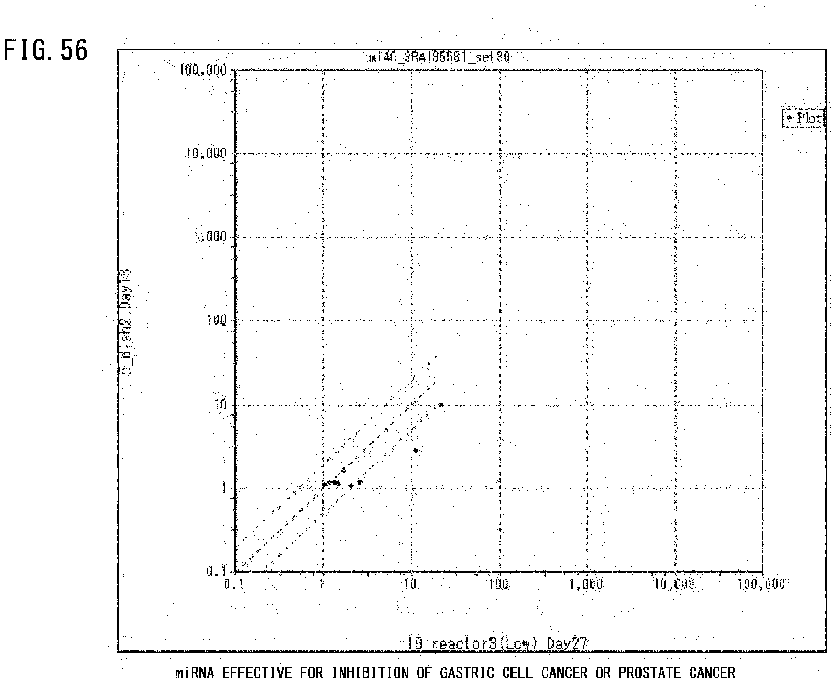

FIG. 56 represents the results of the miRNA array analyses of hsa-miR-26a/b, hsa-miR-23b, hsa-miR-27b, and hsa-miR-24-1, which are miRNAs described in NPL 6 and NPL 7 and effective for inhibition of gastric cell cancer or a prostate cancer cell. With the Reactor (5% oxygen) culture in which the porous polyimide membrane modules were used, the production amount of hsa-miR-26a-5p was found to be 4.0 times higher than with Dish_Day 13 that was just about to reach confluence, even in a long-term continuous stable culture state, and the expression amount of each of the other miRNAs was approximately the same.

Comparison of exosome production amount between Comparative Example 1 (Dish Day 6) and Example 4 (Reactor (Normal Oxygen)_Day 27), and miRNA array analysis

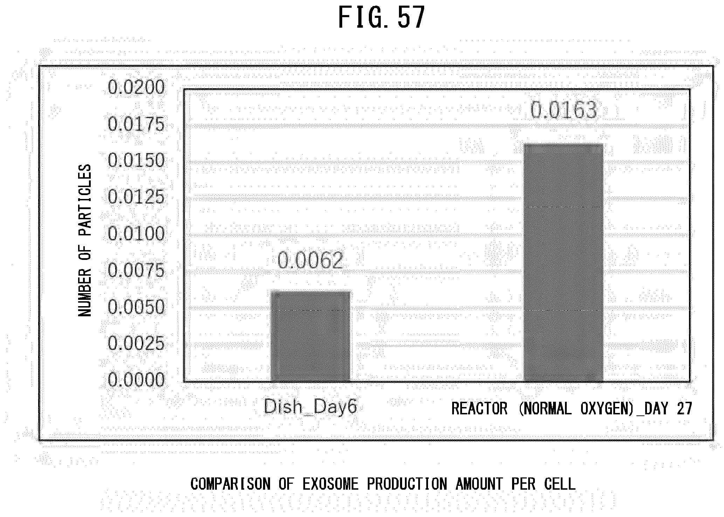

FIG. 57 represents a comparison of the exosome production amount per cell between Comparative Example 1 (Dish Day 6) and Example 4 (Reactor (Normal Oxygen)_Day 27). With the Reactor (Normal Oxygen) culture in which the porous polyimide membrane modules were used, the exosome production performance was found to be 2.6 times higher than with Dish Day 6 even in a long-term continuous stable culture state.

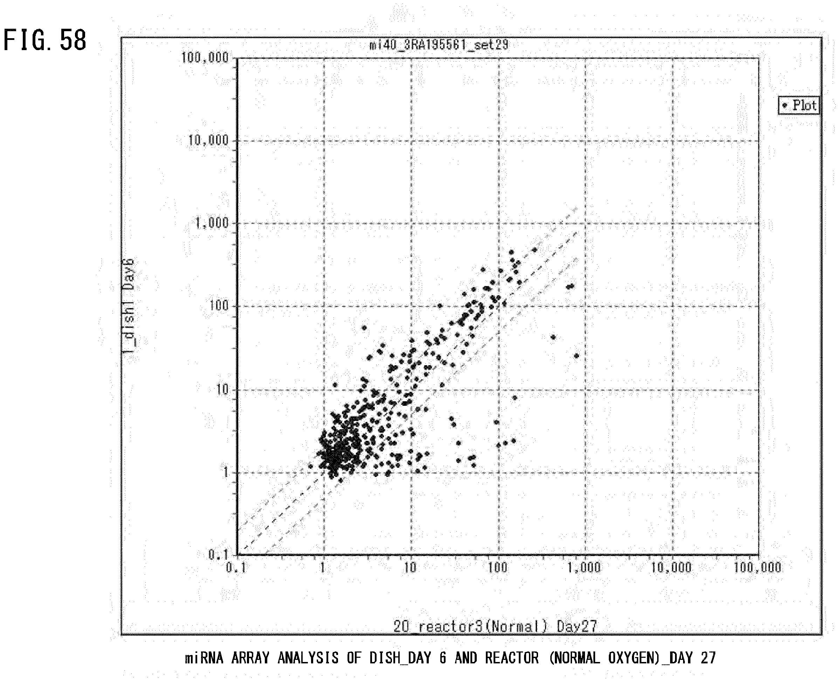

FIG. 58 represents a Scatterplot of the miRNA array analyses in Comparative Example 1 (Dish Day 6) and Example 4 (Reactor (Normal Oxygen)_Day 27). Table 7 lists the miRNAs the expression ratio of which exhibited a tenfold or more difference.

[Table 7]

Table 7. miRNAs the expression ratio of which exhibited a tenfold or more difference

| microRNA Types | Expression Amount in Reactor (Normal Oxygen)_Day 27 Assuming that Expression Amount in Dish Day 6 is 1 |
|---|---|
| hsa-miR-21a-5p | 35.17 |
| hsa-miR-23a-3p | 32.06 |
| hsa-miR-24-3p | 10.10 |
| hsa-miR-26a-5p | 54.97 |
| hsa-miR-214-3p | 15.63 |
| hsa-miR-23b-3p | 24.23 |
| hsa-miR-606 | 12.07 |
| hsa-miR-1246 | 19.88 |
| hsa-miR-548h-3p | 13.06 |
| hsa-m1R-3124-5p | 33.19 |
| hsa-miR-3128 | 43.32 |
| hsa-miR-3201 | 65.26 |
| hsa-miR-548z | 13.06 |
| hsa-miR-4423-3p | 25.95 |
| hsa-miR-7641 | 1/18.81 |
| hsa-miR-8084 | 48.44 |

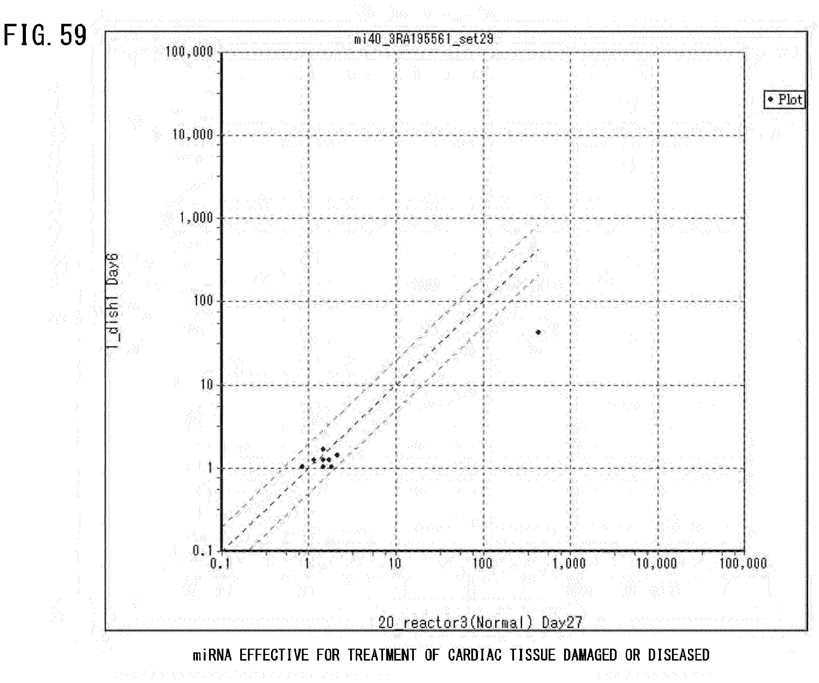

FIG. 59 represents the results of the miRNA array analyses of hsa-miR-146a, hsa-miR-210, hsa-miR-22, and hsa-miR-24, which are miRNAs described in PTL 2 and effective for treatment of a cardiac tissue damaged or diseased. With the Reactor (Normal Oxygen) culture in which the porous polyimide membrane modules were used, the production amount of hsa-miR-24-3p was found to be 10.1 times higher than with Dish_Day 6 even in a long-term continuous stable culture state, and the expression amount of each of the other miRNAs was approximately the same.

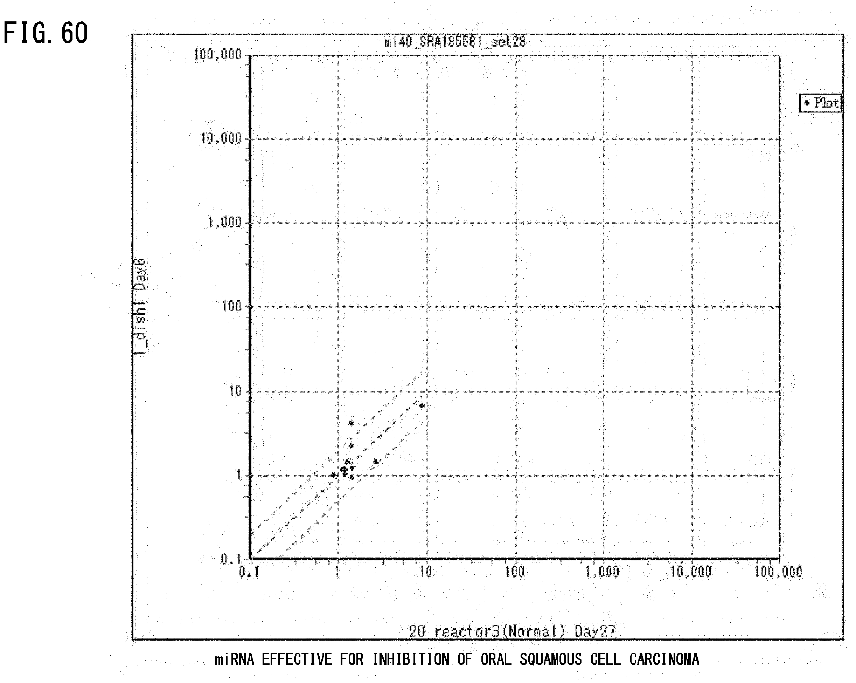

FIG. 60 represents the results of the miRNA array analyses of hsa-miR-34b, hsa-miR-132, hsa-miR-203, hsa-miR-137, and hsa-miR-193, which are miRNAs described in PTL 3 and effective for inhibition of oral squamous cell carcinoma. With the Reactor (Normal Oxygen) culture in which the porous polyimide membrane modules were used, the production amount was found to be the same as with Dish Day 6 even in a long-term continuous stable culture state.

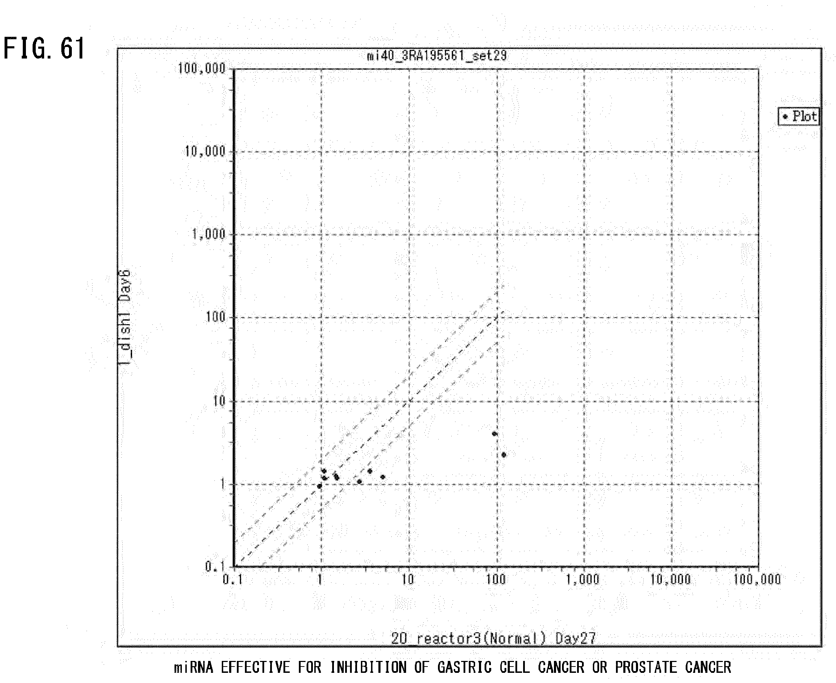

FIG. 61 represents the results of the miRNA array analyses of hsa-miR-26a/b, hsa-miR-23b, hsa-miR-27b, and hsa-miR-24-1, which are miRNAs described in NPL 6 and NPL 7 and effective for inhibition of gastric cell cancer or a prostate cancer cell. With the Reactor (Normal Oxygen) culture in which the porous polyimide membrane modules were used, the production amounts of hsa-miR-26a-5p, hsa-miR-23b-3p, and hsa-miR-27b-3p were found to be 55.0 times, 24.2 times, and 4.2 times respectively higher than with Dish_Day 6 even in a long-term continuous stable culture state, and the expression amount of each of the other miRNAs was approximately the same.

Comparison of exosome production amount between Comparative Example 1 (Dish Day 13) and Example 4 (Reactor (Normal Oxygen)_Day 27), and miRNA array analysis

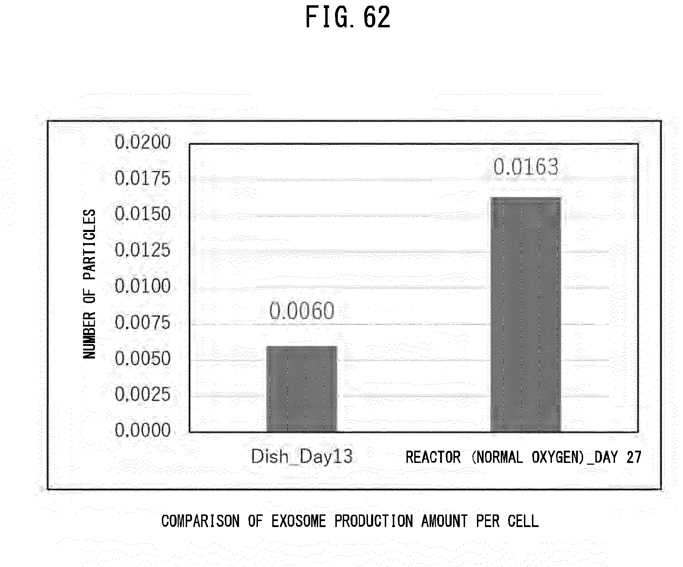

FIG. 62 represents a comparison of the exosome production amount per cell between Comparative Example 1 (Dish Day 13) and Example 4 (Reactor (Normal Oxygen)_Day 27). With the Reactor (Normal Oxygen) culture in which the porous polyimide membrane modules were used, the exosome production performance was found to be 2.7 times higher than with Dish Day 13 even in a long-term continuous stable culture state.

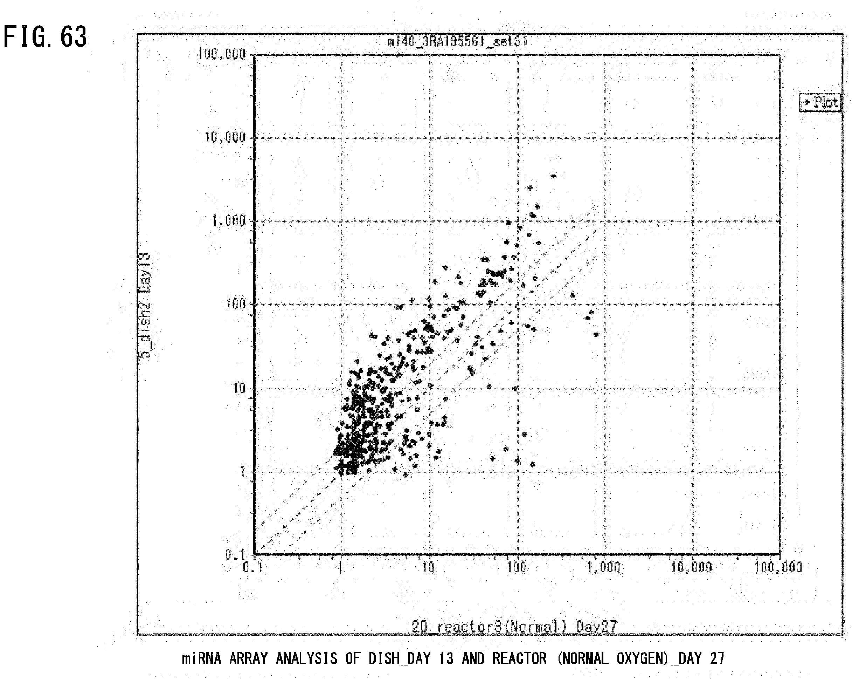

FIG. 63 represents a Scatterplot of the miRNA array analyses in Comparative Example 1 (Dish_Day 13) and Example 4 (Reactor (Normal Oxygen)_Day 27), and Table 8 lists the miRNAs the expression ratio of which exhibited a tenfold or more difference.

[Table 8]

Table 8. miRNAs the expression ratio of which exhibited a tenfold or more difference

| microRNA Types | Expression Amount in Reactor(Normal Oxygen)_Day 27 Assuming that Expression Amount in Dish Day 13 is 1 |
|---|---|
| hsa-miR-21a-5p | 37.23 |
| hsa-miR-23a-3p | 18.07 |
| hsa-miR-26a-5p | 43.80 |
| hsa-miR-199a-3p | 39.87 |
| hsa-miR-199b-3p | 39.87 |
| hsa-miR-320a | 1/16.21 |
| hsa-miR-423-5p | 1/10.48 |
| hsa-miR-92b-5p | 1/16.97 |
| hsa-miR-320c | 1/11.52 |
| hsa-miR-1207-5p | 1/10.73 |
| hsa-miR-3201 | 125.77 |
| hsa-miR-4270 | 1/17.88 |
| hsa-miR-4532 | 1/13.67 |
| hsa-miR-3960 | 1/13.24 |
| hsa-miR-4739 | 1/21.40 |
| hsa-miR-1273g-3p | 1/19.38 |
| hsa-miR-6087 | 1/17.19 |
| hsa-miR-6126 | 1/18.14 |
| hsa-miR-6780b-5p | 1/19.15 |
| hsa-miR-7704 | 1/11.96 |
| hsa-miR-4433b-3p | 1/11.71 |
| hsa-miR-7845-5p | 1/12.01 |
| hsa-miR-8084 | 76.74 |

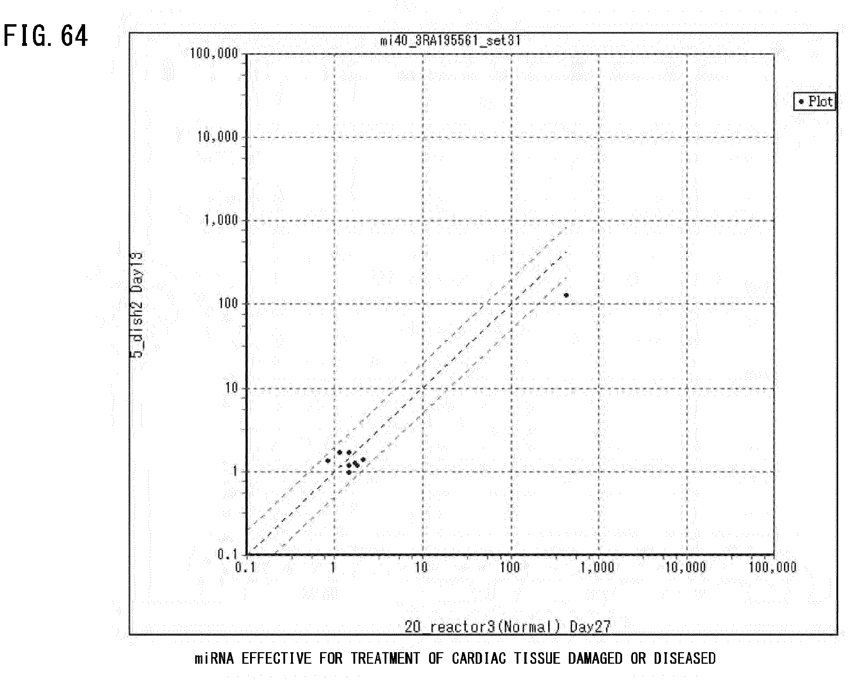

FIG. 64 represents the results of the miRNA array analyses of hsa-miR-146a, hsa-miR-210, hsa-miR-22, and hsa-miR-24, which are miRNAs described in PTL 2 and effective for treatment of a cardiac tissue damaged or diseased. With the Reactor (Normal Oxygen) culture in which the porous polyimide membrane modules were used, the production amount of hsa-miR-24-3p was found to be 3.4 times higher than with Dish_Day 13 even in a long-term continuous stable culture state, and the expression amount of each of the other miRNAs was approximately the same.

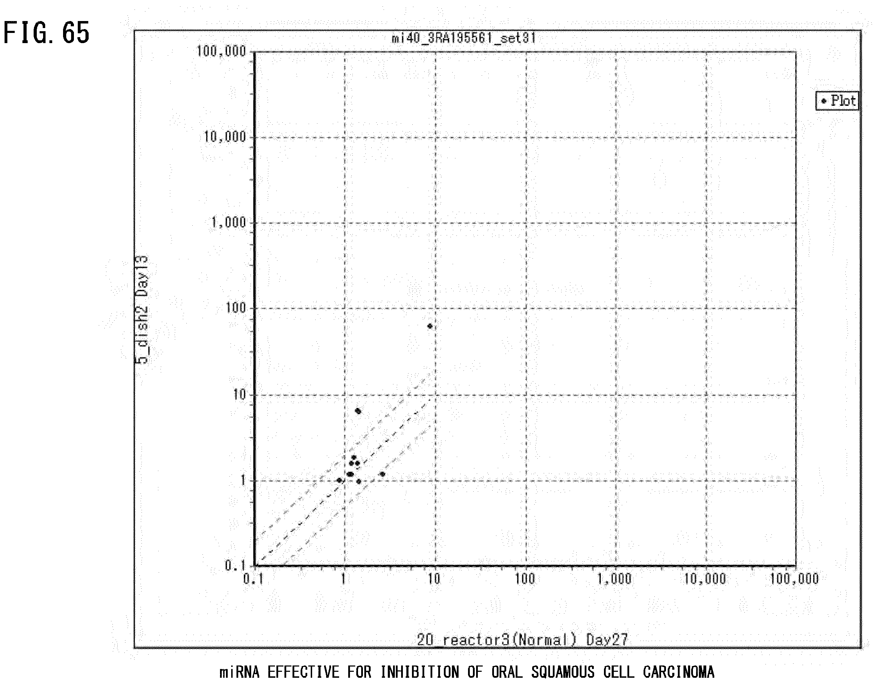

FIG. 65 represents the results of the miRNA array analyses of hsa-miR-34b, hsa-miR-132, hsa-miR-203, hsa-miR-137, and hsa-miR-193, which are miRNAs described in PTL 3 and effective for inhibition of oral squamous cell carcinoma. With the Reactor (Normal Oxygen) culture in which the porous polyimide membrane modules were used, the production amounts of hsa-miR-193a-5p, hsa-miR-193b-5p, and hsa-miR-193b-3p were found to be 7.0 times, 4.6 times, and 4.4 times respectively higher than with Dish Day 13 that was just about to reach confluence, even in a long-term continuous stable culture state, and the expression amount of each of the other miRNAs was approximately the same.

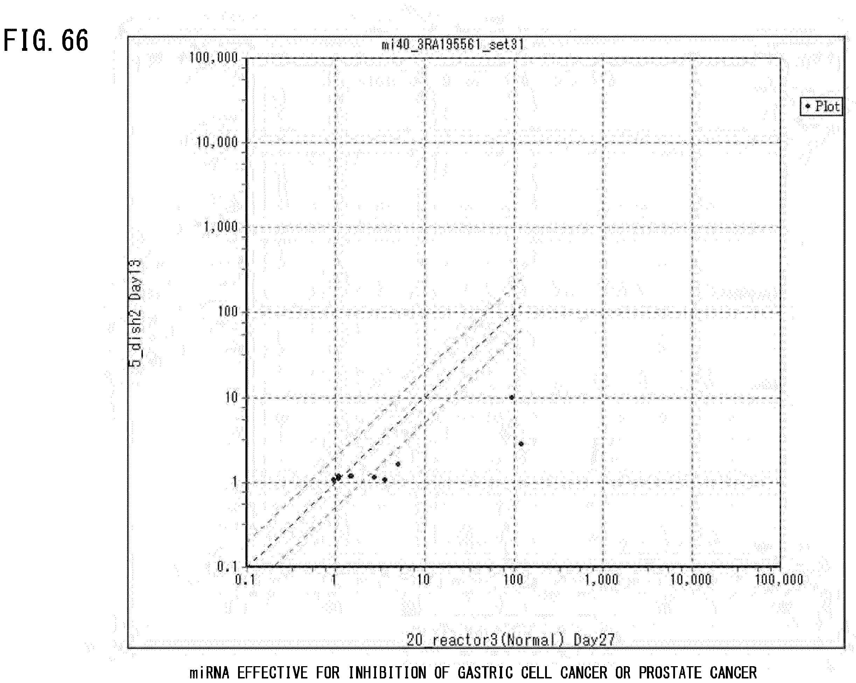

FIG. 66 represents the results of the miRNA array analyses of hsa-miR-26a/b, hsa-miR-23b, hsa-miR-27b, and hsa-miR-24-1, which are miRNAs described in NPL 6 and NPL 7 and effective for inhibition of gastric cell cancer or a prostate cancer cell. With the Reactor (Normal Oxygen) culture in which the porous polyimide membrane modules were used, the production amounts of hsa-miR-26a-5p, hsa-miR-23b-3p, hsa-miR-26b-3p, and hsa-miR-27b-3p were found to be 43.8 times, 9.7 times, 3.4 times, and 3.2 times respectively higher than with Dish_Day 13 that was just about to reach confluence, even in a long-term continuous stable culture state, and the expression amount of each of the other miRNAs was approximately the same.

Example 6: Cell culture method using modules in WAVE reactor

In a manner similar to Example 5, 60 modules were preliminarily wetted for 2 days or more with a culture medium (Ham's F-12 culture medium manufactured by Fujifilm Wako Pure Chemical Corporation and containing glutamine and phenol red), and 250 mL of culture medium (Xeno-free culture medium, KBM ADSC-4R, manufactured by Kohjin Bio Co., Ltd.) was poured into a WAVE culture bag (CELLBAG DISPOSABLE BIOREACTOR Part # CB0002L11-33) manufactured by GE and containing the 60 modules. The bag was placed in a WAVE reactor (Ready-ToProcess WAVE25) manufactured by GE, and then the modules were shaken through stirring for approximately 1 hour under conditions: 5% $CO_2$, a temperature of 37°C, a vibration speed of 15 rpm, and a vibration angle of 9°.

Passage 4 mesenchymal stem cells (21Y Male, Lot 70011721, manufactured by ATCC) cultured in a dish were peeled off using "Trypsin-EDTA (0.05%), phenol red" manufactured by Gibco, and suspended with 11 mL of culture medium (Xeno-free culture medium, KBM ADSC-4R, manufactured by Kohjin Bio Co., Ltd.). The cell suspension containing $1.0 \times 10^4$ cells ($1.1 \times 10^7$ cells in total) per $cm^2$ of the area of the porous polyimide membrane as a culture substrate in the module was disseminated. The cells were allowed to be adsorbed under the same vibration conditions as during the module wetting. A long-term culture was then started.

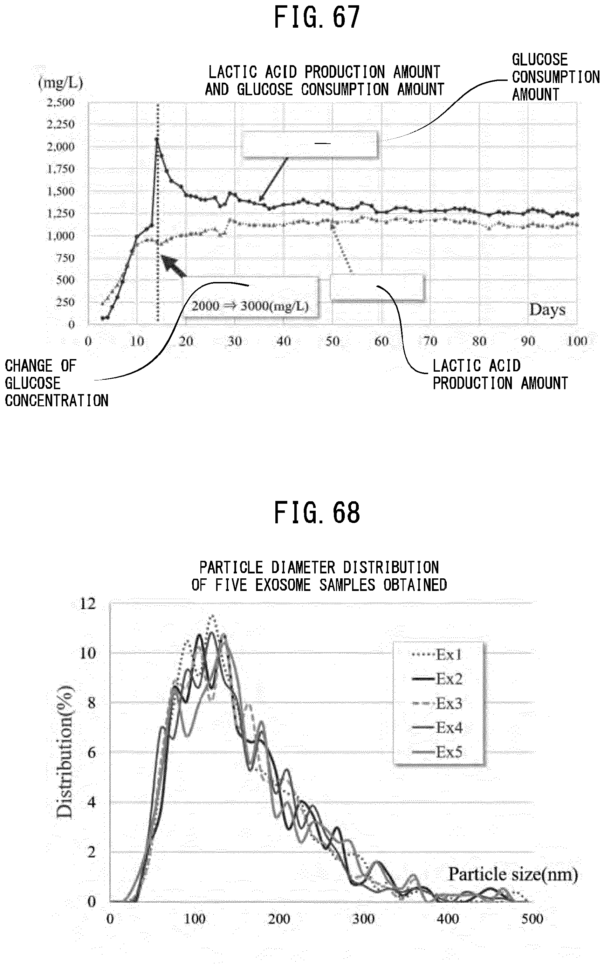

After an oscillating culture was continued under the same conditions for 3 days, the perfusion function (bag weight control function) of the reactor was utilized to withdraw the cell culture solution from the WAVE culture bag manufactured by GE, via the Harvest tube pump at a speed of 110 mL/day, and a culture medium (glucose-adjusted KBM ADSC-4R) having the same volume as the above-mentioned culture medium withdrawn was liquid-transferred into the WAVE culture bag manufactured by GE, via the Feed tube pump. In this regard, the liquid-transfer speed of the harvest pump attached to WAVE25 is too high, and thus, the culture medium was collected using a microtube pump set FP100-1 manufactured by As One Corporation. Using CedexBio manufactured by F. Hoffmann-La Roche, Ltd., the cell growth behavior of the culture medium collected was observed with reference to changes in the metabolism parameters, with the result that stable cell proliferation and growth over a long period of time was observed. Additionally, when necessary, color reaction based on Cell Counting Kit-8 manufactured by Dojindo Laboratories was utilized to measure the total number of cells grown in the modules in the bag and check how the cells were growing. Changes over time in the glucose consumption amount and the lactic acid production amount are illustrated in FIG. 67. In this regard, in cases where the glucose was consumed in an increased amount and was predicted to become insufficient, the glucose concentration was adjusted by adding glucose solution to avoid the depletion thereof.

To measure the number of exosomes produced per day and contained in the cell culture solution collected, the culture media collected for approximately 20 days each were combined into 1 batch each, and five collected solutions were prepared. Table 9 includes the results obtained by measuring each solution in accordance with the procedures in the above-mentioned "Exosome Amount Measurement Using Zeta-potential/Particle Diameter Distribution Measurement Device" after foreign substances were removed from the solution using a 0.2 μm filter. With the five collected solutions which represented 100 culture days, the continuation of stable exosome production was verified.

[Table 9]

| Evaluation of Exosome Amount of Collected Stock Solution | | |
|---|---|---|
| Name of Sample | Culture Period | Number of Exosome Particles ZEECOM Measurement |
| W25-MSC 01 EX1 | Day 0 to Day 24 | 1322 |
| W25-MSC 01 EX2 | Day 25 to Day 42 | 1309 |
| W25-MSC 01 EX3 | Day 43 to Day 63 | 1109 |
| W25-MSC 01 EX4 | Day 64 to Day 82 | 1323 |
| W25-MSC 01 EX5 | Day 83 to Day 100 | 1224 |

Furthermore, these five samples (culture collected solutions) were concentrated approximately 30-fold to 50-fold using a Tangential Flow filtration method (VIVAFLOW 200 100,000 MWCO HY, manufactured by Sartorius AG), and then supplemented with Total Exosome Isolation Reagent (from cell culture media), manufactured by Thermo Fisher Scientific Inc., in an amount equal to half of the amount of the concentrated solution. The resulting mixture was stirred, left to stand under refrigeration overnight, and centrifuged (at 10000 G/60 min). The resulting precipitate was separated. Onto this precipitate, DPBS was poured in an amount approximately equal to the amount of the solution from which the precipitate had been removed. The precipitate was suspended and dissolved, and furthermore, a 0.2 μm filter was used to remove foreign substances from the resulting mixture to obtain an exosome solution. In this manner, five exosome solution samples for the respective culture periods were obtained.

These five samples were measured in accordance with the procedures in the above-mentioned "Exosome Amount Measurement Using Zeta-potential/Particle Diameter Distribution Measurement Device". In addition, the exosome amount was quantitated in terms of the protein amount of CD63, using a CD9/CD63ELISA kit (product number_EXH0102EL, manufactured by Cosmo Bio Co., Ltd.) for quantitating a human-derived exosome. The evaluation results of the exosome amount are listed in Table 10, and the results of the exosome particle size distribution are illustrated in FIG. 68.

[Table 10]

| Evaluation of Exosome Amount of Purified and Concentrated Solution Obtained | | | |
|---|---|---|---|
| Name of Sample | Culture Period | Number of Exosome Particles ZEECOM Measurement | ELISA Measured Values* (pg/ml) |
| W25-MSC 01 EX1 | Day 0 to Day 24 | 32063 | 2523 |
| W25-MSC 01 EX2 | Day 25 to Day 42 | 117850 | 7725 |
| W25-MSC 01 EX3 | Day 43 to Day 63 | 73769 | 4284 |
| W25-MSC 01 EX4 | Day 64 to Day 82 | 92408 | 3938 |
| W25-MSC 01 EX5 | Day 83 to Day 100 | 71850 | 4003 |
| * CD9/CD63 Exosome ELISA Kit, Human Cat No EXH0102EL, manufactured by Cosmo Bio Co., Ltd. | | | |

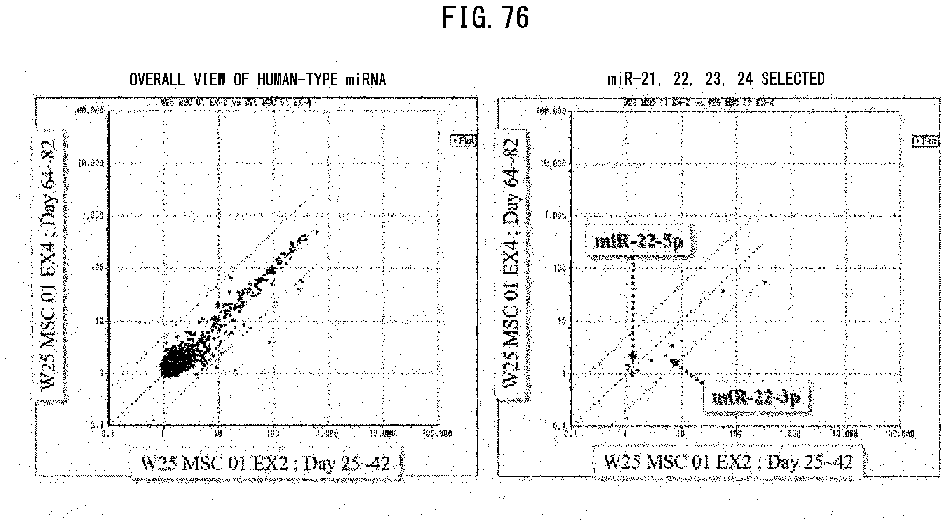

Each of the exosome samples was subjected to miRNA array analysis using GeneChip (trademark) miRNA 4.0 Array described in Example 5. The gene expression comparison analysis performed resulted in verifying stable gene expression in the exosomes, independent of the culture period (FIG. 76).

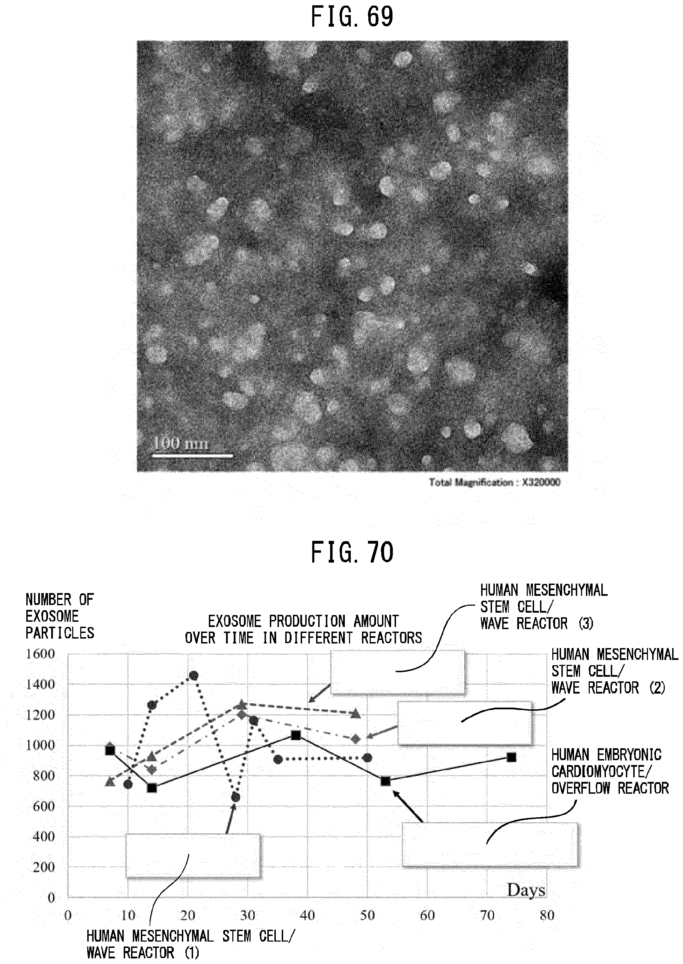

The exosomes obtained by the same methods as in the above-mentioned Examples were purified using a gel filtration column (HiLoad 16/600 Superdex 200 prep grade), concentrated using an ultrafiltration film, and then, observed under a TEM (transmission electron microscope). The sample solution was adsorbed onto a Cu mesh with a carbon support film, then washed, stained, washed, and dried to make a sample for TEM observation. The sample was photographed using a Model JEM-2100F field-emission transmission electron microscope manufactured by JEOL Ltd. An example of measurement is depicted in FIG. 69.

Example 7: Cell culture method using modules in WAVE reactor

In a manner similar to Example 5, 70 modules were preliminarily wetted for 2 days or more with a culture medium (Ham's F-12 culture medium manufactured by Fujifilm Wako Pure Chemical Corporation and containing glutamine and phenol red), and 250 mL of culture medium (Xeno-free culture medium, KBM ADSC-4R, manufactured by Kohjin Bio Co., Ltd.) was poured into a WAVE culture bag (CELLBAG DISPOSABLE BIOREACTOR Part # CB0002L11-03) manufactured by GE and containing the 70 modules. The bag was placed in a WAVE reactor (Xuri Cell Expansion System W5) manufactured by GE, and then the modules were shaken through stirring for approximately 1 hour under conditions: 5% $CO_2$, a temperature of 37°C, a vibration speed of 11 rpm, and a vibration angle of 9°.

Passage 2 mesenchymal stem cells (21Y Male, Lot 70011721, manufactured by ATCC) cultured in a dish were peeled off using "Trypsin-EDTA (0.05%), phenol red" manufactured by Gibco, and suspended with 11 mL of culture medium (Xeno-free culture medium, KBM ADSC-4R, manufactured by Kohjin Bio Co., Ltd.). The cell suspension con-

taining $1.0 \times 10^4$ cells ($1.3 \times 10^7$ cells in total) per $cm^2$ of the area of the porous polyimide membrane as a culture substrate in the module was disseminated. The cells were allowed to be adsorbed under the same vibration conditions as during the module wetting. A long-term culture was then started. Additionally, at the same time as the mesenchymal stem cells were disseminated in the module, cells were disseminated for subculture in a Collagen Type 1 Coat dish manufactured by IWAKI, and cultured for an exosome production comparison experiment.

After an oscillating culture was continued under the same conditions for 3 days, the perfusion function (bag weight control function) of the reactor accessory (Xuri Cell Expansion System W5 Perfusion Controller) was utilized to withdraw approximately 20 mL of the cell culture solution from the WAVE culture bag manufactured by GE, via the Harvest tube pump approximately every 3 hours, and a culture medium (glucose-adjusted KBM ADSC-4R) having the same volume as the above-mentioned culture medium withdrawn was liquid-transferred into the WAVE culture bag manufactured by GE, via the Feed tube pump to exchange the solution at 150 mL/day. Using CedexBio manufactured by F. Hoffmann-La Roche, Ltd., the cell growth behavior of the culture medium collected was observed with reference to changes in the metabolism parameters, with the result that stable cell proliferation and growth over a long period of time was observed. Additionally, on Day 14, Day 21, Day 28, and Day 50, color reaction based on Cell Counting Kit-8 manufactured by Dojindo Laboratories was utilized to measure the per-$cm^2$ cell density of human mesenchymal stem cells grown in the modules in the bag and the total number of cells in the bag, and check how the cells were growing. The measurement results are depicted in Table 11.

[Table 11]

Table 11. Comparison of Number of Cells Grown among Culture Periods

| Culture Period (days) | Cell Density (cells/$cm^2$) | Total Number of Cells (cells) |
|---|---|---|
| 14 | $6.1 \times 10^4$ | $7.7 \times 10^7$ |
| 21 | $8.4 \times 10^4$ | $1.1 \times 10^8$ |
| 28 | $9.4 \times 10^4$ | $1.2 \times 10^8$ |
| 50 | $8.5 \times 10^4$ | $8.5 \times 10^7$ |

To measure the number of exosomes produced per day and contained in the cell culture solution collected, the samples were taken out at regular intervals, foreign substances were removed from the solution using a 0.2 $\mu$m filter, and each solution was measured in accordance with the procedures in the above-mentioned "Exosome Amount Measurement Using Zeta-potential/Particle Diameter Distribution Measurement Device". The measurement results including the results of operation with other cells are illustrated in FIG. 70. The human mesenchymal stem cell WAVE reactor (1) in the drawing represents the results of the experiment. As verified clearly from the results, this culture method allowed stable exosome production to be continued for 50 culture days.

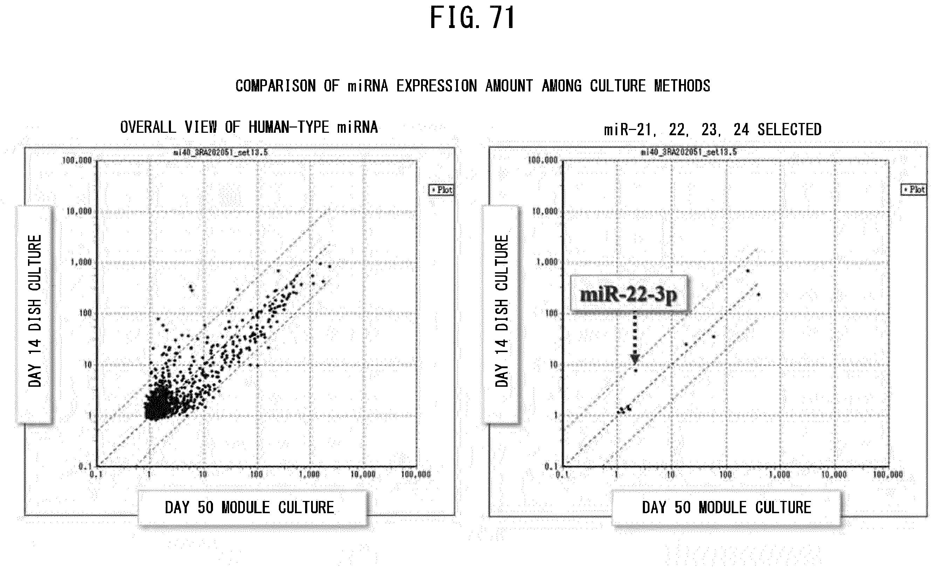

In addition, an miRNA array analysis using GeneChip (trademark) miRNA 4.0 Array described in Example 5 was performed for exosome comparison using the following: a culture supernatant collected on culture Day 14 from the mesenchymal stem cells cultured in the Xeno-free culture medium, KBM ADSC-4R, manufactured by Kohjin Bio Co., Ltd. in a Collagen Type 1 Coat dish manufactured by IWAKI; and the culture medium collected on Day 50 from the continuous culture system using the above-mentioned WAVE reactor. The results are illustrated in FIG. 71.

A comparison of the miRNA expression amount between two different culture conditions is illustrated as a distribution, wherein the amounts are leveled between the same amounts of RNA. The whole distribution of the human-type miRNA is illustrated on the left side, and the partial distribution limited to miR-21 to -24 is illustrated on the right side. As verified clearly by the comparison in the graph on the left side, the expression amount of miRNA is generally larger in the module culture system even though the RNA amounts are the same. In addition, contrary to this tendency, the expression amount of miR-22-3p is larger in the plate culture than in the module culture, as verified. As is known, miR-22 is a miRNA the expression amount of which increases with the cellular aging. As discovered, the expression amount of the aging-related miRNA was three or more times more in the plate culture system although the period of the module culture was three or more times longer than the period of the plate-based plane culture used as a subject of comparison. As verified, the module culture has made it possible to culture cells for a long period of time with the characteristics maintained favorably.

Example 8: Cell culture method using modules in WAVE reactor

In a manner similar to Example 5, 55 modules were preliminarily wetted for 2 days or more with a culture medium (Ham's F-12 culture medium manufactured by Fujifilm Wako Pure Chemical Corporation and containing glutamine and phenol red), and 250 mL of culture medium (Xeno-free culture medium, KBM ADSC-4R, manufactured by Kohjin Bio Co., Ltd.) was poured into two WAVE culture bags (CELLBAG DISPOSABLE BIOREACTOR Part # CB0002L11-03) manufactured by GE and containing the 55 modules and 55 type-2 modules respectively. The bags were placed in a WAVE reactor (Xuri Cell Expansion System W5) manufactured by GE, and then the modules were shaken through stirring for approximately 1 hour under conditions: 5% $CO_2$, a temperature of 37°C, a vibration speed of 13 rpm, and a vibration angle of 9°.

Passage 2 mesenchymal stem cells (25Y Male, Lot 00451491, manufactured by Lonza Group AG) cultured in a dish were peeled off using "Trypsin-EDTA (0.05%), phenol red" manufactured by Gibco, and suspended with 11 mL of culture medium (Xeno-free culture medium, KBM ADSC-4R, manufactured by Kohjin Bio Co., Ltd.). The cell suspension containing $1.0 \times 10^4$ cells ($1.3 \times 10^7$ cells in total) per $cm^2$ of the area of the porous polyimide membrane as a culture substrate in the module was disseminated. The cells were allowed to be adsorbed under the same vibration conditions as during the module wetting. A long-term culture was then started.

After an oscillating culture was continued under the same conditions for 3 days, the perfusion function (bag weight control function) of the reactor accessory (Xuri Cell Expansion System W5 Perfusion Controller) was utilized to withdraw approximately 20 mL of the cell culture solution from the WAVE culture bag manufactured by GE, via the Harvest tube pump approximately every 3 hours, and a culture medium (glucose-adjusted KBM ADSC-4R) having the same volume as the above-mentioned culture medium withdrawn was liquid-transferred into the WAVE culture bag manufactured by GE, via the Feed tube pump to exchange the solution at 150 mL/day. Using CedexBio manufactured by F. Hoffmann-La Roche, Ltd., the cell growth behavior of the culture medium collected was observed with reference to changes in the metabolism parameters, with the result that stable cell proliferation and growth over a long period of time was observed. Additionally, on Day 7, Day 14, Day 21, Day 29, and Day 48, color reaction based on Cell Counting Kit-8 manufactured by Dojindo Laboratories was utilized to exhibit the per-$cm^2$ number of cells grown in the modules in the bag as the cell density, measure the number of exosome particles discharged into the collected culture medium during measurement, and thereby check how the cells were growing and how the continuous exosome production behavior was. The measurement results are depicted in Table 12.

[Table 12]

Table 12. Comparison of Cell Density of Cells Grown and Comparison of Exosome Production Amount among Culture Periods

| Culture Period (days) | Normal Module | | Type 2 Module | |
|---|---|---|---|---|
| | Cell Density (cells/$cm^2$) | Number of Exosome Particles ZEECOM Measurement | Cell Density (cells/$cm^2$) | Number of Exosome Particles ZEECOM Measurement |
| 7 | $4.3 \times 10^4$ | 975 | $4.0 \times 10^4$ | 767 |
| 14 | $8.7 \times 10^4$ | 841 | $9.1 \times 10^4$ | 932 |
| 21 | $9.7 \times 10^4$ | Not Evaluated | $1.2 \times 10^4$ | not evaluated |
| 29 | $1.2 \times 10^4$ | 1200 | $1.3 \times 10^4$ | 1272 |
| 48 | not evaluated | 1041 | not evaluated | 1211 |

In this regard, the exosome production behavior is illustrated in FIG. 70 to verify consistency with the other experiments. The experiment results of the usual modules are illustrated by the human mesenchymal stem cell WAVE reactor (2) in the drawing, and the experiment results of the type 2 modules are illustrated by the same type of reactor (3).

Example 9: Long-term module culture of osteoblast in overflow reactor

Preliminarily, 50 modules were wetted with a culture medium (Ham's F-12 culture medium containing glutamine and phenol red; manufactured by Fujifilm Wako Pure Chemical Corporation) for 2 days or more, and the modules were added into an overflow reactor culture vessel, into which 100 mL of culture medium (Xeno-free culture medium, KBM ADSC-4, manufactured by Kohjin Bio Co., Ltd.) was poured. Then, the vessel was placed in a $CO_2$ incubator (aniCell) manufactured by N-BIOTEK, having a built-in humidification-handling shaker, and set to 5% $CO_2$ and 37°C. Then, the resulting mixture was shaken through stirring at a rotation speed of 80 rpm for approximately 1 hour.

Passage 6 human osteoblasts (Y30 female_439Z037.2) manufactured by Promo Cell GmbH and cultured in a cell culture FALCON dish manufactured by Corning Incorporated were peeled off using "Trypsin-EDTA (0.05%), phenol red" manufactured by Gibco, and centrifuged to collect the cells, which were suspended with 9 ml of the culture medium (KBM ADSC-4). The cell suspension containing $1.0 \times 10^4$ cells ($9.0 \times 10^6$ cells in total) per $cm^2$ of the area of the porous polyimide membrane as a culture substrate in the module was poured into an overflow reactor culture vessel. Then, the cells started to be adsorbed and to be cultured under stirring conditions at a rotation speed of 80 rpm.

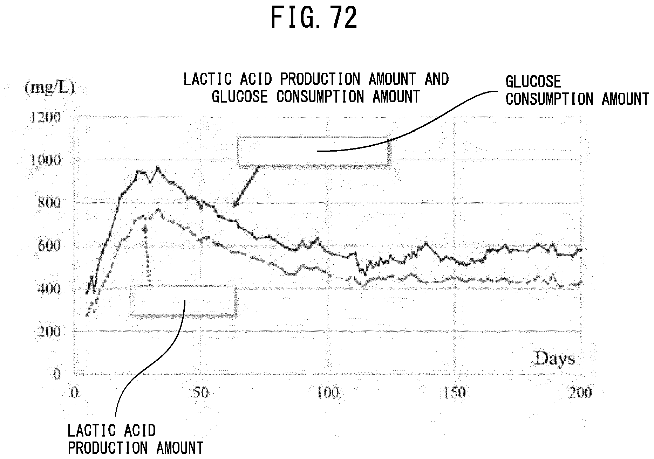

After the adsorption of the cell, the culture medium solution (glucose-adjusted KBM ADSC-4) started to be transferred into an overflow reactor culture vessel using a tube pump at a speed of 100 mL/day. The culture medium overflown through the culture medium collection outlet disposed in the wall face of the culture vessel was collected into a collection liquid pool bottle. A long-term cell culture was performed as follows: using CedexBio manufactured by F. Hoffmann-La Roche, Ltd., the cell growth behavior of the culture medium collected was observed with reference to changes in the metabolism parameters of the solution collected; and the medium feeding speed·and the rotation speed were changed, as appropriate, in accordance with the consumption of glucose and the like of the cell. Changes over time in the glucose consumption amount and the lactic acid production amount are illustrated in FIG. 72. Stable glucose consumption and lactic acid production were observed over a period as long as 200 days.

To measure the exosome production amount in the culture medium collected, each solution was measured in accordance with the procedures in the above-mentioned "Exosome Amount Measurement Using Zeta-potential/Particle Diameter Distribution Measurement Device". The measurement results are tabulated in Table 13. In the same manner as the metabolism behavior, stable exosome production behavior was verified over a period as long as 100 days or more.

The culture media collected throughout the culture period were sorted by the periods, filtrated through a filter, furthermore concentrated using a Tangential Flow filtration method (VIVAFLOW 200 100,000 MWCO HY manufactured by Sartorius AG), and supplemented with Total Exosome Isolation Reagent (from cell culture media) manufactured by Thermo Fisher Scientific Inc. The resulting mixture was stirred, left to stand under refrigeration, and centrifuged (at 10000 G/60 min) to obtain an exosome precursor in the form of a precipitate. Onto this, DPBS was poured in a suitable amount, and a 0.2 $\mu$m filter was used to remove foreign substances from the resulting mixture to obtain an exosome solution. In this manner, exosome solutions for the respective culture periods were obtained.

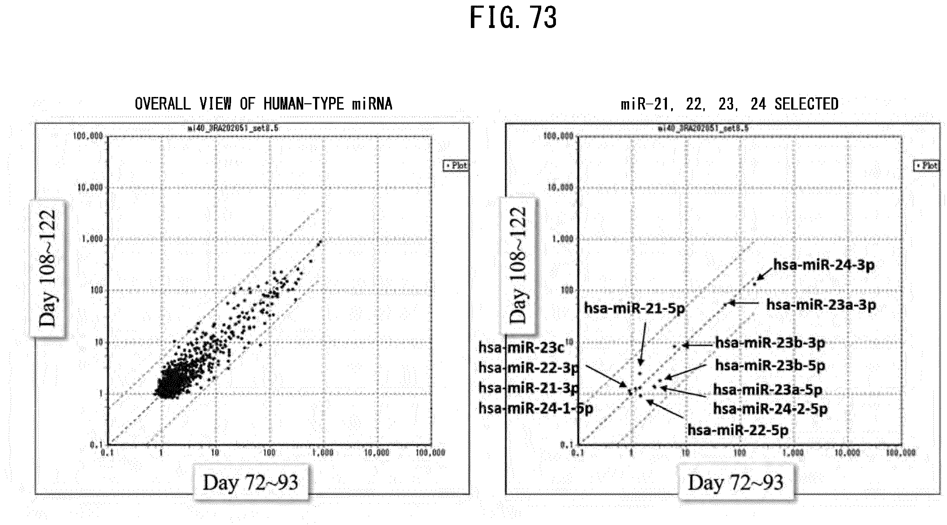

From among these, two samples of Day 72 to Day 93 and Day 108 to Day 122 were selected, and used for the expression amount comparison of each miRNA in the miRNA array measurement. The results are illustrated in FIG. 73.

[Table 13]

Table 13. Correlation between Culture Period and Exosome Production Behavior

| Culture Period (days) | Number of Exosome Particles ZEECOM Measurement |
|---|---|
| 24 | 1413 |
| 56 | 491 |
| 87 | 826 |
| 122 | 937 |
| 133 | 1180 |

As clear from the test results, the expression amount of miRNA is hardly different between both the exosome samples, revealing that exosomes having stable quality were obtained over a long period of time.

Example 10: Long-term module culture of osteoblast in WAVE reactor

Preliminarily, 70 modules were wetted for 2 days or more with a culture medium (Ham's F-12 culture medium manufactured by Fujifilm Wako Pure Chemical Corporation and containing glutamine and phenol red), and the modules were added into a WAVE reactor culture vessel, into which 100 mL of culture medium (Xeno-free culture medium, KBM ADSC-4R, manufactured by Kohjin Bio Co., Ltd.) was poured. A culture medium (Xeno-free culture medium, KBM ADSC-4R, manufactured by Kohjin Bio Co., Ltd.) in an amount of 250 mL was poured into a WAVE culture bag (CELLBAG DISPOSABLE BIOREACTOR Part # CB0002L11-03) manufactured by GE The bag was placed in a WAVE reactor (Xuri Cell Expansion System W5) manufactured by GE, and then the modules were shaken through stirring for approximately 1 hour under conditions: 5% $CO_2$, a temperature of 37°C, a vibration speed of 15 rpm, and a vibration angle of 9°.

Passage 3 human osteoblasts (Y30 female_439Z037.2) manufactured by Promo Cell GmbH and cultured in a cell culture FALCON dish manufactured by Corning Incorporated were peeled off using "Trypsin-EDTA (0.05%), phenol red" manufactured by Gibco, and centrifuged to collect the cells, which were suspended with 13 ml of the culture medium (KBM ADSC-4R). The cell suspension containing $1.0 \times 10^4$ cells ($1.3 \times 10^7$ cells in total) per $cm^2$ of the area of the porous polyimide membrane as a culture substrate in the module was poured into a WAVE reactor culture vessel. Then, the cells started to be adsorbed and to be cultured under vibration conditions: a vibration speed of 15 rpm and a vibration angle of 9°.

After an oscillating culture was continued under the same conditions for 3 days, the perfusion function (bag weight control function) of the reactor accessory (Xuri Cell Expansion System W5 Perfusion Controller) was utilized to withdraw approximately 20 mL of the cell culture solution from the WAVE culture bag manufactured by GE, via the Harvest tube pump approximately every 3 hours, and a culture medium (glucose-adjusted KBM ADSC-4R) having the same volume as the above-mentioned culture medium withdrawn was liquid-transferred into the WAVE culture bag manufactured by GE, via the Feed tube pump to exchange the solution at 150 mL/day. Using CedexBio manufactured by F. Hoffmann-La Roche, Ltd., the cell growth behavior of the culture medium collected was observed with reference to changes in the metabolism parameters, with the result that stable cell proliferation and growth over a long period of time was observed. Additionally, on Day 8, Day 22, Day 36, and Day 58, color reaction based on Cell Counting Kit-8 manufactured by Dojindo Laboratories was utilized to measure the per-$cm^2$ cell density of human osteoblasts grown in the modules in the bag and the total number of cells in the bag, and check how the cells were growing. The results are illustrated in Table 14. In addition, to measure the exosome production amount in the culture medium collected, each solution was measured in accordance with the procedures in the above-mentioned "Exosome Amount Measurement Using Zeta-potential/Particle Diameter Distribution Measurement Device". The measurement results are tabulated in Table 15. In the same manner as the metabolism behavior, stable exosome production behavior was verified over a period as long as 130 days or more.

[Table 14]

Table 14. Comparison of Number of Cells Grown among Culture Periods

| Culture Period (days) | Cell Density (cells/$cm^2$) | Total Number of Cells (cells) |
|---|---|---|
| 8 | $1.7 \times 10^4$ | $2.1 \times 10^7$ |
| 22 | $1.9 \times 10^4$ | $2.4 \times 10^7$ |
| 36 | $2.5 \times 10^4$ | $3.2 \times 10^7$ |
| 58 | $2.6 \times 10^4$ | $3.3 \times 10^7$ |

[Table 15]

Table 15. Correlation between Culture Period and Exosome Production Behavior

| Culture Period (days) | Number of Exosome Particles ZEECOM Measurement |
|---|---|
| 14 | 838 |
| 22 | 967 |
| 50 | 759 |
| 140 | 558 |

Example 11: Long-term module culture of embryonic cardiomyocyte in overflow reactor

Preliminarily, 40 modules were wetted with a culture medium (Ham's F-12 culture medium containing glutamine and phenol red; manufactured by Fujifilm Wako Pure Chemical Corporation) for 2 days or more, and the modules were added into an overflow reactor culture vessel, into which 100 mL of culture medium (Xeno-free culture medium, KBM ADSC-4, manufactured by Kohjin Bio Co., Ltd.) was poured. Then, the vessel was placed in a $CO_2$ incubator (aniCell) manufactured by N-BIOTEK, having a built-in humidification-resistant shaker, and set to 5% $CO_2$ and 37°C. Then, the resulting mixture was shaken through stirring at a rotation speed of 80 rpm for approximately 1 hour.

Passage 1 human embryonic cardiomyocytes (Lot No. 21757) manufactured by ScienCell Research Laboratories, Inc. and cultured in a cell culture FALCON dish manufactured by Corning Incorporated were peeled off using "Trypsin-EDTA (0.05%), phenol red" manufactured by Gibco, and centrifuged to collect the cells, which were suspended with 7.2 ml of the culture medium (KBM ADSC-4). The cell suspension containing $1.0 \times 10^4$ cells ($7.2 \times 10^6$ cells in total) per $cm^2$ of the area of the porous polyimide membrane as a culture substrate in the module was poured into an overflow reactor culture vessel. Then, the cells started to be adsorbed and to be cultured under stirring conditions at a rotation speed of 80 rpm.

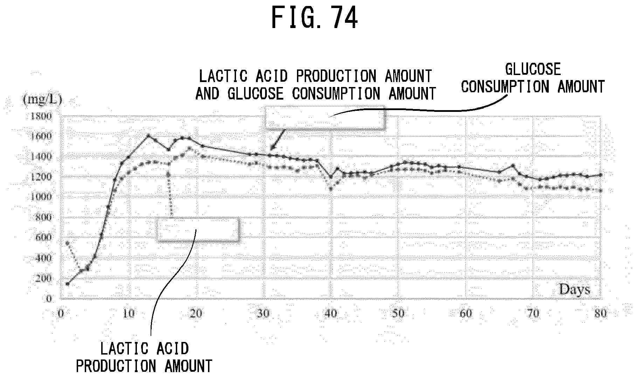

After 24 hours, the culture medium solution (glucose-adjusted KBM ADSC-4) started to be transferred into an overflow reactor culture vessel using a tube pump at a speed of 100 mL/day. The culture medium overflown through the culture medium collection outlet disposed in the wall face of the culture vessel was collected into a collection liquid pool bottle. A long-term cell culture was performed as follows: using CedexBio manufactured by F. Hoffmann-La Roche, Ltd., the cell growth behavior of the culture medium collected was observed with reference to changes in the metabolism parameters of the solution collected; and the medium feeding speed·and the rotation speed were changed, as appropriate, in accordance with the consumption of glucose and the like of the cell. Changes over time in the glucose consumption amount and the lactic acid production amount are illustrated in FIG. 74. Stable glucose consumption and lactic acid production were observed over a period as long as 80 days.

On Day 14, Day 38, Day 93, and Day 114, color reaction based on Cell Counting Kit-8 manufactured by Dojindo Laboratories was utilized to measure the per-$cm^2$ cell density of human embryonic cardiomyocytes grown in the modules in the bag and the total number of cells in the reactor, and check how the cells were growing. The results are illustrated in Table 16. From the beginning, the measurement exhibited very high proliferation, thus revealing that substantially the same value was maintained as the cell density throughout the culture period. The same behavior was assumed also from the glucose consumption amount and lactic acid production amount.

To measure the exosome production amount in the culture medium collected, each solution was measured in accordance with the procedures in the above-mentioned "Exosome Amount Measurement Using Zeta-potential/Particle Diameter Distribution Measurement Device". The measurement results are tabulated in Table 17. In the same manner as the metabolism behavior, stable exosome production behavior was verified over a period as long as 100 days or more.

[Table 16]

| Culture Period (days) | Cell Density (cells/cm$^2$) | Total Number of Cells (cells) |
|---|---|---|
| 14 | $3.6 \times 10^4$ | $2.6 \times 10^7$ |
| 38 | $2.4 \times 10^4$ | $1.7 \times 10^7$ |
| 93 | $3.5 \times 10^4$ | $2.5 \times 10^7$ |
| 114 | $3.4 \times 10^4$ | $2.4 \times 10^7$ |

[Table 17]

| Culture Period (days) | Number of Exosome Particles ZEECOM Measurement |
|---|---|
| 14 | 722 |
| 38 | 1068 |
| 53 | 767 |
| 74 | 924 |

Example 12: Long-term module culture of embryonic cardiomyocyte in WAVE reactor

Preliminarily, 50 type-2 modules were wetted for 2 days or more with DPBS (manufactured by Fujifilm Wako Pure Chemical Corporation), and the modules were added into a WAVE culture bag (CELLBAG DISPOSABLE BIOREACTOR Part # CB0002L11-03), manufactured by GE, into which 250 mL of culture medium (Xeno-free culture medium, KBM ADSC-4R, manufactured by Kohjin Bio Co., Ltd.) was poured. The bag was placed in a WAVE reactor (Xuri Cell Expansion System W5) manufactured by GE, and then the modules were shaken through stirring for approximately 1 hour under conditions: 5% $CO_2$, a temperature of 37°C, a vibration speed of 15 rpm, and a vibration angle of 9°.

Passage 4 human embryonic cardiomyocytes (Lot No. 21757) manufactured by ScienCell Research Laboratories, Inc. and cultured in a cell culture FALCON dish manufactured by Corning Incorporated were peeled off using "Trypsin-EDTA (0.05%), phenol red" manufactured by Gibco, and centrifuged to collect the cells, which were suspended with 10 ml of the culture medium (KBM ADSC-4R). The cell suspension containing $1.0 \times 10^4$ cells ($1.0 \times 10^7$ cells in total) per $cm^2$ of the area of the porous polyimide membrane as a culture substrate in the module was poured into a WAVE reactor culture vessel. Then, the cells started to be adsorbed and to be cultured under vibration conditions: a vibration speed of 11 rpm and a vibration angle of 7°.

After an oscillating culture was continued under the same conditions for 3 days, the perfusion function (bag weight control function) of the reactor accessory (Xuri Cell Expansion System W5 Perfusion Controller) was utilized to withdraw approximately 20 mL of the cell culture solution from the WAVE culture bag manufactured by GE, via the Harvest tube pump approximately every 3 hours, and a culture medium (glucose-adjusted KBM ADSC-4R) having the same volume as the above-mentioned culture medium withdrawn was liquid-transferred into the WAVE culture bag manufactured by GE, via the Feed tube pump to exchange the solution at 150 mL/day. Using CedexBio manufactured by F. Hoffmann-La Roche, Ltd., the cell growth behavior of the culture medium collected was observed with reference to changes in the metabolism parameters, with the result that stable cell proliferation and growth over a long period of time was observed. Additionally, on Day 7, Day 14, Day 21, and Day 35, color reaction based on Cell Counting Kit-8 manufactured by Dojindo Laboratories was utilized to measure the per-$cm^2$ cell density of human embryonic cardiomyocytes grown in the modules in the bag and the total number of cells in the bag, and verify the favorable cell growth state peculiar to the embryonic cardiomyocyte. The results are illustrated in Table 18.

[Table 18]

| Culture Period (days) | Cell Density (cells/cm$^2$) | Total Number of Cells (cells) |
|---|---|---|
| 7 | $1.4 \times 10^4$ | $1.4 \times 10^7$ |
| 14 | $8.1 \times 10^4$ | $8.1 \times 10^7$ |
| 21 | $7.8 \times 10^4$ | $7.8 \times 10^7$ |
| 35 | $7.9 \times 10^4$ | $7.9 \times 10^7$ |

Additionally, to measure the exosome production amount in the culture medium collected, the culture media discharged were sorted into equivalents, one for approximately every 20 days, to prepare three collected stock solution batches. These were filtrated through a filter, and measured in accordance with the procedures in the above-mentioned "Exosome Amount Measurement Using Zeta-potential/Particle Diameter Distribution Measurement Device" in the same manner as in Example 6. In addition, the exosome production amount was measured as the protein amount of CD63 by an ELISA method. The measurement results are tabulated in Table 19. Stable exosome production behavior was verified, except during the culture start period.

[Table 19]

| Name of Sample | Culture Period | Number of Exosome Particles ZEECOM Measurement | ELISA Measured Values* (pg/ml) |
|---|---|---|---|
| W5-CMF 01 EX1 | Day 0 to Day 17 | 1807 | 169 |
| W5-CMF 01 EX2 | Day 18 to Day 34 | 1512 | 335 |
| W5-CMF 01 EX3 | Day 35 to Day 56 | 1537 | 406 |

Example 13: Chondrocyte module culture using Erlenmeyer flask and porous membrane fragments

Preliminarily, 5 type-2 modules were wetted with DPBS (manufactured by Fujifilm Wako Pure Chemical Corporation) for two days or more, and the modules were added into each of a 125-mL Erlenmeyer flask and a 250-mL Erlenmeyer flask, which are both manufactured by Thermo Fisher Scientific Inc. Together with these flasks, a 125-ml square PET-made storage bottle (with a 45-mm cap) manufactured by Corning, Inc. and containing sterilized porous polyimide membrane fragments (1 mm × 1 mm), 120 cm$^2$, was prepared. Into the Erlenmeyer flasks, 20 mL of culture medium (Xeno-free culture medium, KBM ADSC-4R, manufactured by Kohjin Bio Co., Ltd.) the glucose value of which was adjusted to 3000 mg/L was poured. Into the square Corning bottle containing porous membrane fragments, 30 ml of the same kind of culture medium was poured.

Passage 4 human chondrocytes (Y30 female_439Z037.3) manufactured by Promo Cell GmbH and cultured in an IWAKI Collagen I Coat dish were peeled off using "Trypsin-EDTA (0.05%), phenol red" manufactured by Gibco, and centrifuged to collect the cells, which were suspended with the culture medium (glucose-adjusted KBM ADSC-4R). A cell suspension was poured at a suspension cell density of $1.0 \times 10^6$ cells per ml of the cell suspension, wherein the cell suspension contained $1.0 \times 10^4$ cells per cm$^2$ of the area of the porous polyimide membrane as a culture substrate in the module and per cm$^2$ of the area of the porous polyimide membrane fragments ($1.0 \times 10^6$ cells in total for the Erlenmeyer flasks, and, $1.2 \times 10^6$ cells in total for the porous membrane fragments). The culture in the Erlenmeyer flasks and the culture of chondrocytes in the porous membrane fragments were performed with a shake culture vessel in the above-mentioned aniCell and with a stationary culture using a $CO_2$ incubator respectively.

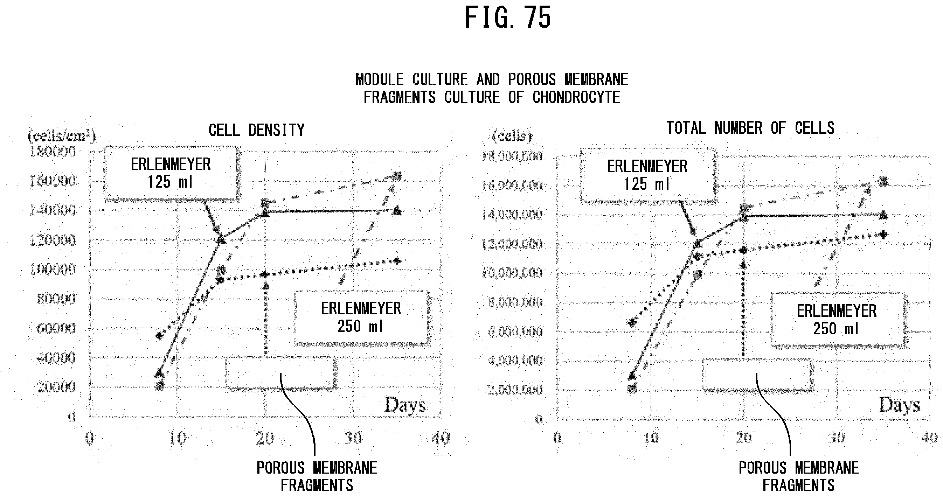

On the next day after the culture, 10 ml of glucose-adjusted KBM ADSC-4R culture medium was further added into the Erlenmeyer flasks. The resulting mixture was subjected to orbital shaking culture at 80 rpm using the above-mentioned aniCell, and the culture was continued, during which the culture medium was exchanged approximately twice a week. The module culture (5 type-2 modules) in the Erlenmeyer flasks and the culture in the porous membrane fragments (porous polyimide membrane fragments, 120 cm$^2$) were both performed using 30 ml of culture medium. On Day 8, Day 15, Day 20, and Day 35, color reaction based on Cell Counting Kit-8 manufactured by Dojindo Laboratories was utilized to measure the total number of the cells grown in the modules in each reactor and the cells grown in the porous membrane fragments. The cell growth state where human chondrocytes were favorable was verified with reference to the proliferation behavior peculiar to each reactor. The cell density and the total number of cells are illustrated in FIG. 75. The results have revealed that large amounts of cells can be easily cultured in diverse forms stably for a long period of time, using a small culture vessel.

In addition, the exosome production amount in the collected culture medium on culture Day 19 was measured, using an ELISA method, with the exosome production amount regarded as the protein amount of CD63. After three-day pooling, the exosome production was found to be 218 pg/ml, 141 pg/ml, and 117 pg/ml in the modules used in the 125-ml Erlenmeyer flask vessel, in the modules used in the 250-ml vessel, and in the culture system having porous membrane fragments respectively. Efficient and stable exosome production was verified in the intermittent culture system as well as in the above-mentioned continuous culture system.

**Claims**

1.  A method for producing an exosome using a cell, the method comprising the steps of:

    applying the cell to a cell culture module to culture the cell; and
    allowing the cell to produce the exosome,
    wherein the cell culture module comprises:

    a porous polymer membrane; and
    a casing having two or more medium flow inlets/outlets and containing the porous polymer membrane.

2.  The method according to claim 1,

    wherein the porous polymer membrane is a three-layer structure porous polymer membrane having a surface layer A and a surface layer B, the surface layers having a plurality of pores, and a macrovoid layer sandwiched between the surface layers A and B,
    wherein an average pore diameter of the pores present in the surface layer A is smaller than an average pore diameter of the pores present in the surface layer B,
    wherein the macrovoid layer has a partition wall bonded to the surface layers A and B, and a plurality of macrovoids surrounded by the partition wall and the surface layers A and B,

wherein the pores in the surface layers A and B communicate with the macrovoid, and
wherein the porous polymer membrane is contained within the casing with:

(i) the two or more independent porous polymer membranes being aggregated;
(ii) the porous polymer membranes being folded up;
(iii) the porous polymer membranes being wound into a roll-like shape; and/or
(iv) the porous polymer membranes being tied together into a rope-like shape.

3. The method according to claim 1 or 2, wherein the diameter of the medium flow inlet/outlet is larger than the diameter of the cell, and smaller than the diameter at which the porous polymer membranes flow out.

4. The method according to any one of claims 1 to 3, wherein the casing has a mesh-like structure.

5. The method according to any one of claims 1 to 4, wherein the casing consists of an inflexible material.

6. The method according to any one of claims 1 to 5, wherein the porous polymer membrane has a plurality of pores having an average pore diameter of 0.01 to 100 $\mu$m.

7. The method according to any one of claims 2 to 6, wherein an average pore diameter of the surface layer A is 0.01 to 50 $\mu$m.

8. The method according to any one of claims 2 to 7, wherein an average pore diameter of the surface layer B is 20 to 100 $\mu$m.

9. The method according to any one of claims 1 to 8, wherein a total membrane thickness of the porous polymer membrane is 5 to 500 $\mu$m.

10. The method according to any one of claims 1 to 9, wherein the porous polymer membrane is a porous polyimide membrane.

11. The method according to claim 10, wherein the porous polyimide membrane is a porous polyimide membrane comprising a polyimide derived from tetracarboxylic dianhydride and diamine.

12. The method according to claim 10 or 11, wherein the porous polyimide membrane is a colored porous polyimide membrane that is obtained by molding a polyamic acid solution composition comprising a polyamic acid solution derived from tetracarboxylic dianhydride and diamine, and a coloring precursor, and subsequently heat-treating the resultant composition at 250°C or higher.

13. The method according to any one of claims 1 to 12, wherein the step of culturing a cell is performed under stationary culture conditions.

14. The method according to any one of claims 1 to 12, wherein the step of culturing a cell is performed under rotating or stirring culture conditions.

15. The method according to any one of claims 1 to 14, wherein the step of culturing a cell is performed continuously.

16. The method according to any one of claims 1 to 15,
wherein the step of culturing a cell is performed in a cell culture device placed in an incubator, the cell culture device comprising:

a culture unit that contains the cell culture module configured to support the cell, and comprises a medium supply port and a medium discharge port; and
a culture medium supply unit comprising:

a culture medium storage container;
a medium supply line; and
a liquid-transfer pump configured to liquid-transfer a culture medium via the medium supply line, wherein a first end of the medium supply line is in contact with the culture medium in the culture medium storage container, and a second end of the medium supply line communicates with the culture unit via the medium supply port of the culture unit.

17. The method according to claim 16, wherein the cell culture device does not have the medium supply line, the liquid-transfer pump, an air supply port, an air discharge port, and an oxygen permeation membrane.

18. The method according to any one of claims 1 to 17, wherein the cell is selected from the group consisting of pluripotent stem cells, tissue stem cells, somatic cells, germ cells, sarcoma cells, established cell lines, and transformants.

19. The method according to any one of claims 1 to 18, wherein the cell is selected from the group consisting of human mesenchymal stem cells, osteoblasts, chondrocytes, and cardiomyocytes.

20. The method according to any one of claims 1 to 19, wherein the step of producing an exosome is at least partially the same as the step of culturing a cell.

21. The method according to any one of claims 1 to 20, wherein the step of producing an exosome is continued over 1 month, 2 months, 3 months, 6 months, or a longer period of time.

22. An exosome production device for use in the method according to any one of claims 1 to 21, the device comprising the cell culture module.

23. A kit for use in the method according to any one of claims 1 to 21, the device comprising the cell culture module.

24. Use of the cell culture module for the method according to any one of claims 1 to 21.

25. An exosome obtained by the method according to any one of claims 1 to 21, the method comprising the cell culture module.

# FIG. 1

CELL CULTURE MEDIUM FLOW INLET/OUTLET

CELL CULTURE MEDIUM FLOW INLET/OUTLET

CELL CULTURE MEDIUM FLOW INLET/OUTLET

CASING

EP 4 012 014 A1

# FIG. 2

POROUS POLYMER MEMBRANE

POROUS POLYMER MEMBRANE

EP 4 012 014 A1

FIG. 3

(A)  (B)

FIG. 4

# FIG. 5

AT THE TIME OF MEDIUM EXCHANGE

ONE HOUR AFTER MEDIUM EXCHANGE

EP 4 012 014 A1

FIG. 6

FIG. 7

MESH SURFACE

LARGE PORE SURFACE

APPARENT MEMBER VOLUME

MEMBER VOLUME CONTAINING CELL SURVIVAL ZONE

# FIG. 8

SIPHON TYPE REACTOR

LID BODY

MEDIUM SUPPLY MEANS

MEDIUM UPPER
LIMIT LINE

U-SHAPED TUBE

MEDIUM SUPPLY LINE

MAIN BODY UNIT

CELL CULTURE
MODULE

PUMP

P

MEDIUM DISCHARGE
PORT

MEDIUM DISCHARGE
PIPE

MEDIUM DISCHARGE LINE

MEDIUM SUPPLY LINE

MEDIUM STORAGE TANK

MEDIUM

# FIG. 9

CELL CULTURE UNIT SUPPORT

HANDLE

CELL CULTURE UNIT

SIPHON TYPE CELL CULTURE DEVICE

SUMP UNIT

MEDIUM SUPPLY MEANS

SUPPLY

PILLAR

LIQUID LEVEL

INVERTED U-SHAPED TUBE

MEDIUM COLLECTING MEANS

MEDIUM DISCHARGE MEANS

RETURN

FIG. 10

(A)

CELL CULTURE DEVICE

MEDIUM DROPPING NOZZLE

MEDIUM SUPPLY MEANS

OUTER CYLINDER

CELL CULTURE UNIT

MEDIUM SUPPLY LINE

PUMP

MEDIUM COLLECTING MEANS

MEDIUM DISCHARGE PORT

MEDIUM DISCHARGE LINE

MEDIUM

MEDIUM SUPPLY LINE

MEDIUM STORAGE TANK

(B)

CELL CULTURE UNIT

BOTTOM PART

SIDE PART

MEDIUM DISCHARGE PORT

CELL CULTURE UNIT

MEDIUM SUPPLY PORT

UPPER PART

SIDE PART

BOTTOM PART

EP 4 012 014 A1

# FIG. 11

MEDIUM SUPPLY MEANS

OVERFLOW TUBE

MEDIUM DROPPING NOZZLE

MEDIUM DISCHARGE PORT

LID UNIT

MEDIUM SUPPLY PORT

SIDE PART

BOTTOM PART

MEDIUM DISCHARGE PORT

CELL CULTURE UNIT

# FIG. 12

FIG. 13

(A)

CELL CULTURE DEVICE

CELL CULTURE UNIT

MEDIUM TANK

SHAFT

INSTALLATION STAGE

(B)

MEDIUM SUPPLY LINE

MEDIUM DISCHARGE TANK

MEDIUM SUPPLY LINE

MEDIUM DISCHARGE LINE

PUMP

EP 4 012 014 A1

# FIG. 14

(A)

CYLINDRICAL VESSEL

MEDIUM FLOW INLET/OUTLET

SPIRAL FLOW CHANNEL

SPIRAL FLOW CHANNEL

MEDIUM FLOW INLET/OUTLET

SHAFT PENETRATING PORT

MEDIUM FLOW INLET/OUTLET

(B)

EP 4 012 014 A1

# FIG. 15

(A)

(B)

# FIG. 16

## (A)

## (B)

## (C)

# FIG. 17

CELL CULTURE DEVICE

OUTER CYLINDER LID

MEDIUM DROPLET
SUPPLY MEANS

DROPLET MESH

MEDIUM SUPPLY LINE

OUTER CYLINDER

CELL CULTURE
MODULE

PUMP

P

CELL CULTURE UNIT

MEDIUM
COLLECTING
MEANS

MEDIUM DISCHARGE PORT

MEDIUM DISCHARGE LINE

MEDIUM SUPPLY LINE

MEDIUM

MEDIUM STORAGE TANK

# FIG. 18

A

CHANGES OVER TIME IN EXOSOME
PRODUCTION AMOUNT IN DISH

B

PARTICLE DIAMETER DISTRIBUTION
OF DISH_DAY 13

# FIG. 19

A

CHANGES OVER TIME IN NUMBER
OF EXOSOMES IN BOTTLE (5% OXYGEN)

B

PARTICLE DIAMETER DISTRIBUTION
OF BOTTLE (5% OXYGEN)_DAY 27

# FIG. 20

A

CHANGES OVER TIME IN NUMBER
OF EXOSOMES IN BOTTLE (NORMAL OXYGEN)

B

PARTICLE DIAMETER DISTRIBUTION
OF BOTTLE (NORMAL OXYGEN)_DAY 27

# FIG. 21

A

GLUCOSE CONSUMPTION AMOUNT AND LACTIC ACID PRODUCTION AMOUNT

B

OVERFLOW CELL CULTURE SYSTEM

# FIG. 22

A

NUMBER OF EXOSOMES PRODUCED PER DAY
OVERFLOW REACTOR (5% OXYGEN)

B

PARTICLE DIAMETER DISTRIBUTION
OF REACTOR (5% OXYGEN)_DAY 85

# FIG. 23

A

GLUCOSE CONSUMPTION AMOUNT AND
LACTIC ACID PRODUCTION AMOUNT

B

OVERFLOW CELL CULTURE SYSTEM

# FIG. 24

A

NUMBER OF EXOSOMES PRODUCED PER DAY
OVERFLOW REACTOR (NORMAL OXYGEN)

B

PARTICLE DIAMETER DISTRIBUTION
OF REACTOR (NORMAL OXYGEN)_DAY 85

# FIG. 25

A

GLUCOSE CONSUMPTION AMOUNT AND LACTIC ACID PRODUCTION AMOUNT

B

CHANGES OVER TIME IN NUMBER OF EXOSOMES IN WAVE REACTOR

C

PARTICLE DIAMETER DISTRIBUTION OF
WAVE REACTOR (NORMAL OXYGEN)_DAY 39

FIG. 26

COMPARISON OF EXOSOME PRODUCTIVITY PER CELL (DAY 13)

# FIG. 27

COMPARISON OF EXOSOME PRODUCTION AMOUNT PER CELL

FIG. 28

miRNA ARRAY ANALYSIS OF DISH_DAY 6 AND BOTTLE (5% OXYGEN)_DAY 27

EP 4 012 014 A1

EP 4 012 014 A1

FIG. 29

miRNA EFFECTIVE FOR TREATMENT OF CARDIAC TISSUE DAMAGED OR DISEASED

FIG. 30

mi40_3RA195561_set32

miRNA EFFECTIVE FOR INHIBITION OF ORAL SQUAMOUS CELL CARCINOMA

EP 4 012 014 A1

FIG. 31

miRNA EFFECTIVE FOR INHIBITION OF GASTRIC CELL CANCER OR PROSTATE CANCER

EP 4 012 014 A1

# FIG. 32

COMPARISON OF EXOSOME PRODUCTION AMOUNT PER CELL

FIG. 33

miRNA ARRAY ANALYSIS OF DISH_DAY 13 AND BOTTLE (5% OXYGEN)_DAY 27

EP 4 012 014 A1

FIG. 34

miRNA EFFECTIVE FOR TREATMENT OF CARDIAC TISSUE DAMAGED OR DISEASED

FIG. 35

miRNA EFFECTIVE FOR INHIBITION OF ORAL SQUAMOUS CELL CARCINOMA

FIG. 36

miRNA EFFECTIVE FOR INHIBITION OF GASTRIC CELL CANCER OR PROSTATE CANCER

# FIG. 37

COMPARISON OF EXOSOME PRODUCTION AMOUNT PER CELL

EP 4 012 014 A1

FIG. 38

miRNA ARRAY ANALYSIS OF DISH_DAY 6 AND BOTTLE (NORMAL OXYGEN)_DAY 27

EP 4 012 014 A1

FIG. 39

miRNA EFFECTIVE FOR TREATMENT OF CARDIAC TISSUE DAMAGED OR DISEASED

FIG. 40

mi40_3RA195561_set33

miRNA EFFECTIVE FOR INHIBITION OF ORAL SQUAMOUS CELL CARCINOMA

EP 4 012 014 A1

EP 4 012 014 A1

FIG. 41

mi40_3RA195561_set33

100,000

10,000

1,000

100

10

1

0.1

1_dish1 Day6

0.1    1    10    100    1,000    10,000    100,000

18_bottle4(Normal) Day27

Plot

miRNA EFFECTIVE FOR INHIBITION OF GASTRIC CELL CANCER OR PROSTATE CANCER

# FIG. 42

COMPARISON OF EXOSOME PRODUCTION AMOUNT PER CELL

EP 4 012 014 A1

FIG. 43

mi40_3RA195561_set35

miRNA ARRAY ANALYSIS OF DISH_DAY 13 AND BOTTLE (NORMAL OXYGEN)_DAY 27

EP 4 012 014 A1

FIG. 44

miRNA EFFECTIVE FOR TREATMENT OF CARDIAC TISSUE DAMAGED OR DISEASED

FIG. 45

miRNA EFFECTIVE FOR INHIBITION OF ORAL SQUAMOUS CELL CARCINOMA

FIG. 46

mi40_3RA195561_set35

18_bottle4(Normal) Day27

5_dish2 Day13

miRNA EFFECTIVE OR INHIBITION OF GASTRIC CELL CANCER OR PROSTATE CANCER

# FIG. 47

COMPARISON OF EXOSOME PRODUCTION AMOUNT PER CELL

EP 4 012 014 A1

FIG. 48

miRNA ARRAY ANALYSIS OF DISH_DAY 6 AND REACTOR (5% OXYGEN)_DAY 27

EP 4 012 014 A1

FIG. 49

miRNA EFFECTIVE FOR TREATMENT OF CARDIAC TISSUE DAMAGED OR DISEASED

EP 4 012 014 A1

FIG. 50

miRNA EFFECTIVE FOR INHIBITION OF ORAL SQUAMOUS CELL CARCINOMA

EP 4 012 014 A1

FIG. 51

miRNA EFFECTIVE FOR INHIBITION OF GASTRIC CELL CANCER OR PROSTATE CANCER

FIG. 52

COMPARISON OF EXOSOME PRODUCTION AMOUNT PER CELL

FIG. 53

miRNA ARRAY ANALYSIS OF DISH_DAY 13 AND REACTOR (5% OXYGEN)_DAY 27

EP 4 012 014 A1

FIG. 54

miRNA EFFECTIVE FOR TREATMENT OF CARDIAC TISSUE DAMAGED OR DISEASED

FIG. 55

miRNA EFFECTIVE FOR INHIBITION OF ORAL SQUAMOUS CELL CARCINOMA

FIG. 56

miRNA EFFECTIVE FOR INHIBITION OF GASTRIC CELL CANCER OR PROSTATE CANCER

# FIG. 57

COMPARISON OF EXOSOME PRODUCTION AMOUNT PER CELL

EP 4 012 014 A1

EP 4 012 014 A1

FIG. 58

miRNA ARRAY ANALYSIS OF DISH_DAY 6 AND REACTOR (NORMAL OXYGEN)_DAY 27

EP 4 012 014 A1

FIG. 59

miRNA EFFECTIVE FOR TREATMENT OF CARDIAC TISSUE DAMAGED OR DISEASED

FIG. 60

miRNA EFFECTIVE FOR INHIBITION OF ORAL SQUAMOUS CELL CARCINOMA

FIG. 61

miRNA EFFECTIVE FOR INHIBITION OF GASTRIC CELL CANCER OR PROSTATE CANCER

EP 4 012 014 A1

# FIG. 62

COMPARISON OF EXOSOME PRODUCTION AMOUNT PER CELL

## FIG. 63

EP 4 012 014 A1

miRNA ARRAY ANALYSIS OF DISH_DAY 13 AND REACTOR (NORMAL OXYGEN)_DAY 27

FIG. 64

miRNA EFFECTIVE FOR TREATMENT OF CARDIAC TISSUE DAMAGED OR DISEASED

EP 4 012 014 A1

FIG. 65

mi40_3RA195561_set31

5_dish2 Day13 (y-axis)

20_reactor3(Normal) Day27 (x-axis)

miRNA EFFECTIVE FOR INHIBITION OF ORAL SQUAMOUS CELL CARCINOMA

FIG. 66

mi40_3RA195561_set31

miRNA EFFECTIVE FOR INHIBITION OF GASTRIC CELL CANCER OR PROSTATE CANCER

EP 4 012 014 A1

# FIG. 67

LACTIC ACID PRODUCTION AMOUNT
AND GLUCOSE CONSUMPTION AMOUNT

GLUCOSE
CONSUMPTION
AMOUNT

(mg/L)

2000 ⇒ 3000(mg/L)

Days

CHANGE OF
GLUCOSE
CONCENTRATION

LACTIC ACID
PRODUCTION AMOUNT

# FIG. 68

PARTICLE DIAMETER DISTRIBUTION
OF FIVE EXOSOME SAMPLES OBTAINED

····Ex1
—Ex2
---Ex3
—Ex4
—Ex5

Distribution(%)

Particle size(nm)

## FIG. 69

100 nm

Total Magnification : X320000

## FIG. 70

NUMBER OF
EXOSOME
PARTICLES

EXOSOME PRODUCTION AMOUNT
OVER TIME IN DIFFERENT REACTORS

HUMAN MESENCHYMAL
STEM CELL/
WAVE REACTOR (3)

HUMAN MESENCHYMAL
STEM CELL/
WAVE REACTOR (2)

HUMAN EMBRYONIC
CARDIOMYOCYTE/
OVERFLOW REACTOR

HUMAN MESENCHYMAL STEM CELL/
WAVE REACTOR (1)

Days

# FIG. 71

COMPARISON OF miRNA EXPRESSION AMOUNT AMONG CULTURE METHODS

OVERALL VIEW OF HUMAN-TYPE miRNA

miR-21, 22, 23, 24 SELECTED

EP 4 012 014 A1

# FIG. 72

# FIG. 73

OVERALL VIEW OF HUMAN-TYPE miRNA

miR-21, 22, 23, 24 SELECTED

EP 4 012 014 A1

# FIG. 74

# FIG. 75

MODULE CULTURE AND POROUS MEMBRANE
FRAGMENTS CULTURE OF CHONDROCYTE

TOTAL NUMBER OF CELLS

CELL DENSITY

# FIG. 76

OVERALL VIEW OF HUMAN-TYPE miRNA

miR-21, 22, 23, 24 SELECTED

FIG. 77

TO HARVEST
TUBE PUMP

## INTERNATIONAL SEARCH REPORT

| International application No. |
|---|
| PCT/JP2020/030613 |

A. CLASSIFICATION OF SUBJECT MATTER

C12M 1/00(2006.01)i; C12M 3/00(2006.01)i; C12N 5/07(2010.01)i; C12N 5/071(2010.01)i; C12N 5/0735(2010.01)i; C12N 5/074(2010.01)i; C12N 5/075(2010.01)i; C12N 5/076(2010.01)i; C12N 5/0775(2010.01)i; C12N 5/09(2010.01)i; C12N 5/10(2006.01)i

FI: C12N5/0775; C12M1/00 A; C12M3/00 A; C12N5/071; C12N5/0735; C12N5/074; C12N5/075; C12N5/076; C12N5/09; C12N5/10; C12N5/07

According to International Patent Classification (IPC) or to both national classification and IPC

B. FIELDS SEARCHED

Minimum documentation searched (classification system followed by classification symbols)

C12M1/00; C12M3/00; C12N5/07; C12N5/071; C12N5/0735; C12N5/074; C12N5/075; C12N5/076; C12N5/0775; C12N5/09; C12N5/10

Documentation searched other than minimum documentation to the extent that such documents are included in the fields searched

| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

JSTPlus/JMEDPlus/JST7580 (JDreamIII); CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X | HARASZTI, R. A. et al., "Exosomes Produced from 3D Cultures of MSCs by Tangential Flow Filtration Show Higher Yield and Improved Activity", Molecular Therapy, 2018. vol. 26, no 12, pp. 2838-2847, in particular, abstract, page 2839, left column, paragraph [0002], fig. 1 | 25 |
| Y | in particular, abstract, page 2839, left column, paragraphs [0002]-[0003], page 2843, right column, paragraphs [0002]-[0005], fig. 1, 2 | 1-24 |
| Y | WO 2018/021368 A1 (UBE INDUSTRIES, LTD.) 01.02.2018 (2018-02-01) in particular, claims 1-12, examples 12, 13 | 1-24 |

☒ Further documents are listed in the continuation of Box C. ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 01 October 2020 (01.10.2020) | 13 October 2020 (13.10.2020) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2020/030613 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y | WO 2018/021367 A1 (UBE INDUSTRIES, LTD.) 01.02.2018 (2018-02-01) in particular, claims 1-19, examples 12, 13 | 1-24 |
| A | 天野 愛、外 2 名，外部場による細胞外ベシクル放出誘導法の開発とバイオ機能，第 67 回高分子学会予稿集，2018, vol. 67, no. 2, #3U07, entire text, non-official translation (AMANO, Ai et al., "Development of a method for inducing the release of extracellular vesicular by the external field and bio-functions", Preprints of the 67th conference of the Society of Polymer Science, Japan) | 1-25 |
| A | JP 2019-126341 A (UBE INDUSTRIES, LTD.) 01.08.2019 (2019-08-01) entire text | 1-25 |
| A | JP 2018-076291 A (ASAHI KASEI MEDICAL CO., LTD.) 17.05.2018 (2018-05-17) entire text | 1-25 |

Form PCT/ISA/210 (continuation of second sheet) (January 2015)

**INTERNATIONAL SEARCH REPORT**

Information on patent family members

| International application No. |
|---|
| PCT/JP2020/030613 |

| Patent Documents referred in the Report | Publication Date | Patent Family | Publication Date |
|---|---|---|---|
| WO 2018/021368 A1 | 01 Feb. 2018 | US 2019/0330581 A1<br>in particular, claims 1-12, examples 12, 13<br>EP 3489348 A1<br>CN 109563464 A | |
| WO 2018/021367 A1 | 01 Feb. 2018 | US 2019/0264150 A1<br>in particular, claims 1-19, examples 12, 13<br>EP 3489351 A1<br>CN 109563476 A | |
| JP 2019-126341 A | 01 Aug. 2019 | (Family: none) | |
| JP 2018-076291 A | 17 May 2018 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013150303 A
- JP 2019038840 A
- JP 2009171876 A
- JP 2018064550 A
- WO 2003054174 A
- WO 2015012415 A
- WO 2010038873 A
- JP 2011219585 A
- JP 2011219586 A
- WO 2018021368 A
- WO 2009123349 A
- JP 2009057041 W

### Non-patent literature cited in the description

- *J. of Extracellular Vesicles,* 2015, vol. 4
- *JAMA Oncology,* 2016, vol. 2, 882-889
- *Trends in Biotechnology,* 2017, vol. 7, 665-676
- *Transplantation,* 2003, vol. 76, 1503-10
- *Am. J. Transplant.,* 2006, vol. 6, 1541-50
- *Int J Oncol.,* May 2016, vol. 48 (5), 1837-46
- *Oncotarget.,* 2014, vol. 5, 7748-7759
- *Molecular Therapy,* 2018, vol. 26 (12), 2838-2847